Europäisches Patentamt

(19) European Patent Office

Office européen des brevets

(11) Publication number: **0 211 073 B1**

# EUROPEAN PATENT SPECIFICATION

(45) Date of publication of patent specification: **18.05.94** (51) Int. Cl.5: **G05D 7/06, A61L 2/00**

(21) Application number: **86901586.7**

(22) Date of filing: **04.02.86**

(86) International application number:
**PCT/US86/00258**

(87) International publication number:
**WO 86/04698 (14.08.86 86/18)**

(54) GAS STERILANT SYSTEM.

(30) Priority: **05.02.85 US 698434**

(43) Date of publication of application:
**25.02.87 Bulletin 87/09**

(45) Publication of the grant of the patent:
**18.05.94 Bulletin 94/20**

(84) Designated Contracting States:
**AT BE CH DE FR GB IT LI LU NL SE**

(56) References cited:
WO-A-84/01507          GB-A- 2 052 800
US-A- 3 910 761        US-A- 3 982 893
US-A- 4 067 691        US-A- 4 164 538
US-A- 4 261 950        US-A- 4 294 804
US-A- 4 372 916        US-A- 4 404 651
US-A- 4 431 159        US-A- 4 447 399
US-A- 4 457 892        US-A- 4 504 442
US-A-42 397 31

Microprocessors & Microsystems,Volum 3,
No.8,published October 1979((Great Britain),
R.N. Mewis,"Triplicated Microprocessor Con-
trolled Automatic Shutdown System",see

pages 347 to 351.

(73) Proprietor: **Johnson & Johnson**
**P.O. Box 1222**
**One Johnson & Johnson Plaza**
**New Brunswick, N.J. 08903(US)**

(72) Inventor: **JEFFERIS, Raymond, P., III**
**276 Hillcrest Rd.**
**Wayne, PA 19087(US)**
Inventor: **ENGLER, Philip, V.**
**21 Maple Street**
**Tarrytown, NY 10591(US)**
Inventor: **ROSENBLATT, Aaron, A.**
**32 West 76th Street**
**New York, NY 10591(US)**

(74) Representative: **VOSSIUS & PARTNER**
**Postfach 86 07 67**
**D-81634 München (DE)**

EP 0 211 073 B1

## Description

The present invention relates to systems for delivering a gas to a confined chamber and to systems for sterilizing substances and articles and particularly to systems using a sterilizing gas to sterilize articles, for example medical apparatus such as utensils and instruments which may have been contaminated by foreign substances. The system of the present invention can also be used to sterilize non-medical articles and substances, as required. The system of the present invention relates particularly to a gas sterilizing system wherein two components which react to provide sterilizing amounts of a gas are combined in the field by the apparatus of the present invention. This allows the components which react to form the sterilizing gas to be shipped separately, which minimizes the possibility of accidents.

In particular, the present invention relates to a system using chlorine dioxide as the sterilizing gas. Chlorine dioxide gas is both unstable and toxic to humans. For example, chlorine dioxide gas, will, over time, decompose into its constituent parts and accordingly, it cannot be transported easily. It is therefore undesirable to transport chloride dioxide gas. Moreover, chlorine dioxide gas is somewhat explosive and also has a propensity to undergo catalytic decomposition. The components which react to form chlorine dioxide gas (e.g., sodium chlorite and chlorine gas), however, may be transported relatively easily and reacted on site to provide the sterilizing gas chlorine dioxide.

Prior systems have typically used ethylene oxide gas as a sterilant. For example, the castle 4040 ethylene oxide sterilizer manufactured by Sybron Corporation, Medical Products Division, is an example of such a prior system. Although ethylene oxide has been used as a sterilizing gas in the prior systems, chlorine dioxide is a preferred sterilant.

Furthermore, the systems used in the past have typically been of rather simple design and have not included advanced means for maintaining the reliability of the devices and safeguarding against accidents. Additionally, these systems have not provided a great deal of redundancy so that if a component of the system failed, manual intervention or service personnel was required to correct the failure before the sterilizing process could continue.

The invention is related to US-A-4 681 739 published on 21 July 1987.

US-A-4,067,691 relates to a sterilizing system and automatic control therefore. The sterilizing apparatus incorporates a vessel adapted to be heated and pressurized and electrically operated controls spaced above the vessel for controlling the operation of the apparatus during a sterilizing process, such controls being accessible for maintenance and repair activities within the neutral zone. An automatic controller for the sterilizing system includes sensing devices for monitoring the progress of the sterilization process on a step by step basis and furnishing signals to the various electrically operated controls to initiate each step of the sterilizing process when the preceding step is completed. In response to the occurrence of a malfunction in the sterilizing system, the controller prevents further operation of the system and signals an alarm condition. A printer associated with the computer prints out a record of the time at which each step is preformed so that proper operation of the apparatus can be verified.

Apparatus is provided for insuring that a quality assurance cycle is conducted periodically and such cycle must be successfully completed before the equipment can be returned to automatic service. Following any maintenance procedures, an equipment test cycle must be successfully performed, followed by a quality assurance cycle, before automatic operation can resume.

In the article "Triplicated microprocessor controlled automatic shutdown system" by R. N. Mewies in Microprocessors & Microsystems, Vol.3, No.8, Oct.1979, GB, a shutdown system is described which protects equipment, processes, environment and personnel in process plants like oil and gas production platforms or petrochemical process plants. This system takes into account multiple input combinations and initiates the appropriate outputs in the form of predetermined shutdown sequences.

This system has the capability of running all of a number of sequences simultaneously and asynchronously as time variant input alarms inititiate different shutdown sequences at different times. The conclusion of any sequence may require that it be automatically reset which is achieved providing all the reset conditions are fulfilled. Outputs coming to several sequences have to be taken into account so that, if one sequence is reset, it does not clear outputs from another sequence which may still be in progress.

The shutting down of the system is carried out in a particular order. For example, the system appears to be designed around a fractionating column for distilling petroleum, and various stages are shut down in a particular order which facilitates the orderly shutdown of the fractionating tower.

It is an object of the present invention to provide a sterilizing system which uses a gas having bacteriocidal, sporicidal, fungicidal and/or viricidal properties to sterilize articles.

It is a further object of the invention to provide a sterilizing system in which at least two components which react to provide a sterilizing gas are reacted on site within the apparatus of the present invention to

2

EP 0 211 073 B1

provide effective amounts of the sterilizing gas.

It is yet a further object of the present invention to provide a sterilizing system wherein the sterilant is chlorine dioxide gas.

It is still a further object of the present invention to provide a gas sterilizing system having built-in redundancy and means for maintaining the reliability and safety of the system.

It is still yet another object of the present invention to provide a gas sterilizing system which is versatile and which is controlled by a programmed microprocessor.

These objects are achieved by the features of claim 1.

The present invention particularly relates to a system for treating articles with a gas comprising first means for receiving a first component, second means for receiving a second component, the first and second components, when reacted together, forming the gas, means for reacting the two components together for forming the gas, valve means for supplying the gas to the chamber means to treat the article in the chamber means, means for removing the gas from the chamber means, electronic controller means for controlling the means for reacting, means for supplying and means for removing, comprising computer means executing a predetermined sequence of steps so as to cycle the apparatus through a series of successive states defining a cycle in which the article is treated by the gas and wherein the gas is thereafter removed from the chamber means so as to render the atmosphere in the chamber means within acceptable standards of safety.

The computer means include means for aborting the operation of the apparatus to one of a plurality of defined failure states in response to a failure of the apparatus, the selected failure state dependent on the state in the cycle in which the failure occurred.

The computer means preferably include memory means, and the apparatus further comprises means for receiving input signals from the valve means indicative of the closed or open condition of the valve means and means for transmitting output signals to the valve means to selectively open or close the valve means, image signals of the input and output signals being stored in the memory means, mask means being stored in the memory means, the computer means comparing the image signals of the input and output signals and generating an alarm signal if the input and output image signals do not agree in response to the setting of a bit in the mask means.

Other objects, features and advantages of the present invention will be apparent from the description which follows.

BRIEF DESCRIPTION OF THE DRAWINGS

The present invention will be described in greater detail with reference to the accompanying drawings in which:

FIG. 1 is a block diagram of the overall gas sterilant system according to the invention;

FIG. 2 is a block diagram of the sterilizing chamber and the valve and pump block of the gas sterilant system according to the present invention;

FIG. 3 is a block diagram of the electronic control circuitry of the gas sterilant system;

FIG. 3A is a table of addresses used in the electronic controller of FIG. 3 and the corresponding components or signals controlled by the addresses;

FIG. 4 is a block diagram showing how various system clock frequencies and the system interrupt are derived;

FIG. 5 is a front view of one embodiment of a control panel for the gas sterilant system showing the controller display lights and control switches;

FIG. 6 is a state diagram for the gas sterilant system according to the present invention;

FIG. 7 is a state output matrix corresponding to the state diagram of FIG. 6 for the gas sterilant system according to the present invention;

FIGS. 7A and 7B are flowcharts for the sequencing program for implementing the state diagram of FIG. 6;

FIG. 8 is a block diagram of the safety interlock arrangement for the gas sterilant system according to the present invention;

FIG. 9 is a functional flow diagram for the software resident in the memory of the electronic controller of the gas sterilant system according to the present invention;

FIG. 10 is a flow diagram for timed functions of the software for the gas sterilant system;

FIG. 11 is a flow diagram for one of the timed functions of the software for the gas sterilant system;

FIG. 12 is a memory map of the data memory of the electronic control circuitry for the gas sterilant system according to the present invention;

3

EP 0 211 073 B1

FIG. 13 is a flowchart for another of the timed functions of the software of the electronic control circuitry for the gas sterilant system according to the present invention;

FIG. 14 is a flowchart of another of the timed functions of the software for the gas sterilant system according to the present invention;

FIG. 15 is a flowchart for another of the timed functions of the software for the gas sterilant system according to the present invention;

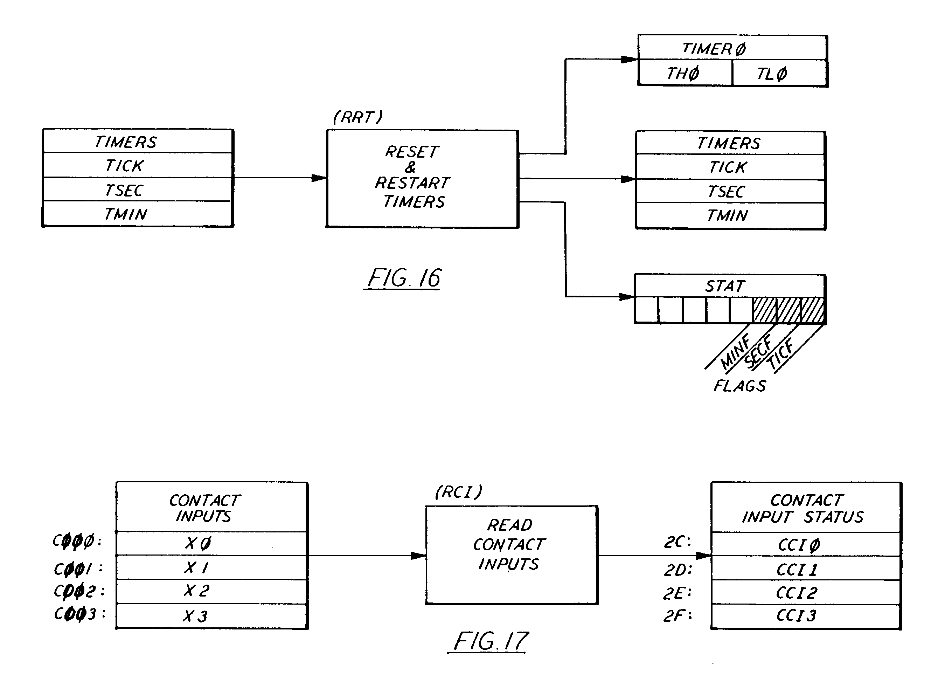

FIG. 16 is a flowchart for a program implemented in the control unit for resetting the control unit timed functions;

FIG. 17 is a flowchart for a program implemented in the control unit for reading in input data from the system according to the invention;

FIG. 18 is a flowchart for a program implemented in the control unit for providing a timeout alarm in the event of a component failure;

FIG. 19 is a flowchart for a program implemented in the control unit for providing an additional alarm in the event of a component failure;

FIG. 20 is a flowchart for a program implemented in the control unit for writing out data to the controlled components of the system;

FIG. 21 is a flowchart for a program implemented in the control unit for reading in analog input data from the controlled system;

FIG. 22 is a general flowchart for a program implemented in the control unit for providing the various timed functions of the system;

FIG. 23 is a flowchart for part of the program of FIG. 22; and

FIG. 24 is a flowchart for a program implemented in the control unit for controlling the system outputs.

DETAILED DESCRIPTION

Overall System

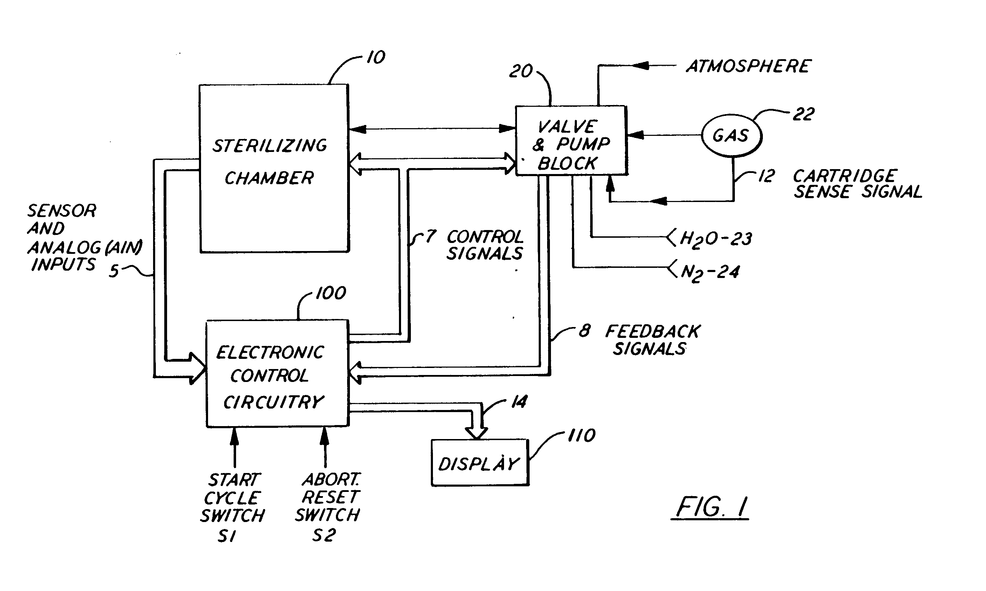

With reference now to the drawing figures, FIG. 1 shows the overall gas sterilant system. The system comprises a sterilizing chamber 10, electronic control circuitry 100 which is preferably microprocessor controlled, valve and pump block 20 and displays 110. Sensor inputs 5 including signals generated by appropriate sensors in chamber 10 and related to temperature, pressure, humidity and sterilizing gas concentration in the chamber 10 are fed from the sterilizing chamber 10 to control circuitry 100. The sensor inputs include both analog signals relating to the above measured chamber parameters and certain digital signals, e.g., a signal indicative of when the temperature in the chamber has reached a desired value, to be explained in more detail below. A START CYCLE switch S1 initiates operation of the system and an ABORT-RESET switch S2, as described in more detail later, is used to recycle the system states to a defined condition if an abort mode is attained, i.e., if a failure or alarm condition occurs. The operation of valve and pump block 20 will be described in more detail below, and includes a source of chlorine dioxide gas 22 which is produced on location from separated components, water vapor 23 and nitrogen 24. The valve and pump block is also vented to the atmosphere, as shown. Valve and pump block 20 includes a number of sequenced and controlled valves and a vacuum pump for providing the necessary conditions in the sterilizing chamber at the appropriate times. Because of the instability and potential toxicity of chlorine dioxide, the preferred sterilizing gas, it is preferable to transport components, which when reacted, form the chlorine dioxide gas. For example, the components may be sodium chlorite, $NaClO_2$ and chlorine gas, $Cl_2$.

Appropriate control signals 7 are fed by the electronic control circuitry 100 to the valve and pump block 20 and chamber 10 for controlling components of the system. Furthermore, feedback signals 8 from the controlled components are fed back to the control circuitry 100 so that the controller can monitor the state of the system and signals 14 are coupled to display panel 110 for informing the operator of the status of the system.

Additionally, a cartridge sense signal 12 is fed from the attached gas cartridge ($Cl_2$ component cartridge) to indicate that a gas component cartridge has been coupled into the system.

General Functions

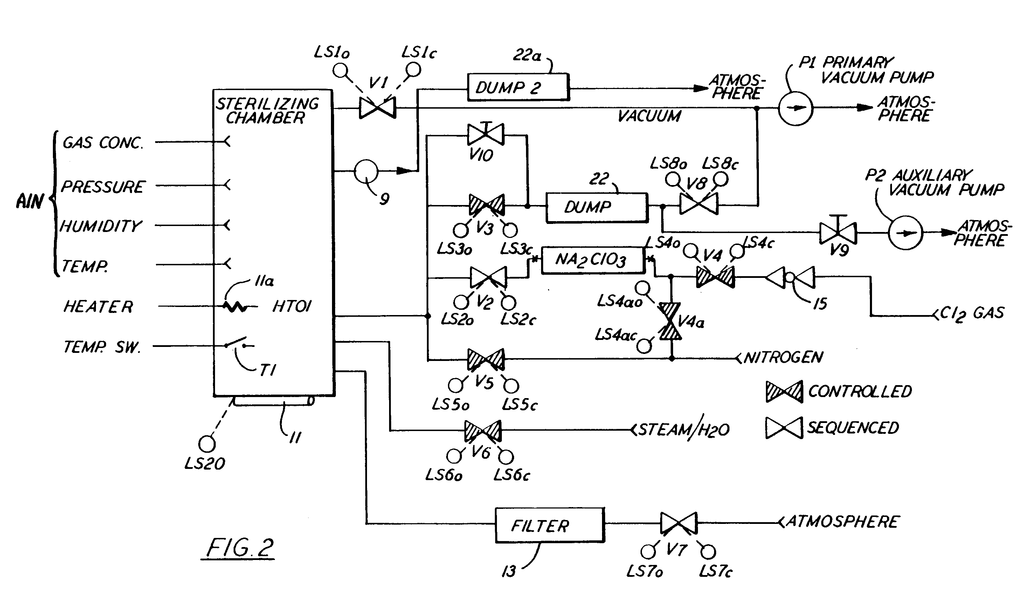

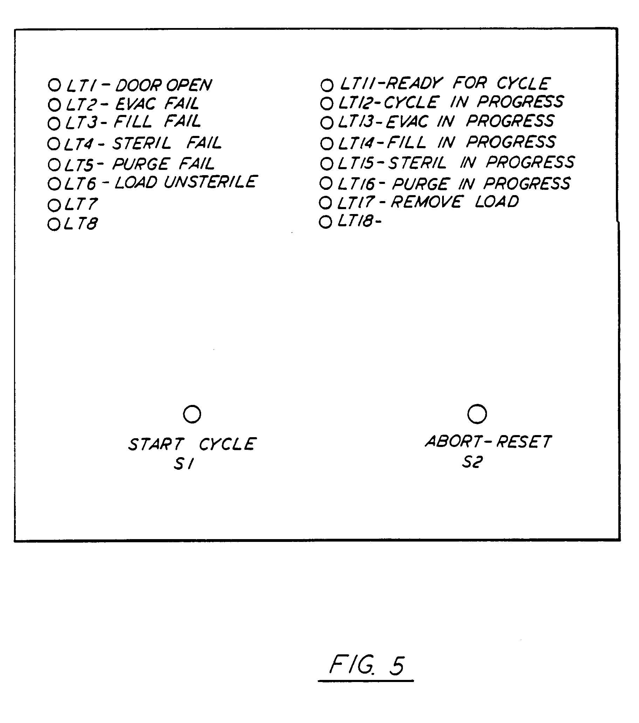

FIG. 2 shows the arrangement of valve and pump block 20 in more detail. Valve and pump block 20 includes a series of valves V1, V2, V3, V4, V4a, V5, V6, V7, V8, V9 and V10, pumps P1 and P2, air filter 13, a detoxifier 22 for detoxifying the evacuated chlorine dioxide gas, which may be implemented as explained in the above mentioned US patent 4 681 739, and appropriate sources of water vapor, nitrogen, $Cl_2$ gas, air,

4

and sodium chlorite. As shown in FIG. 2, some of the valves are merely sequenced, while others are controlled in response to selected ones of the values of the measured process variables, e.g., gas concentration, humidity level and pressure. For safety reasons, each valve (V) is fitted with two limit switches (LS) to indicate the open (e.g. LS2o) or closed condition (e.g. LS2c) of the valve. In the attached software listing, the open limit switches are referred to by the designation LSOx and the closed limit switches by the designation LSCx. Both switches must be in their proper positions at the proper times during the entire cycle in order that the cycle not be aborted. In addition, a number of lights are provided on a display panel, as shown in FIG. 5, which indicate the progress of the sterilization cycle or the occurrence of possible fault conditions. A cycle can be started by the operator, after the chamber door 11 is closed, by momentarily depressing the START-CYCLE (S1) switch. See FIG. 1. Thereafter the cycle proceeds automatically according to a program stored in the microprocessor memory of the electronic controller 100. This process will be described in more detail below.

Furthermore, in order to provide redundancy, a number of manually controlled valves, e.g. valves $V_9$ and $V_{10}$, are provided in case valves $V_3$ and $V_8$ do not open. These valves can be manually operated by service personnel so that potentially toxic gases can be removed via detoxifier 22 in the event valves $V_3$ and $V_8$ fail to open when sterilizing gas is in the chamber. An auxiliary vacuum pump is also provided so that the gas can be drawn out via the manually operated valves.

## Sterilization Cycle

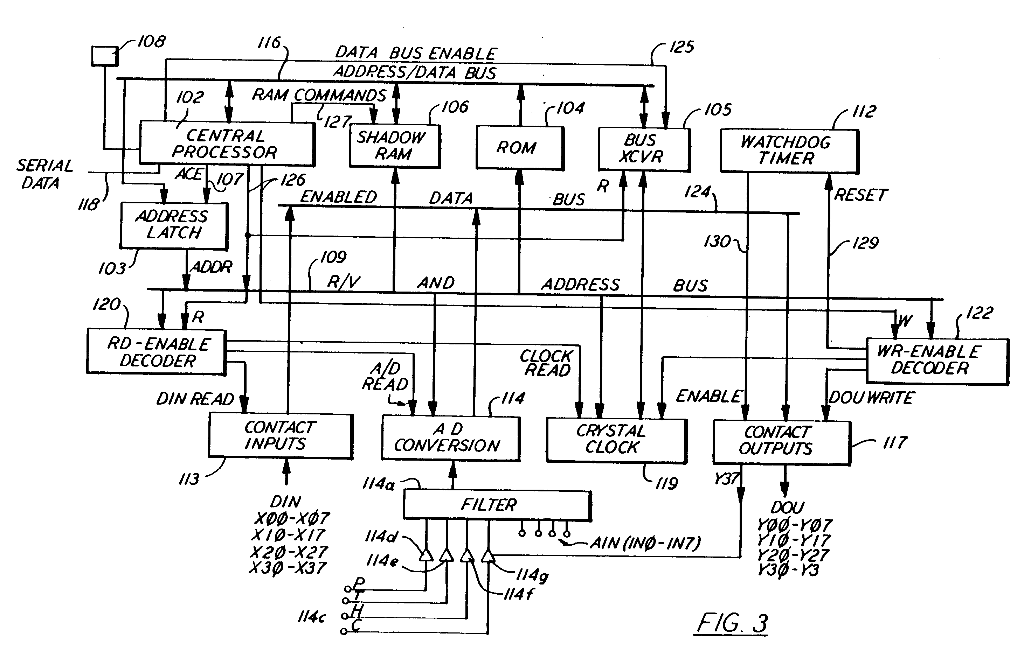

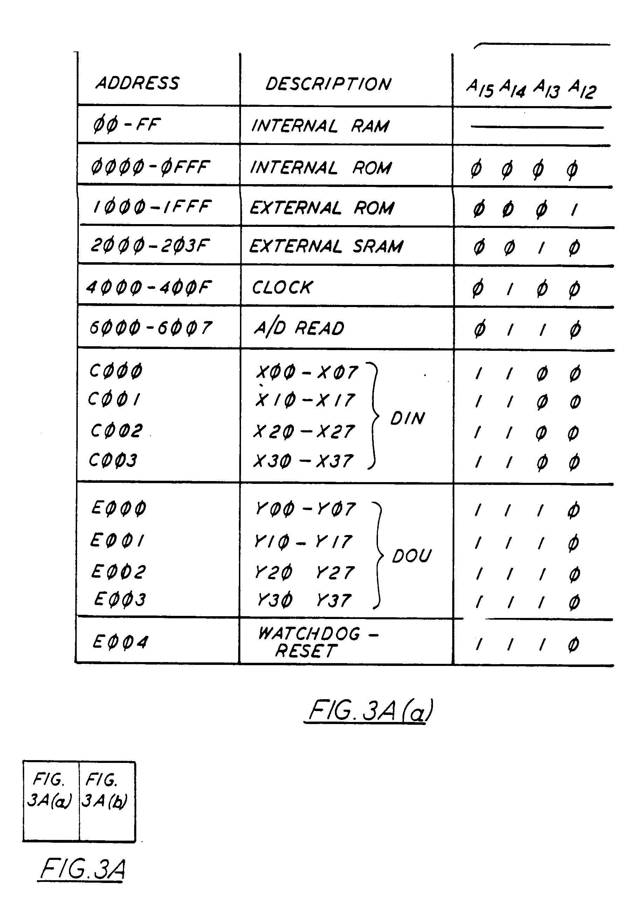

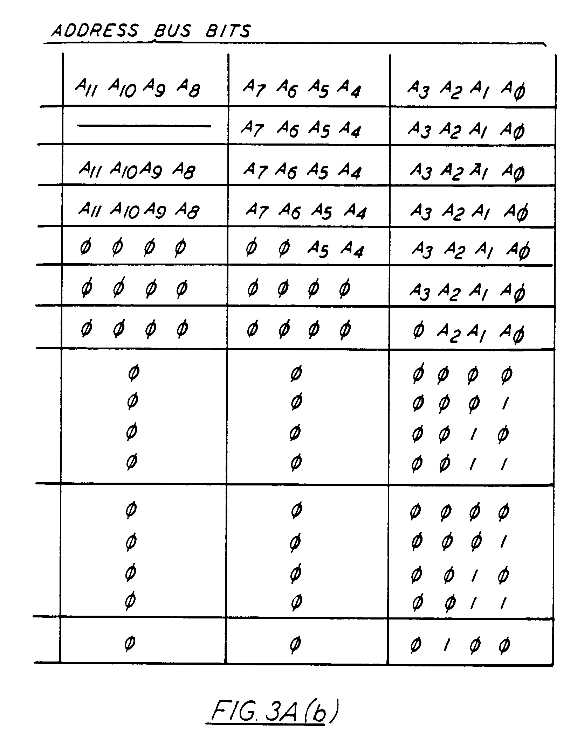

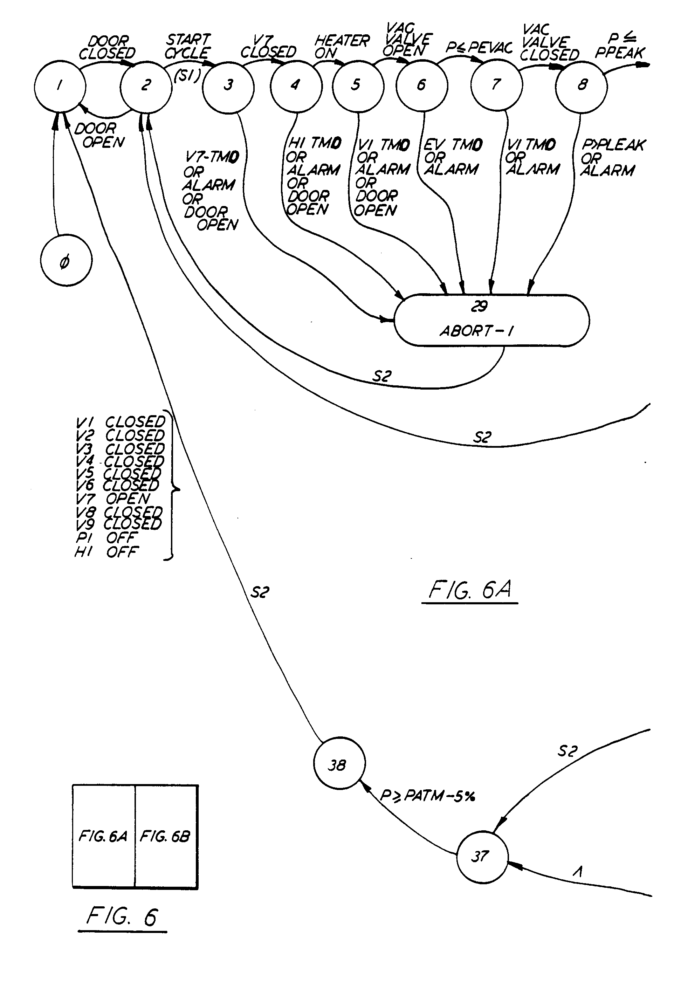

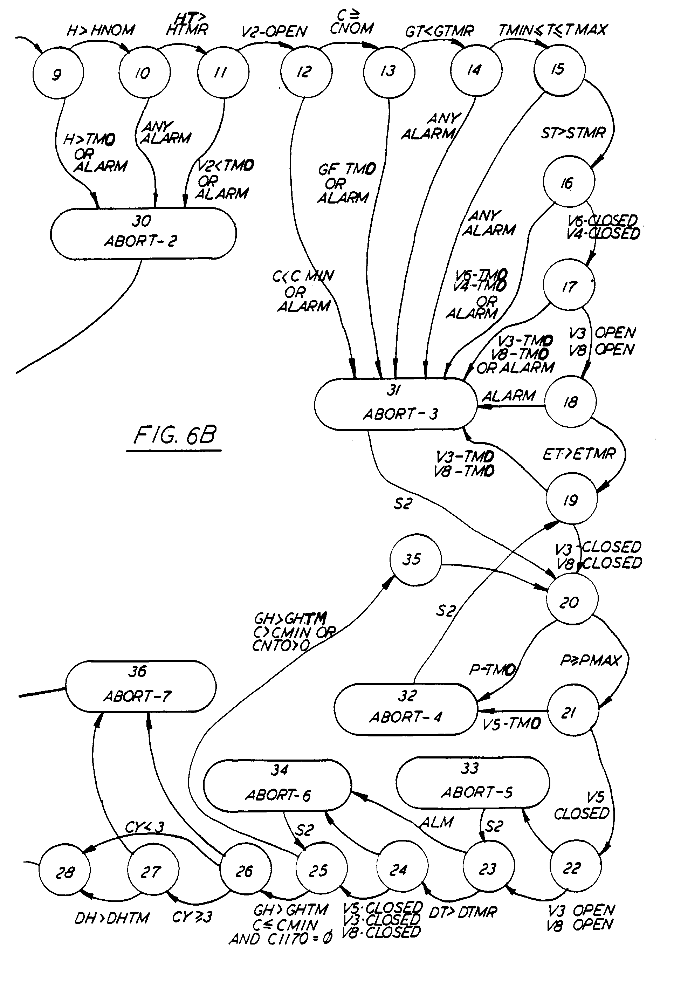

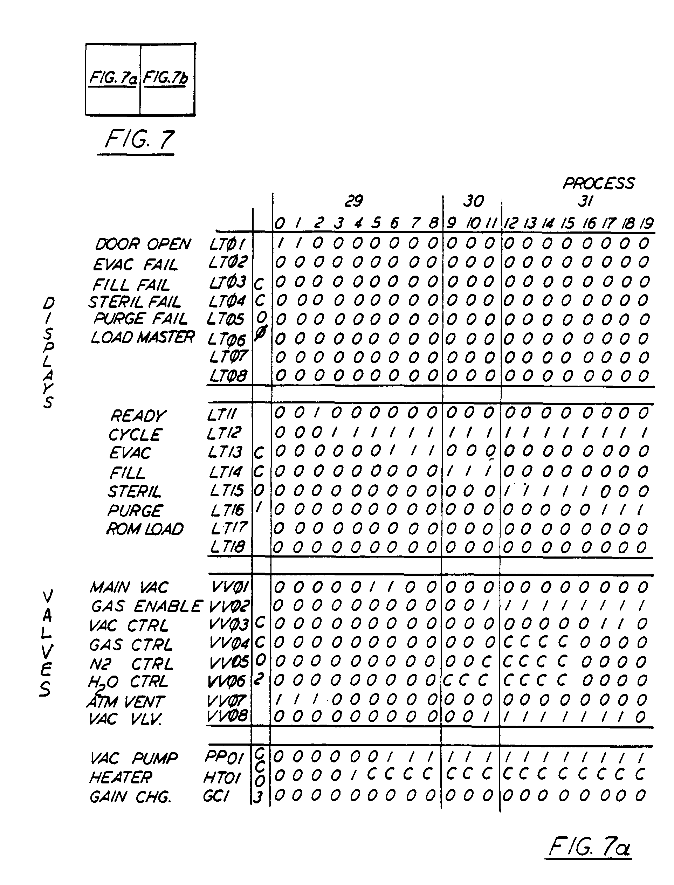

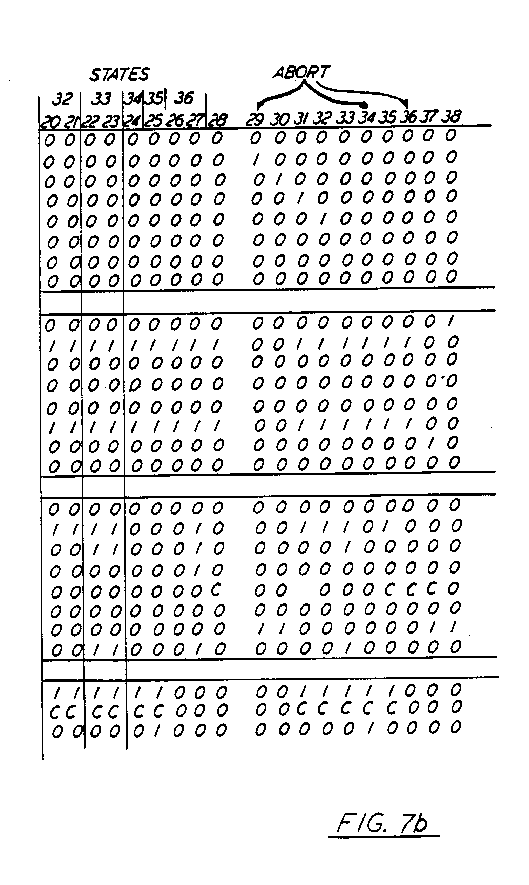

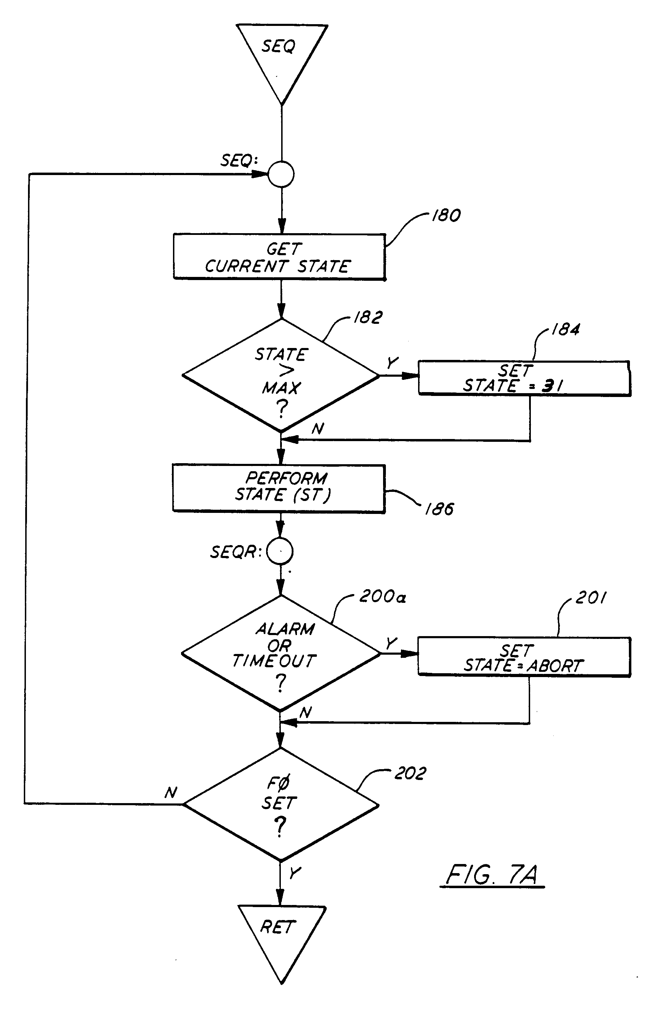

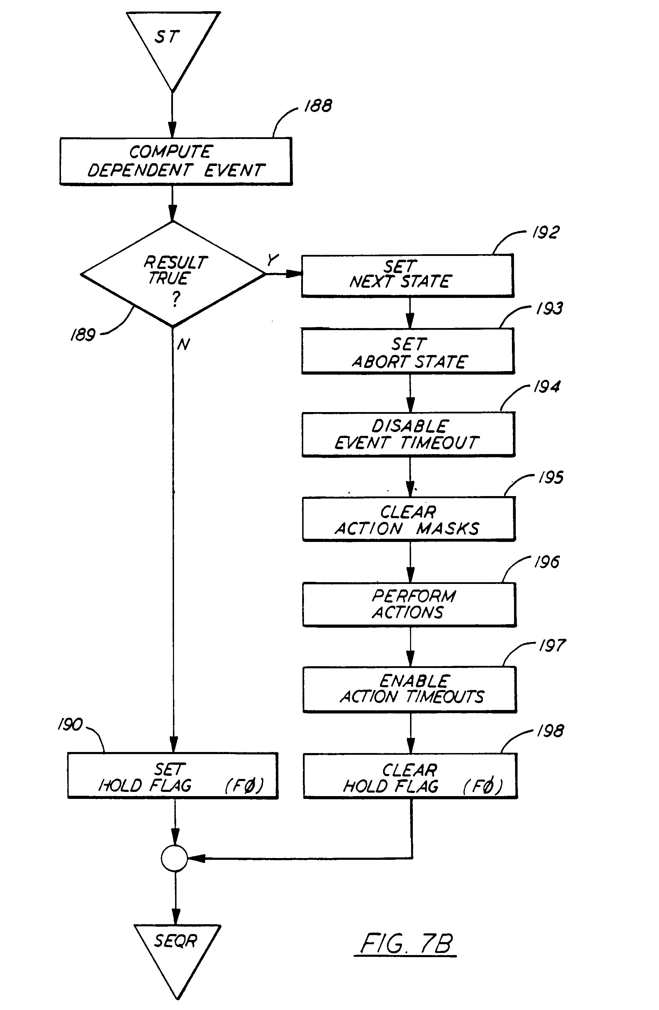

The sterilization cycle is an interlocked sequence of events and consequent actions under microprocessor control. The steps of this sequence are detailed in the state diagram of FIG. 6 and state output matrix of FIG. 7. These steps are performed by a sequencing program, the flowchart for which is shown in FIGS. 7A and 7B and the details of which are disclosed in the program listing contained in the appendix. Two types of events occur during the sequence, independent and dependent events. Some independent events are external events and include contact input signals to the controller from the controlled valves (e.g., the limit switches), and are referred to by the symbols XOx to X3x in FIG. 3. Each contact input signal is one bit of an eight bit word and the collection of such control input signals shall be referred to herein generally as digital inputs (DIN). Independent events also include the reception of signals corresponding to measured or analog process values (AIN), such as pressure, temperature, humidity and sterilizing gas concentration. The measured value signals are associated with logical comparison operations performed by the controller. Other independent events are internally declared, and these typically result in the illumination of an indicator light on the display panel, shown in FIG. 5. The controller evaluates the dependent events, which are logical combinations of independent events, to single TRUE or FALSE results. When the dependent event becomes true, a corresponding action is performed, i.e. the control system moves to a new process state, defined by the state output matrix of FIG. 7. If the dependent event is not true, the controller holds the process state in its memory and waits for a period of 50 milliseconds before reevaluating the dependent event. In the case of a system failure, the system automatically transfers to an appropriate ABORT state immediately, as will be described in greater detail below. This process continues until the cycle has been completed or aborted.

## Safety Considerations

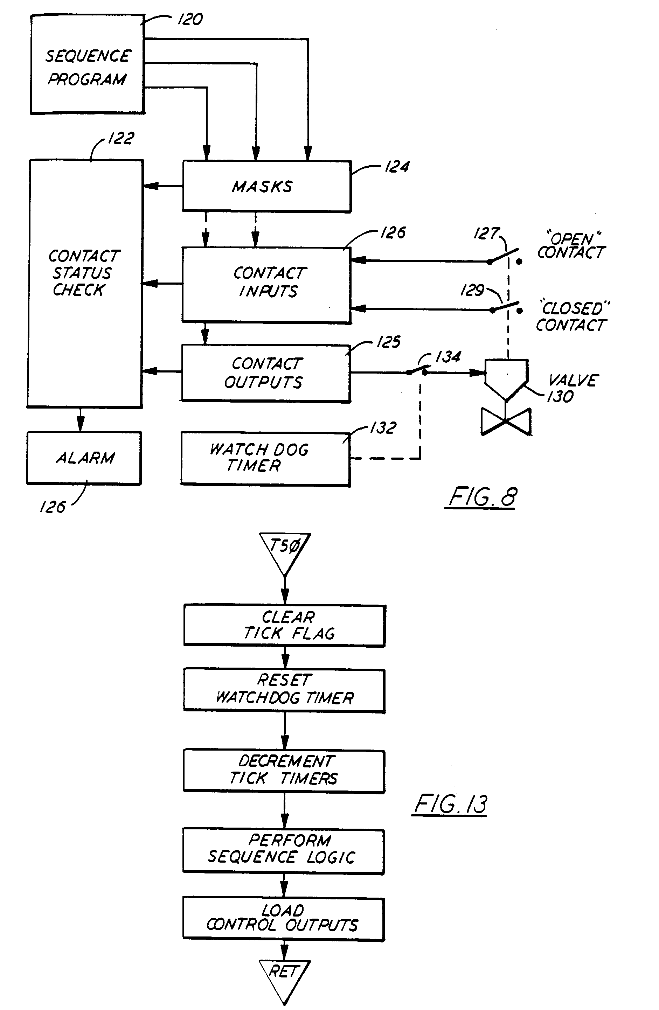

The sterilization system is provided with a number of checks to insure correct operation of the various valves and other components. As will be described in more detail below, interlock software implemented by the controller main timing program confirms the correct position of all valves every 6250 microseconds. An alarm condition is declared any time a valve is not in its commanded state. The operation of these interlocks differs from typical relay logic, or programmable logic controllers, in that interlock checking continues after valve actuation has taken place and can lead to different failure programming (ABORT states) at each process stage. The correct status of a valve is latched into memory after actuation is confirmed, and this latched condition is checked every 6250 microseconds. FIG. 8 is a block diagram of the safety-interlock components necessary to perform this checking routine. Failure to pass either an initial event-timeout condition following actuation or any subsequent status check will result in abnormal termination of the sterilization cycle. A sequence of control actions for safe termination of the cycle is defined for every point in the sterilization cycle, and is initiated immediately in the event of any abnormal (ALARM) process condition. This intensive status checking according to the invention prevents deliberate bypassing of the interlock switches in the field, since if a limit switch is bypassed, at some point in the system cycle the switch will be determined to be in an improper position, thus causing the system to enter an ABORT state.

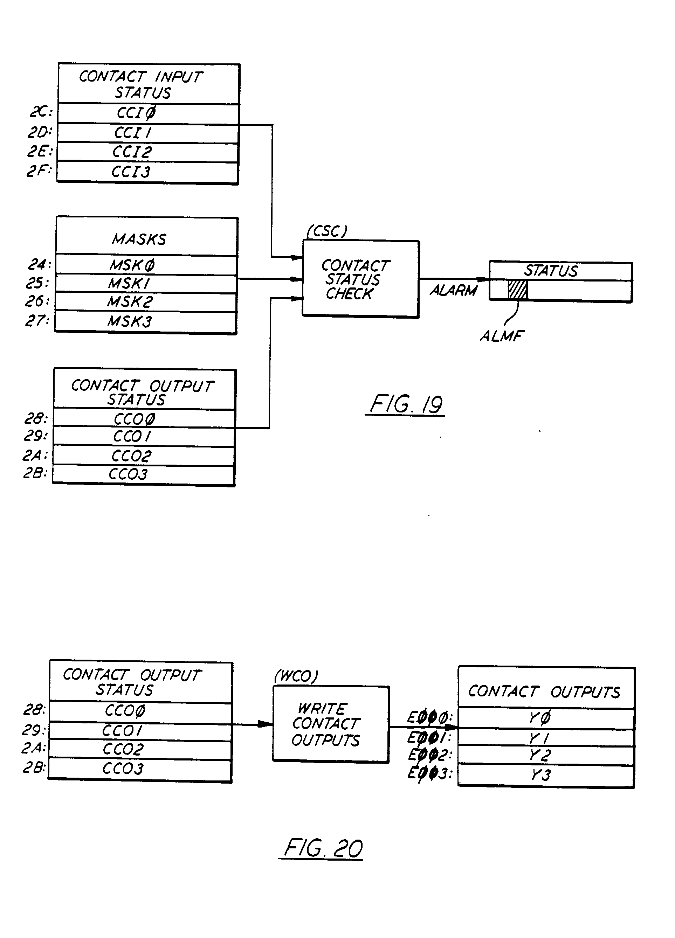

As shown in FIG. 8, the safety interlock system includes a Sequencing Program 120 stored in internal memory (ROM) of the electronic controller. Sequencing program 120 is identified in a listing of the program resident in memory attached hereto, as SEQ and the flowchart for this program is shown in FIGS. 7A and 7B. Also stored in memory is a Contact Status Check program 122 and a series of masks 124 which are determined by the particular point in the sequence program. The Contact Status Check program is identified in the appendix as CSC and a flowchart therefor is shown in FIGS. 18 and 19. Inputs 126, which are images stored in memory of actual input signals from both "open" limit switch contacts 127 (closed when a valve is open and open when a valve is closed) and "closed" contacts 129 (closed when a valve is closed and open when a valve is open) are provided, as well as inputs from other components, such as the sterilizing chamber 10 door 11. A series of contact outputs 125 are also provided by the particular state of the sequencing program. The Contact Status Check program 122 compares the contact inputs with the contact outputs 125. Whenever an input differs from the desired value, as established by the output, an alarm condition is declared if, and only if, a corresponding bit is turned on in the Mask 124. This safety feature detects any incorrect valve position immediately. A hardware implemented watchdog timer 132 is utilized to provide an extra level of safety by disabling all outputs to the valves 130 by opening electronic switches 134 when the timer times out if the microprocessor controller should fail, thereby preventing energization of any of the valves in the valve and pump block 20 in the event of a computer failure.

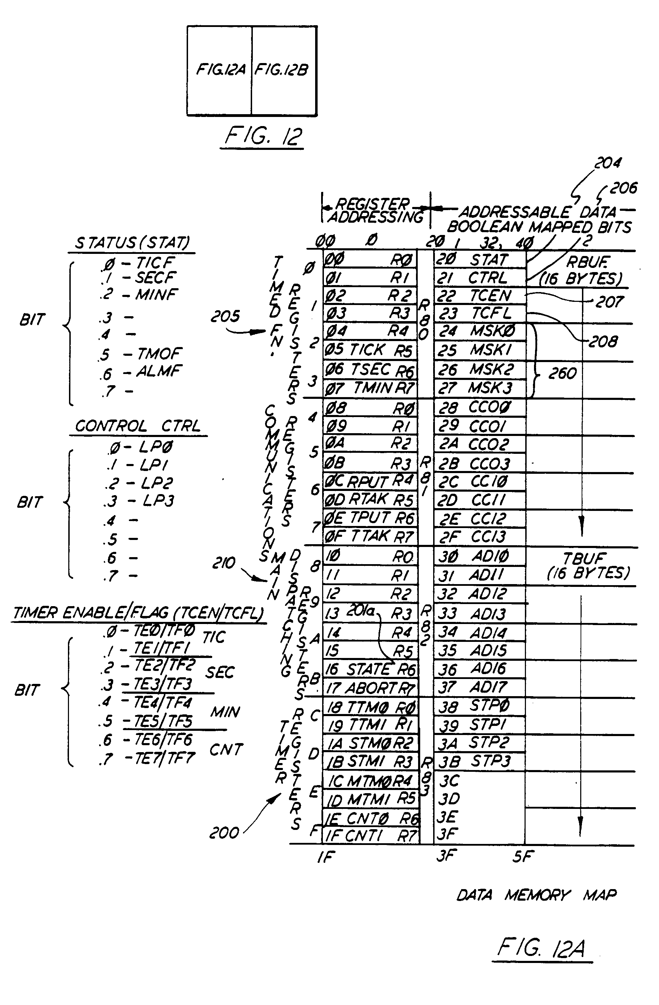

FIGS. 7A and 7B are a flowchart for the sequencing program SEQ. The sequencing program is entered from another program, called the Main Dispatching Program, which essentially checks for flags generated at appropriate time intervals and which determines when specific functions should be performed. As shown in FIG. 7A, when the sequencing program is entered, the current state of the system is retrieved from memory, as shown at 180. The current state is stored in a register 210a in internal CPU RAM, as shown in FIG. 12. The organization of internal CPU RAM will be discussed in more detail in connection with FIG. 12 later. At 182, a check is made to determine if the state exceeds the maximum state number. If it does, an ABORT state, state 31, to be discussed in more detail in connection with FIG. 6, is entered at 184. Otherwise, the conditions for the next state are performed at 186 by entering the program ST, the flowchart for which is shown in FIG. 7B.

As shown in FIG. 7B, program ST first evaluates each dependent event to a single true or false result, as shown at 188 and 189. Each dependent event is a logical combination of a number of independent events, each of which must be specified if the dependent event is true. If the dependent event is not true, a hold flag (FØ) in a memory location in the microprocessor internal RAM (see FIG. 12) is set at 190. Otherwise, the next state is set at 192 and a new ABORT state if a new ABORT state is required, is set, but not entered, at 193.

At 194, the timeout for the previous event must be disabled so that the timeout will not cause an alarm condition to be generated, which could cause an ABORT state to be reached. Timeouts are provided by program implemented timers, which monitor for the occurrence of a specified action, e.g. the movement of a valve, within a preset time defined by the timer. If the specified action has occurred, the timeout must be disabled because the timer continues to run. In order to disable the timeout, as shown in FIG. 18, a flag in the Timer Counter Enable Register (TCEN) 207 in internal RAM (FIG. 12) is cleared. In this way, when the flag for the timer is set into the Timer Counter Flag Register (TCFL) 206 (FIG. 12) when the timer runs out, no alarm will be generated. If a timeout alarm is generated, a bit TMOF is set in the STATUS register, as shown in FIG. 18.

At 195, the masks are cleared, i.e., bits corresponding to the particular events which are to take place are set to a "don't care" condition, so that the change of the corresponding bits in the contact outputs do not set off an alarm condition by the contact status check program. At this point, the action may be performed, as shown at 196. Subsequently, the timeout count for the action is loaded into the appropriate one of the timer registers 200 (FIG. 12) as will be explained in more detail later. The action timeout flag is then enabled to monitor for the timely occurrence of the current monitored action as shown at 197. The hold flag FØ is then cleared at 198 and a return is made to the flowchart of FIG. 7A, to the point denoted SEQR.

At 200a, a test is performed to determine if an alarm or timeout condition has occurred. If an alarm or timeout has occurred, the current state is set to the current ABORT state at 201 immediately. Then, the hold flag FØ is checked at 202 to determine if it has been set. If it hash a return is then made to the background or main dispatching program from which all subroutines are entered. If flag FØ has not been set, the system remains in the sequencing program to continue to the next state and only exits once flag FØ is set.

6

FIG. 19 shows the contact status check program in more detail. As shown, the contact input status corresponding to the contact inputs are stored inappropriate locations in the internal RAM of the system microprocessor. The memory locations are as indicated. See FIG. 12. The same is done for the contact output status bits, which specify the events to occur for a particular state. The Masks MSK0-MSK3, also stored in internal RAM, are evaluated by the contact status check program. If the contact inputs vary from the contact outputs, an alarm condition is generated by setting a bit in the status register 204, which is a location in RAM (see FIG. 12), but this is only done if the corresponding bit in the Mask is turned on. If the bit is off, indicating that a change of the corresponding output is to be allowed to occur, no alarm will be generated, and the contact outputs will be written into an output buffer, to be described in more detail below, to actuate the appropriate controlled or sequenced component, e.g., a valve or pump, without operating an alarm.

Additional safety features are also provided for in the system. As discussed above, manually actuable valves V9 and V10, operated by service personnel, and auxiliary pump P2 are provided in the event valves V8 and V3 and main pump P1 do not operate properly, thus providing a degree of redundancy. Furthermore, as shown in FIG. 2, safety features are provided to prevent the possibility of excessive temperatures and pressures in the sterilizing chamber 10. A thermally activated switch 11a is provided in series with heater HTØ1 in the chamber to detect excessive temperature. For example, should the heater HTØ1 fail to turn off, the thermostatic switch 11a will sense an excessive temperature and interrupt the circuit.

Additionally, should excessive pressures develop in the chamber, a pressure relief valve 9 is provided for venting gases in chamber 10 through a second detoxifier 22a to the atmosphere.

Also provided is a check valve 15 in series with valve V4 which supplies sterilizing chlorine gas to the system. Check valve 15 prevents the possibility of nitrogen gas from the nitrogen cannister pressurizing the chlorine gas cannister should valves V4 and V4a fail to close. Check valve 15 only allows chlorine gas to flow out of the chlorine gas cannister and prevents nitrogen gas from flowing into the chlorine gas cannister if valves V4 and V4a fail to close.

Operator Interactions

The apparatus and sterilization cycle of the system according to the invention provide for minimal operator intervention and maximum safety. FIG. 5 shows an embodiment of a display panel for the invention showing the various display lights. Certain lights are provided but not used, for expansion purposes. The sterilization cycle cannot be initiated until the chamber 10 door 11 has been properly closed. The DOOR-OPEN light (LTI) will then be extinguished, as shown by LT01 changing state from a "1" state in state 1 to a "0" state in state 2 of FIG. 7, and the READY-FOR-CYCLE light (LT11) will be illuminated. See also FIG. 5. To start the cycle, the operator merely presses the START-CYCLE (S1) switch (see FIG. 1) when ready. Thereafter, no operator intervention is required until the cycle ends, with illumination of the REMOVE-LOAD light (LT17), or until an alarm condition has halted the cycle. In the latter eventuality, one of the alarm lights indicating the failure will be on. The operator notes which lights are on, takes the necessary action and then presses the ABORT-RESET (S2) switch when ready to cycle the system back to a defined condition and to avoid the failure condition, if possible. For example, if the PURGE-FAIL light (LT5) is on, due to the possibility of an empty nitrogen tank, the tank should be replaced before pressing the S2 switch. Similarly for other failure modes, an attempt should be made to diagnose and remedy the failure condition before pressing switch S2. The subsequent actions to abort the cycle are then predetermined and automatic. No further operator intervention is necessary. Furthermore, redundancy has-been provided in the system so that if a component fails, another component, e.g., a pump or valve, can take the place of the failed component so that the system can be brought out of its failure state.

Control Circuitry Design

The overall design of the electronic control circuitry 100 is shown in FIG. 3. The controller is microprocessor controlled, and preferably utilizes a type 8031, 8051 or 8751 microprocessor CPU 102 manufactured by Intel Corp., because of the ability of these processors to perform Boolean arithmetic on bit addressable data. The CPU 102 includes self contained Random Access Memory (RAM) and Read Only Memory (ROM). Furthermore, the controller may include external ROM 104 and a non-volatile Shadow RAM (SRAM) 106 which may be a type X2210 manufactured by XICOR Inc. and which, as discussed heretofore, stores critical data after power-down. The controller also includes a clock crystal 108, input latch 113 receiving Digital INputs (DIN), an A/D Converter 114 and filter 114a for Analog INputs (AIN), an output latch 117 for Digital OUtputs (DOU), and a WatchDog Timer 112 (WDT). The latter timer is arranged to disable all outputs to the valves to their denergized state upon failure of the microprocessor, as described above with reference to FIG. 8. Analog to digital converter 114 and analog filter 114a, convert the analog inputs from the measured gas concentration, temperature, humidity and pressure parameters to digital data.

Central processor 102 is coupled to an address/data bus 116, which also couples RAM 106, ROM 104 and a bus tranceiver 105. An address latch 103 is enabled by a line 107 from the CPU/102, and latches addresses to a further bus 109, the Read/Write and Address Bus. Bus 109 allows the DIN Latch 113, A/D converter 114, a time stamp clock 119 and DOU latch 117 to be addressed at the appropriate times during execution of the sterilization sequence program, i.e., when CPU 102 calls for input data from the various valve limit switches, DIN latch 113 is addressed. At other times the A/D converter 114 or DOU latch 117 will be addressed.

Two decoders, a read enable decoder 120 and a write enable decoder 122 are coupled to bus 109 and allow latches 113 and 117 and A/D converter 114 to be either read from or written to. Appropriate read/write commands are coupled on lines 126 for controlling the decoders.

Furthermore, a data bus 124 is also provided for reading data from or to the input and output latches and A/D converter.

Several additional control lines are also employed, including a data bus enable 125 and RAM command lines 127. Line 125 enables bus transceiver 105 only for very short intervals and only during input/output (I/O) subroutines (e.g., subroutines WCO (Write Contact Outputs), RCI (Read Contact Inputs) and RAI (Read Analog Inputs), see appendix), when input and output operations are being performed, e.g., writing output information to DOU latch 117 for controlling the valves. In this way, data on the data bus 124 for actuating the various valves of the valve and pump block cannot be transmitted to the valves except under limited circumstances. This provides an additional degree of system safety. Furthermore, bus transceiver XCVR 105 is bi-directional and the direction of data transfer is controlled by one of the read and write lines, as shown.

RAM command lines 127 issue signals to shadow RAM 106 so that failures can be logged permanently and other critical data can be stored in the event of a power failure.

A reset line 129 is also provided between the Write enable decoder 122 and watchdog timer 112 and an enable line 130 is provided between timer 112 and DOU latch 117. As previously indicated, timer 112 monitors CPU 102 for proper system operation. Normally, CPU 102 constantly resets the watchdog timer via line 129. In the event of a CPU malfunction, the reset signal will fail to appear in time and the timer 112 times out and removes the output enable signal on line 130. The removal of this signal disables all DOU latch 117 outputs, thus preventing valve energization in the event of a CPU failure. Accordingly, a still further degree of safety has been provided in the system described.

Since the elements of the controller are coupled to data buses 116 and 124, as shown in FIG. 3, they have been assigned memory addresses through which they can be accessed by the microprocessor. FIG. 3A shows one arrangement of these addresses, for reference. As indicated above, certain of the devices, such as the SRAM 106 and DOU latch 117, are provided so that the data they contain can only be changed when bits of the microprocessor port lines are sequenced properly. This is a safety feature which prevents some microprocessor failure modes from causing undesired changes in memory contents or valve positions.

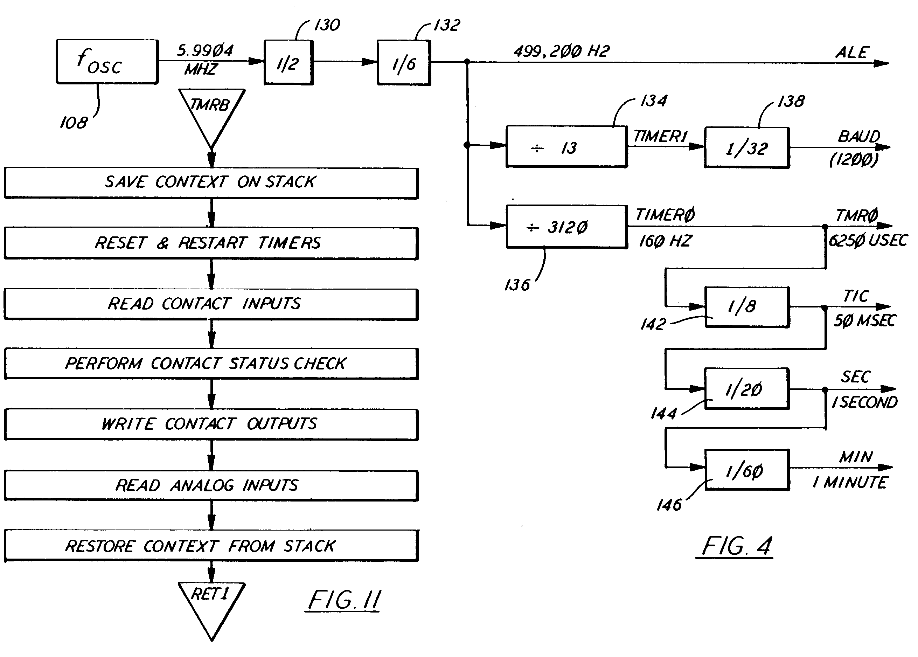

All processor and program timing is derived from the basic clock oscillator 108, which preferably has a frequency of 5.9904 MHz. FIG. 4 illustrates the relationship between the various frequencies used. As indicated in FIG. 3, provision may also be made to add a precision clock 119 to the system, which can be read by way of the data/address bus or via a serial data communications line 118 to provide a clock-calendar for time-stamping the process data.

As shown in FIG. 4, basic processor timing is provided by the CPU internal crystal controlled clock 108. The clock 108 frequency is divided by twelve by CPU internal counter stages 130 and 132 to provide the CPU Address Latch Enable (ALE) signal of 499,200 HZ. The ALE signal is used to strobe address latch 103 so that addresses can be placed on bus 109 and further controls the operation of A/D converter 114.

Signal ALE is also coupled to further internal divider stages 134 and 136. Divider stage 134 provides a signal designated TIMER 1, which is further divided by an internal counter stage 138 into a 1200 bit/sec signal for serial data transfer, which optionally may be provided to transmit system data to remote locations via serial line 118.

Counter stage 136 provides an interrupt, TIMERO. TIMERO provides a transition every 6250 usecs and allows the main timed function program, TMRO, to read all contact inputs and analog inputs and write all contact outputs every 6250 usecs. The operation of this program and other programs of the operating system will be described in more detail later.

The TIMERO interrupt is then further divided by program TMR0 software counter stages 142, 144 and 146, to provide the respective program execution signals designated as TIC, SEC and MIN, which occur at period of 50 msecs, 1 sec and 1 min, respectively. These will be discussed in further detail below.

FIG. 3A details the assignment of addresses on address bus 109. As shown, the bus 109 is a 16 bit bus. Internal CPU RAM is assigned address space 00-FF and bits A0 to A7 on the bus 109 identify the RAM locations. Internal ROM is identified by bits $A_0$ to $A_{15}$, with bits $A_{12}$-$A_{15}$ always being O's, as shown. Addresses from 0000 to 0FFF are used. The other components, external ROM 104, external RAM 106, clock 119, A/D converter 114, DIN latch 113, DOU latch 117 and watchdog timer 112 are assigned the addresses indicated in FIG. 3A. As shown, the DIN and DOU latches each are capable of latching 4 eight bit words, the DIN latches from the various limit switches and other contact inputs and the DOU latches to the various valves, pumps, etc. Digital inputs DIN and digital outputs D0U are each subdivided into four words of 8 bits each, and all eight bits of each group are accessed at one time by the respective addresses indicated in FIG. 3A.

As shown in FIG. 3, the analog pressure, temperature, humidity and chlorine dioxide gas concentration parameters are fed from respective sensors 114c to respective amplifiers 114d, e, f and g. In order to provide an additional degree of system safety when sterilizing chlorine dioxide gas is being evacuated from the sterilizing chamber, it is important that the chlorine dioxide gas concentration levels be accurately measured. Accordingly, amplifier 114g for the gas concentration signal is switched into a high gain mode by a control signal Y37 during the time when the sterilizing chamber is being evacuated. In this way, A/D converter 114 will compare the input concentration analog signal with a greater number of quantizing levels, thus providing a more accurate indication of the actual concentration. At all other times, amplifier 114g will remain in a low gain mode. For example, when chlorine dioxide levels are being measured in the chamber for purposes of determining an adequate sterilizing concentration, much higher concentration levels are being measured, and accordingly, A/D converter 114 provides an accurate digital signal corresponding to the analog concentration level. Therefore, amplifier 114g can remain in a low gain mode. Amplifier 114g may be switched to a high gain mode by signal Y37 changing from a "0" to a "1".

The system data-base may be thought of as being divided into external and internal sections. The external data-base contains the Contact inputs (CCI), which are comprised of the digital inputs DIN; the Contact Outputs (CCO), which comprise the digital outputs DOU; and the Analog INputs (AIN). Images of the external data-base are maintained in an internal data base comprising locations in internal RAM by subroutines of the TIMERO program (TMRO), which is invoked every 6250 microseconds. That is, every 6250 microseconds, all contact inputs and analog measurements are read and stored in the controller internal data-base and images of the contact outputs loaded in the DOU latch. With reference to FIG. 12, which is a memory map for the internal data RAM of CPU 102, images of the contact inputs are stored as the variables CCIO through CCI3, and the filtered analog inputs are stored as the variables ADIO through ADI7. The contact outputs are stored as variables CC00-CC03. Programs using the input data retrieve it only from these locations, and not from the input devices directly. Hence, the programs only operate on images of the inputs and outputs. In addition, the internal data-base includes a number of register banks, RB∅-RB3. In RB∅, a number of timers 205 are provided including a 50 msec timer TICK (50 msec), a second timer TSEC (1 sec) and a minute timer TMIN (1 min). These timers provide timed function intervals for scheduling functions implemented at those intervals by the system main dispatching program. The TICK timer times out after 50 msecs and sets a flag TICF in STATUS register 204 to be used by the main dispatching program to initiate all 50 msec timed functions, including a number of timers 200 in register bank RB3 which are invoked every 50 msecs, TTMx. These timers are preferably invoked for monitoring timeout conditions for the system valves, for example.

The TSEC timer similarly times out after 1 sec and sets a flag (SECF) in STATUS register 204, to be used by the main dispatching program to initiate all 1 second timed functions, including a number of timers 200 in RB3 which are invoked every second, STMx. Similarly, the TMIN timer times out after a minute and sets a flag (MINF) in STATUS register 204 to be used by the main dispatching program to initiate the 1 minute timed functions, including a number of timers 200 in RB3 which are invoked every minute, MTMx. The data memory also includes registers in RB2 for keeping track of the current state and ABORT state used by the sequence program. Also included are the sequence status register 204, TCEN and TCFL registers 207 and 208, already discussed, for the timers, and a control register CTRL for enabling a control calculation to open or close a valve. 4 bits of the control register, as shown, are used for controlling the four control loops of the system, corresponding to the measured temperature, humidity, pressure and gas concentration parameters. An array of bit masks 260 is provided in the internal data-base to permit "don't care" conditions when comparing contact input and output status. Further descriptions of the data elements are found in the controller program source listing in the appendix to this specification.

More particularly, internal RAM of CPU 102 may be organized as follows. The 256 (FF) memory locations are organized into 50 msec, one sec and one minute timers in the timed function registers (memory locations 00 to 07); optional communications program registers (memory locations 08 to 0F) for controlling a receive buffer RBUF and transmit buffer TBUF; main dispatching program registers (memory locations 10 to 17); timers 200 which are implemented at 50 msec, one second and one minute intervals by timers 205 (18 to 1D); (counters 1E and 1F); a status byte 204 (20); a control byte 206 (21); a timer enable byte TCEN (22); a timer flag byte TCFL (23); a series of masks 260 for the inputs; (24-27); the contact output images CC00-CC03 (28-2B); contact input images CCI0 CCI3 (2C-2F); analog inputs ADI0-ADI7 (30-37); and set points for the measured process variables, such as temperature, pressure, concentration and humidity (38-3B). The remainder of the internal RAM is assigned to the communications buffers (40 to 5F), the system stack (60 to 7F) and internal microprocessor registers and storage (80 to FF), the use of which is known to those skilled in the art. Refer to Microcontroller User's Manual, published by Intel Corp., May 1982, document No. 210359-001. Although the entire system program is contained in internal ROM of the CPU 102, an external ROM may also be provided so as to allow additional programming capabilities. Alongside FIG. 12, the contents of the STATUS, CTRL, TCEN and TCFL registers by bit are shown.

State Sequence

The progress of the sterilization cycle can be determined from the PROGRESS lights on the display panel, shown in FIG. 5. During a normal cycle the failure lights should never be on. Whether normal or aborted, both cycle and failure data will be maintained in a non-volatile random access memory or shadow RAM (SRAM). For example, after a designated number of cycles, e.g. three cycles, the gas cartridges will be discharged and must be replaced. The data concerning the number of cycles in which a cartridge has been used is stored in this memory. Also, after a predetermined number of cycles, or repeated failures, the system will be disabled until maintenance has been performed. This is a safety feature which cannot be bypassed in the field, and this data is also stored in the non-volatile memory.

As discussed, FIG. 6 is a state diagram which defines the operation of the sequencing program of the sterilant system. FIG. 7 identifies the condition of the components identified in FIG. 2 as well as the display lamps shown in FIG. 5 for the various process states. The operation of the system may now be described in further detail.

The system always begins in an initialization state, state 0. During this state, all output lines of the microprocessor in control circuitry 100 are set so as to initially deenergize all valves in the valve and pump block 20. After a short time delay, valve V7 is opened to allow air into the chamber, as shown by a "1" appearing opposite VV07 for state 0 in FIG. 7. Furthermore, during this state, the control circuitry 100 stores in memory the state of all output ports of the microprocessor.

In states 0 and 1, the door to the sterilizing chamber 10 is in its open position. Once the door is closed, state 2 is entered. As indicated in FIG. 6, this means that the system is ready to begin its cycle. As further indicated in FIG. 7, in state 2, valves V1-V6 are closed, valve V7 remains open and valve V8 is closed. Display lights LT1-LT6 are off, light LT11 (READY FOR CYCLE) is on and lights LT12-LT17 are off. The corresponding limit switches (LS) are in a position determined by the condition of the associated valve, e.g., for valve V2, which is closed, limit switch LS2o is open while limit switch LS2c is made. As indicated above, two limit switches are provided on each valve, one for the open position and one for the closed position, in order to insure the safety of the system. Both limit switches must be in their proper position, otherwise a failure will occur.

When the door to the chamber 10 is open, the system is in state 1, once the initialization state has been passed. Accordingly, only LT1 is on and the other lights are off, as shown in FIG. 7.

Assuming the chamber door has been closed and the system is in state 2, if the START CYCLE switch S1 is pressed, the system moves to state 3. At this point valve V7 closes, as indicated by the "0" appearing in the column for state 3 in FIG. 6 and light LT12, CYCLE IN PROGRESS, turns on. As indicated in FIG. 2, valve V7 vents the chamber 10 via a filter 13 to the atmosphere when open. Thus, the flow of filtered external air into the chamber is stopped when valve V7 closes.

If the door is opened in state 2, an immediate return to state 1 is made.

Once in state 3, and, if V7 is closed, as indicated by the closed state of limit switch LS7c and open state of limit switch LS7o, state 4 will be entered. If valve V7 does not close within a certain time, as determined by a timeout implemented by one of the TIC timers TTMx in RB3 of the data memory, state 29, ABORT-1 will be entered. Furthermore, it an alarm condition occurs, such as the opening of a valve which should be closed, an alarm condition will be generated and the point of failure indicated on the display panel, indicating to the operator that a malfunction has occurred.

Once in state 3, if the chamber door is opened, the cycle will be aborted, as shown in FIG. 6.

Assuming V7 has closed and state 4 has been entered, the chamber heater HTØ1 is turned on, as indicated by the "1" in the column for state 4 opposite HTØ1. If the temperature within the chamber increases to a sufficient level within a time-out period, state 5 can be entered. If not, ABORT-1, state 29, is entered and a return to state 2 is thereafter made when switch S2 is depressed. A safe operating temperature is reached when temperature switch T1 (FIG. 2) is actuated by the temperature of the atmosphere in the chamber reaching the desired temperature. After this occurs, the temperature in the chamber is controlled by turning the heater on and off as required during the cycle, as indicated by the notation "C" in the columns of FIG. 7 opposite "HTØ1".

Once state 5 is entered, valve V1 is opened, in preparation for starting vacuum pump P1 so that the atmospheric contents of chamber 10 can be evacuated. Again, if valve V1 does not open within a timeout period, ABORT-1, state 29 is entered.

State 6 is entered when vacuum valve V1 opens within the timeout interval. At this point, the vacuum pump P1 is started and light LT13 indicates that evacuation is in process. A timer is started which determines the length of time that the pump remains on.

Once in state 6, the chamber door 11 can no longer be opened, because, at this point in the cycle, the chamber is under a vacuum.

In state 6, the pressure in the chamber is checked to determine if it has been reduced sufficiently so that it is less than or equal to a nominal value, defined as PEVAC. If the pressure is less than PEVAC, then state 7 is entered and valve V1 is closed.

Should the pressure within the chamber be greater than PEVAC after the evacuation time has passed, indicating a less than adequate vacuum level, state 29 is entered. Similarly, if valve V1 does not close within a specified time, state 29 is entered from state 7.

After the valve V1 has been closed in state 7, a leak-hold test is commenced in state 8. If the pressure after a leak-hold time is less than a nominal value PLEAK, state 9 is entered. If not, abort state 29 is entered.

In state 9, water vapor is allowed to enter the chamber, i.e., valve V6 is placed in a controlled open state, as indicated by "C" in FIG. 6, and a determination is made whether the humidity has reached a specified level in a certain time. Should a nominal humidity HNOM not be reached within the specified time, state 30, ABORT-2, will be entered. Since evacuation has been completed, light LT13 is turned off and light LT14, which indicates a FILL IN PROGRESS, is turned on. By FILL is meant the supply of non-sterilizing gases into the chamber, e.g., steam and nitrogen gas. At this point, the system enters a new point in the state diagram wherein malfunctions allow the system to return to a different abort state, state 30. The state of the various valves and displays for ABORT-2 (state 30) is indicated in FIG. 7.

In state 9, the humidity timer times out. If the humidity level is greater than a nominal value HNOM, state 10 is entered. Otherwise, state 30 is entered and the cycle is aborted.

In state 10, a humidity hold test is performed wherein the humidity level is monitored for a predetermined time period. If the humidity level is not maintained for the predetermined time, state 30 is entered. Otherwise, state 11 is entered. Valves V2 and V8 are opened and valve V5, along with valve V6, is then controlled on.

Valve V5 allows nitrogen to enter the system. At this point, even though valve V2 is open, chlorine dioxide cannot enter the chamber because valves V4 and V4A, which are controlled together, are closed.

In state 11, valve V2 is checked to determine that it has opened. If it has not opened withing a specified time, state 30 is entered. If valve V2 has opened in time, state 12 is entered, and valves V4 and V4A are controlled on, allowing chlorine dioxide to enter the chamber. A timer is started during which time the chlorine dioxide gas concentration levels in the chamber are measured. As explained previously, chlorine dioxide may be generated by the reaction of two components, $Cl_2$ gas and sodium chlorite, $Na_2ClO_3$, on site. Chlorine gas is contained in a canister which can be coupled to the system via a connecting port, as known in the art. A container of sodium chlorite is coupled into the system between valve V2 and valve V4, as shown in FIG. 2. In state 12, LT14 is turned off and LT15, STERILIZATION IN PROGRESS, is turned on.

Once the gas concentration measured in state 12 has reached a concentration greater than or equal to a nominal concentration CNOM within a preset time period, state 13 is entered. An acceptable sterilizing gas concentration might be, e.g., 1.0 mg/L to about 300 mg/L. Otherwise a new abort state, ABORT-3, state 31, is entered. This new abort state is necessary because new conditions are now present in the sterilization chamber, since sterilizing chlorine dioxide gas is now present in the chamber. This requires a different set of procedures to be followed in the event of a failure, and accordingly, a new abort state is provided.

In state 13, a gas-hold test is commenced. If the gas concentration is greater than or equal to CNOM for a predetermined time period GTMR, state 14 is entered. Otherwise, state 31 is entered and the cycle is aborted.

In state 14, the temperature in the chamber is measured. If it is greater than a minimum temperature TMIN but not higher than a maximum temperature TMAX, state 15 is entered and a sterilization timer is started. If the temperature is not adequate, state 31 is entered and an abort occurs. A typical operating temperature is approximately 30~C.

During state 15, sterilization is in progress. Valve V6, for humidity control, is still controlled open, and valves V4 and V4A are also controlled open. Should an alarm condition occur, e.g., if any condition changes, i.e., a valve does not remain in its proper state, state 31 is entered. State 16 is entered only after a sterilization time STMR has elapsed, which typically might be several hours.

In state 16, valves V4, V4A and V6 are closed (if they do not close in the required timeout period, state 31 is entered), valve V3 is in a controlled state and valve V8 is still open. In state 17, light LT15 is turned off and light LT16 is turned on. Light LT15 is turned off when the sterilization timer has timed out and valves V4, V4A have closed. Light LT16 indicates that a purge is in progress. During state 17, the gases in the chamber are removed via valves V3 and V8 and detoxifier 22, labelled DUMP 22 in FIG. 2, which converts the chlorine dioxide into a harmless substance. The detoxification may be accomplished as explained in the above copending patent application S.N. 601,443, by passing the evacuated chlorine dioxide gas through a reducing agent, e.g., sodium thiosulfate. The detoxified gases are removed via valve V8 by vacuum pump P1. Should valves V3 and V8 fail to open within a timeout period, ABORT-3, state 31, is entered. During state 17, an evacuation timer is started which controls the amount of time during which chamber 10 is evacuated. State 18 is entered only if both valves V3 and V8 have opened in a predetermined time interval.

In state 18, once the evacuation timer has timed out past a time ETMR, state 19 is entered and valves V3 and V8 are closed. State 20 is entered when valves V3 and V8 close.

As shown in FIG. 6, should an alarm condition occur or should valves V3 or V8 fail to close within a specific time, state 31 is entered.

In state 20, valve V5 is in a controlled state. This allows nitrogen gas to enter the system as required and also prepares the system for the removal of any remaining sterilizing gases behind valve V2 via detoxifier 22 once valve V3 is reopened in state 22. In state 20, the pressure is checked. If it is greater than a maximum pressure PMAX, valve V5 is closed in state 21, turning off the nitrogen supply. If the pressure is less than PMAX, a new abort state, ABORT-4, state 32, is entered.

In state 21, valve V5 is checked to determine that it has closed within a prescribed timeout period. If it has not, state 32 is entered and the cycle is aborted. In state 22, the remaining sterilizing gases in the system are detoxified via detoxifier 22 and reopened valves V3 and V8 and the gases removed. Once valves V3 and V8 have opened for a sufficient period of time, state 23 is entered but only if valves V3 and V8 have opened. In state 23, another timer, denoted the DESORB timer, is activated. This allows sterilizing gases which have been absorbed into the materials in the chamber to be removed or desorbed over a time period DTMR.

Should valves V3 and V8 fail to open, ABORT-5, state 33, is entered. In this circumstance, the operator will be instructed to manually activate valves V9 and/or V10 so that sterilizing gas can be removed from the system. The manually operable nature of valves V9 and V10 is indicated in FIG. 2 by a T above the valve symbols. If valves V9 and V10 are manually opened, state 33, ABORT-5 will automatically be entered.

If state 23 is successfully reached and the DESORB timer times out after a time DTMR, state 24 will be entered. At this point, valves V2, V3 and V8 are closed and a check is made to determine that these valves are closed. Then, state 25 is entered, during which a low-gas-hold test is performed. If the gas concentration is less than or equal to an acceptable value CMIN within a time period GHTM, state 26 is entered. An acceptable level of safety might be, for example, less than .5 ppm of chlorine dioxide. Otherwise, a dummy state 35 is entered, before a return is made to state 20 by operation of switch S2. This provides a delay time in which to open valves.

In state 25, the gain of amplifier 14g (See FIG. 3) is changed so that the amplifier is placed in a high gain mode during the measurement of chlorine dioxide gas concentration levels during evacuation. This is indicated by the "1" in state 25 opposite GC1 (gain change control). This provides more accurate measurement of concentration levels during evacuation, providing an extra degree of system safety, as discussed previously. Also, in state 25, a counter CNT (see RB3 of FIG. 12) is decremented. This counter forces the system to cycle through states 25, 20, 21, 22, 23 and 24 via state 35 for a specified number of times determined by the initial count in the counter CNTØ. Accordingly, state 35 will be entered whenever the concentration level CMIN has not been reached within time GHTM or if the counter CNT has not reached Ø. State 26 will be entered from state 25 when both the concentration is less than CMIN and

CNTØ is Ø. This is provided to insure system safety in the event the concentration sensor in the sterilizing chamber should fail, By going through a number of cycles via state 35, the gas concentration will be decreased, thus insuring that, even if the concentration sensor indicates the gas concentration levels are below CMIN, the system will automatically cycle through a number of times necessary to reduce the concentration to acceptable safety levels. This is important, because if the concentration sensor failed and this additional safety feature was not provided, the system might indicate that the gas concentration level was within acceptable levels of safety although it actually might not be.

In state 26, a counter is checked which is incremented each time the system cycles at least to step 26. If, e.g., the count is less than 3, a jump is made to state 28. If greater than or equal to 3, state 27 is entered. In state 28, valve V5 is controlled on, and the count is then incremented. This allows nitrogen gas to enter the chamber.

If the cycle count is greater than or equal to 3, then state 27 is entered directly. In state 27, valves V2, V3, V4 and V8 are opened, and all remaining gas is dumped from the system and the $Cl_2$ gas in the cartridge is also dumped. Once sufficient time has elapsed, i.e., the Dump Hold time DHTM has elapsed, state 28 is entered. From state 28, the system enters state 37, during which the pressure in the chamber is monitored until it is within 5% of atmospheric pressure. At this point light LT17, REMOVE LOAD, is turned on. At this point, state 38 is entered, light LT11 is turned on and actuation of switch S2 enables a return to state 1. The operator will be notified to replace the gas cartridge if the system has gone through state 27.

As indicated in FIGS. 6 and 7, after ABORT states 29 and 30 are entered, a return is made to state 2 after switch S2 is depressed and state 2 conditions are set.

In ABORT state 31 a return is, made to state 20 and state 20 conditions are set once switch S2 is depressed. In ABORT state 32, return is made to state 19, and state 19 conditions are set. In ABORT states 33, 34, and 36, return is made to states 23, 25 and 37, respectively. If state 38 is reached, the operator receives an indication that the cycle is complete and light LT17 is turned on. To allow the chamber door to be opened, switch S2 is actuated, and state 1 is entered. If any ABORT state is reached, the appropriate failure light is illuminated. When a return is made to states 20, 23, or 26 from an ABORT state, the system then proceeds to cycle through the states which normally follow in the sequence.

General Software Functions

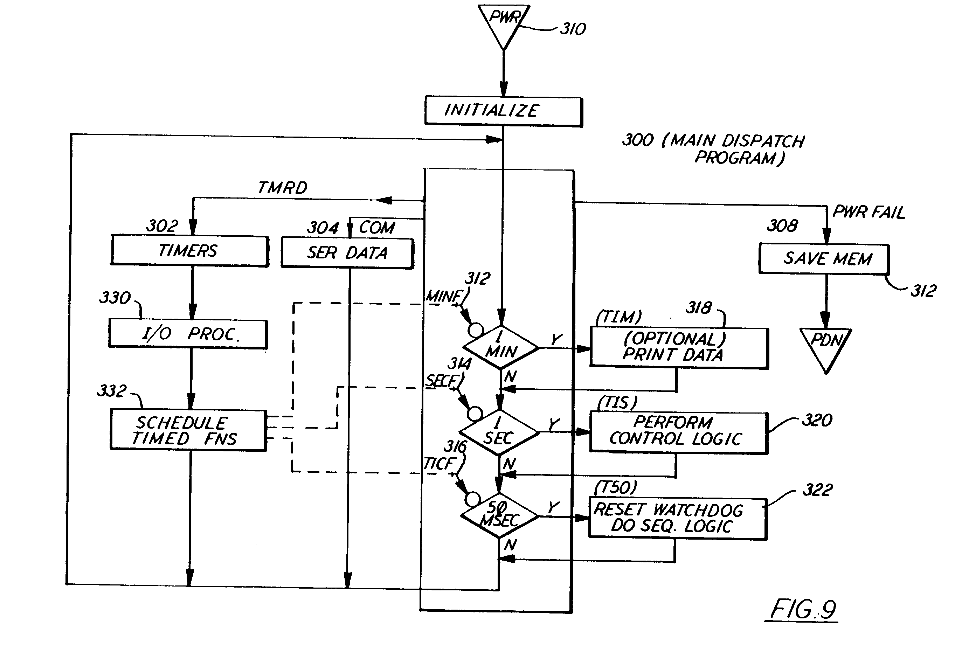

The sequencing program has already been described. Generally, software for the sterilization system controller is interrupt driven. Until an interrupt occurs a background task is always running via the main dispatching program. Upon interrupt, from any of several possible event sources, software control is passed to the appropriate interrupt handling program. This is illustrated in FIG. 9.

In FIG. 9, the main dispatching program 300 is shown. This program can also be found under this heading in the program listing attached hereto. Essentially, this program monitors for the occurrence of a timer flag indicating 50 msec, 1 sec or 1 minute functions must be performed. These flags are stored in the status register (STAT) 204 of FIG. 12. When a flag occurs, the program 300 jumps to the appropriate timer Program 318, 300 or 322. The timer programs are performed on a priority basis such that one minute functions are performed first and 50 msec (T50) functions last.

There are four sources of interrupting events: power-up, timer, communications, and power-down. Power-up, power-down and communications are external hardware interrupts, while the timer interrupt, TMRO, is an internal hardware interrupt under program control. Except for power-up, each interrupt handling program saves the running processor context in the CPU stack before starting its task function, and the context is restored before resumption of the interrupted program. The timer interrupt handler (TMRO) sequences all other non-interrupt programming functions. As discussed, it accomplishes this by passing one or more flags (MINF 312, SECF 314, TICF 316), signifying which of, the timed tasks is to run, through the STATUS register 204 of FIG. 12. The main dispatching program 300 tests the flags and will cause the selected functions to be executed as shown by 318-322. This method permits further interrupt action while lower priority functions are being completed. Some of the functions performed at one minute, one second and 50 msec (TIC) intervals are as indicated in FIG. 9 at 318, 320 and 322, respectively. The descriptions to follow will explain the tasks to be performed under each category of interrupt event in greater detail.

Main Dispatching Program

Essentially, the main dispatching program looks for timer flags and when one is found, calls the appropriate subroutine. See FIG. 9. The main dispatching program may be found in the attached program

13

listing.

## Power Up

Upon power-up as shown at 310, the processor stack, register bank, and other functions must be initialized. This interrupt function does not require saving of the processor context. Instead, previous process information will be read from the electrically reprogrammable memory SRAM 106, the clock 119 is reset and the process will resume from whichever state has been prescribed. The watchdog timer will be reset, and control will then pass back to the main dispatching program 300.

The power-up routine is found in the program listing under the program title INIT.

## Power Fail

A power fail program is preferably implemented. One embodiment for this program, as shown in FIG. 9, stores critical memory contents at 312 into the SRAM 106, where the data will be preserved until power is restored. The power-fail interrupt may be designed to occur whenever the 5 volt logic line drops below 4.55 volts, and recovery to 4.75 volts may be utilized for power-up. The power fail program can be found in the attached program listing.

## Communications

A communications feature (COM) may optionally be provided in the system according to the invention. The communications program is activated every time a character is removed from a serial output buffer or enters a serial input buffer. The function of this program is to feed characters to the transmit buffer as they are sent and to remove characters from the receive buffer as they are received. Two FIFO queues may be provided to hold the input and output data streams. The communications program tests the input and output data streams for the presence of termination or control characters. Flags are set in the event of termination characters. Programs, well known in the art, may be provided for processing control characters for typical serial interface devices connected to the control circuitry. For example, it may be desirable to transmit information for recording purposes over telephone lines to a printer or display device. Other programs, known in the art, can be employed to handle the standard modem control functions, e.g., RS232C commands. Hardware I/O lines may be provided for the necessary modem control signals. The communications program saves and restores the processor context.

## Timed Functions

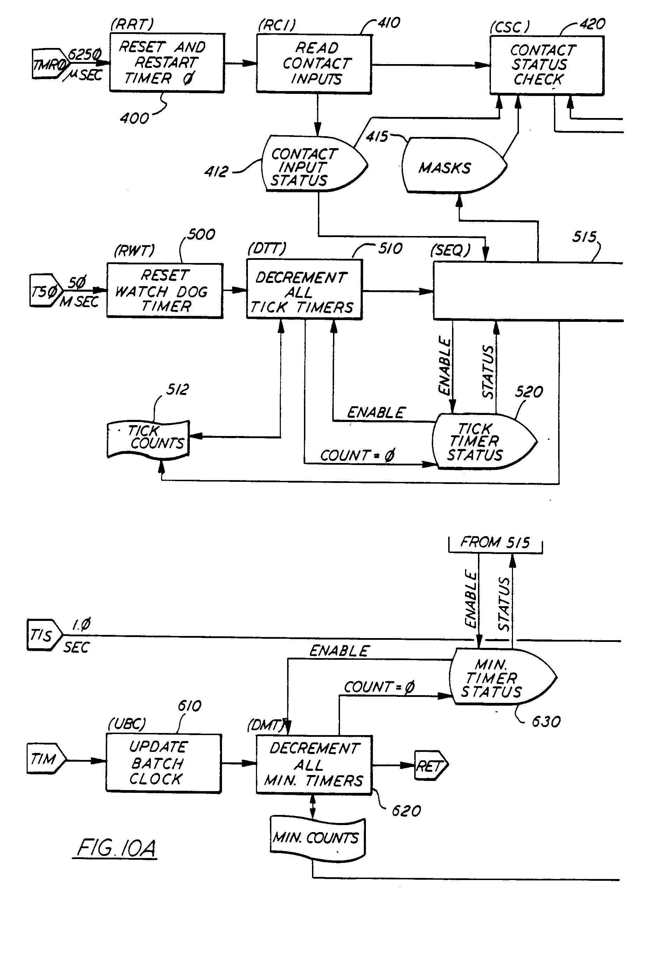

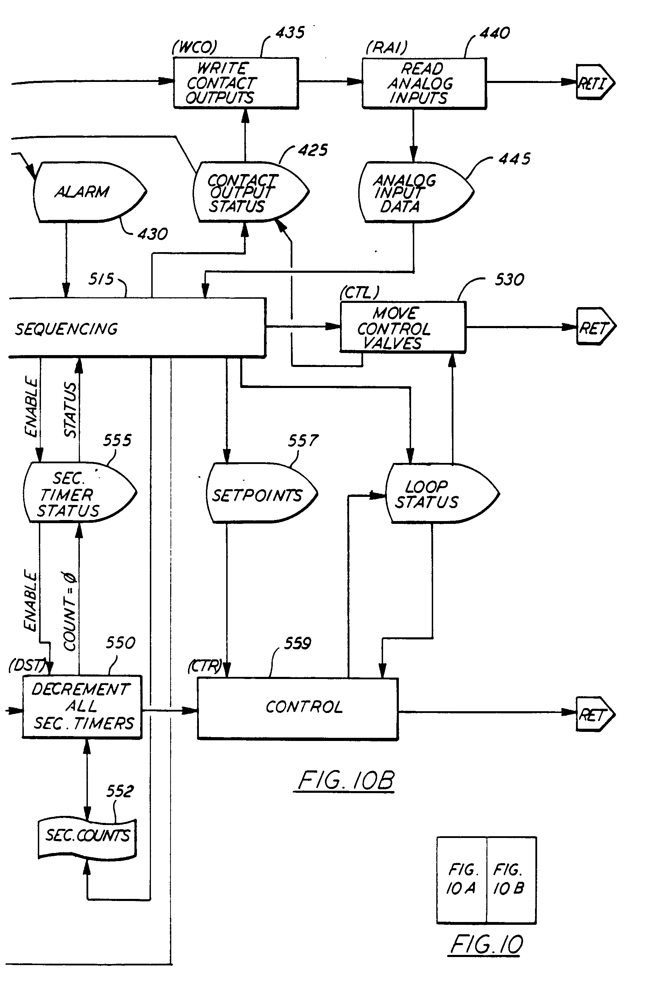

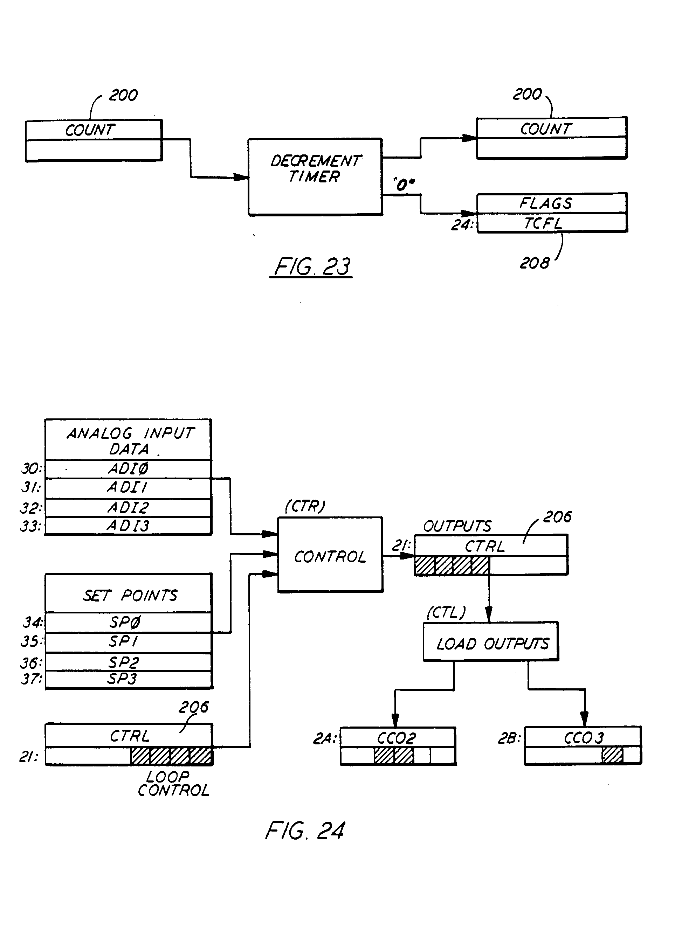

Timed functions in the controller occur on four levels as follows: functions triggered by the TIMER0 timer (every 6250 microseconds), functions initiated every 50 milliseconds (TICS), functions started every second, and functions which run every minute. Data is exchanged between these levels through defined data areas in the microprocessor data-base, as indicated more clearly in FIG. 10. The TMR0 program also accesses the input and output devices connected to the controller. The control function (CTR), which is activated every second, transmits valve commands to the upper four bits of the CTRL register when enabled by the lower four bits of the CTRL register on a bit by bit basis, as shown in FIG. 24.

As shown in FIG. 4, timer interrupts (TMR0) occur at intervals of 6250 microseconds (6.25 milliseconds). At each interrupt, the TMR0 program is entered, and all timed functions are scheduled. As the basic cycle time of the processor is approximately two microseconds, 3120 instruction cycles will elapse before the next such interrupt. Some of this time is used at each timer interrupt to perform data gathering and interlock functions, e.g. the analog inputs and data inputs are read and stored in CPU internal RAM. This is indicated at 330 in FIG. 9. Immediately following a timer interrupt the processor context will be saved in the appropriate registers. The interrupting timer, TIMER0, will then be reset and restarted. Program functions which are to occur at intervals of 50 msec., 1 sec., and 1 min. will be scheduled as shown at 332, by passing flags, as discussed, whenever the respective time interval has elapsed. Data inputs, status checks, and outputs are performed next. Finally, the previous program context is restored, and an interrupt return is executed. If any timed events are to occur, they will be performed in sequence by the main dispatching program. Otherwise the main disptaching program will be resumed.

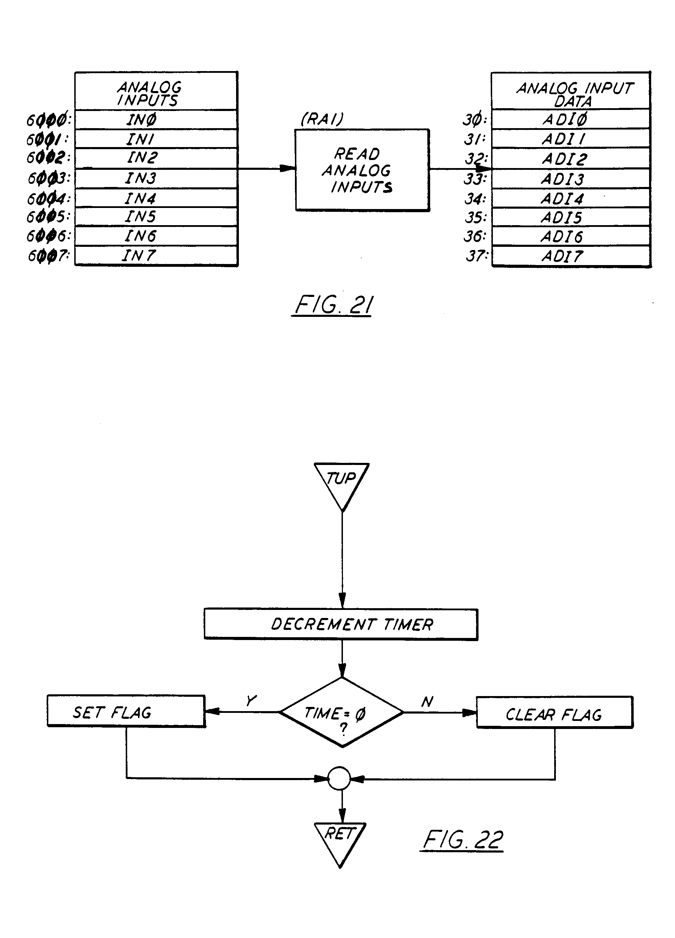

The basic timer program, which is executed for each timer, is shown in the flowchart of FIG. 22. As shown, the timer is first decremented and a check is made to determine if the timer has timed out, i.e., reached a count of 0. If so, the corresponding timer flag is set in TCFL register 208 shown in FIG. 12. If not, the corresponding flag is cleared. Then the program is executed for the next timer, and once all timers have been completed, a return is then made to the main dispatching program.

The decrement timer function is shown in FIG. 23. As shown, when a timer is decremented, a flag is set in the TCFL register if the time has timed out, and the current count is then stored in the appropriate timer register 200.

## 1. TIMERO Timer (TMR0)

The lowest level timed function, occurring every 6250 microseconds, is initiated by the interrupt TIMER0. This is indicated in the uppermost portion of FIG. 10, which is a flowchart for the various timed functions. After saving the processor context, the first function of the TMRO interrupt program is to reset and restart the timer as indicated at 400. This is performed by a subroutine RRT. In FIG. 10, the corresponding program for implementing the desired function is indicated above the flowchart symbol, and can be found in the listing in the appendix. The TMRO program is a time-critical function. Once the timer has been restarted, all of the contact inputs to the controller are read into their corresponding memory images, CCI0 - CCI3 as shown at 410 and 412. These images reside in a portion of the microcomputer memory which is bit addressable. This greatly facilitates logical processing. The subroutine for implementing this function is shown in FIG. 17 and is also shown in the attached program listing as subroutine RCI. The contact output information is also located in this memory, at CCO0 - CCO3 and is indicated in FIG. 10 at 425. The interrupt program next performs a masked comparison of the contact input and output status bits, using bit masks 415 also stored in this memory area. This is shown at 420. If any bits do not match their corresponding desired outputs, when masked for "don't care" conditions, an alarm condition is set by setting a bit in the STATUS register 204 (FIG. 12), as shown at 430.

Timeout alarms are also implemented by the TMRO program. A subroutine CSC2, as shown in FIG. 18 and the attached program listing, shows how timeouts are determined. When a timer times out, e.g., a timer for determining whether a valve has closed or opened in time, a flag will be set in the timer flag register TCFL. If the setting of the flag requires an abort upon failure, e.g., if the failure of a valve to close in time is to cause an abort condition, then a flag must be set in the timer enable register TCEN. This informs the timeout alarm program that an alarm condition should be set, which will cause the alarm condition to be loaded into the STATUS register. This will cause transfer to an ABORT state by the sequencing program.

Next the current contact output status is loaded from its memory image into the output contact latch by program WCO, as shown at 435. Finally, as shown at 440 and 445, the current analog input data 445 is read (RAI), exponentially filtered (FILTER), and stored in the correct memory locations outside the bit addressable space. See FIG. 21. Eight timer interrupts take 50 milliseconds. Thus, a well-filtered analog input scan of all eight analog inputs (only four need be used for the four control loops corresponding to gas concentration, pressure, temperature and humidity) will be available each time the 50-millisecond program is entered. Therefore, every 50 msecs, the RAI program obtains 64 input samples, 8 for each channel, the eight samples for each channel then being averaged to obtain a single analog value for each channel. A return is then made to the main dispatching program. The TIMER0 program is summarized in the flowchart of FIG. 11.

## 2. TIC Timer (T50)

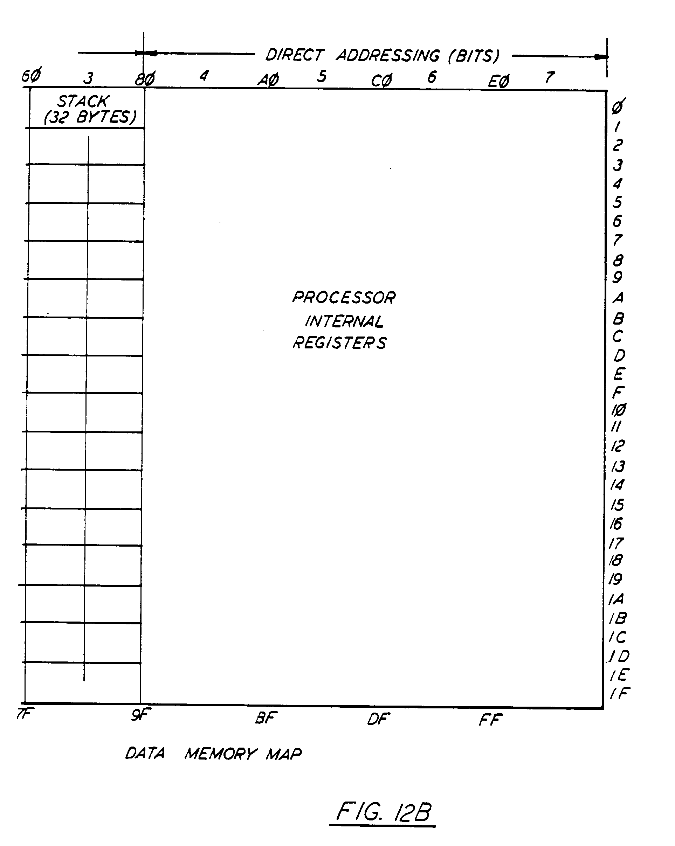

The TIC functions are those which are performed every 50 milliseconds, and include the performance of the sequencing (SEQ) program. The first function performed is that of resetting the watchdog timer as shown at 500, because if this timer is not reset in time, all valve outputs will be disabled as described with reference to FIG. 8. Next, all tick timers (TTM) are decremented at 510, their counts stored at 512, and their corresponding status flags set or cleared at 520 in register TCFL 208 of FIG. 12. The setting of the timeout flags in the TCFL register 208 (See FIG. 12) also requires that the status of a corresponding bit be determined in the Timer Counter Enable Register (TCEN) 207 by the sequencing program, as shown. In this way, if the corresponding TCEN bit is not set, this informs the controller not to enter an ABORT state when the timer flag comes on. For example, when the sterilization timer times out (approximately after 4 hours), an ABORT state should not be entered. For valve time-outs however, it is desired to abort if the timer times out and the valve has not opened or closed in time, and accordingly, the corresponding TCEN bit will be set by the sequencing program, thus allowing an alarm to be generated. If the valve closes in time, its corresponding TCEN bit will be disabled, and no alarm will be generated. Once the TICK timers have been decremented, the main sequencing logic 515 (SEQ), which controls the progression from one state to the next described hereinabove, is performed until it cannot progress further, due to a hold for a

15

specified status condition not yet present. Then, the outputs are loaded into the contact output image in memory (CCO) at 530, e.g., the output data for the appropriate valves or heater to be controlled are stored in memory. Then, the TMRO program subroutine WCO will write the output images to the controlled devices on its next pass. The TIC function program is summarized in the flowchart of FIG. 13.

### 3. Second Timer (T1S)

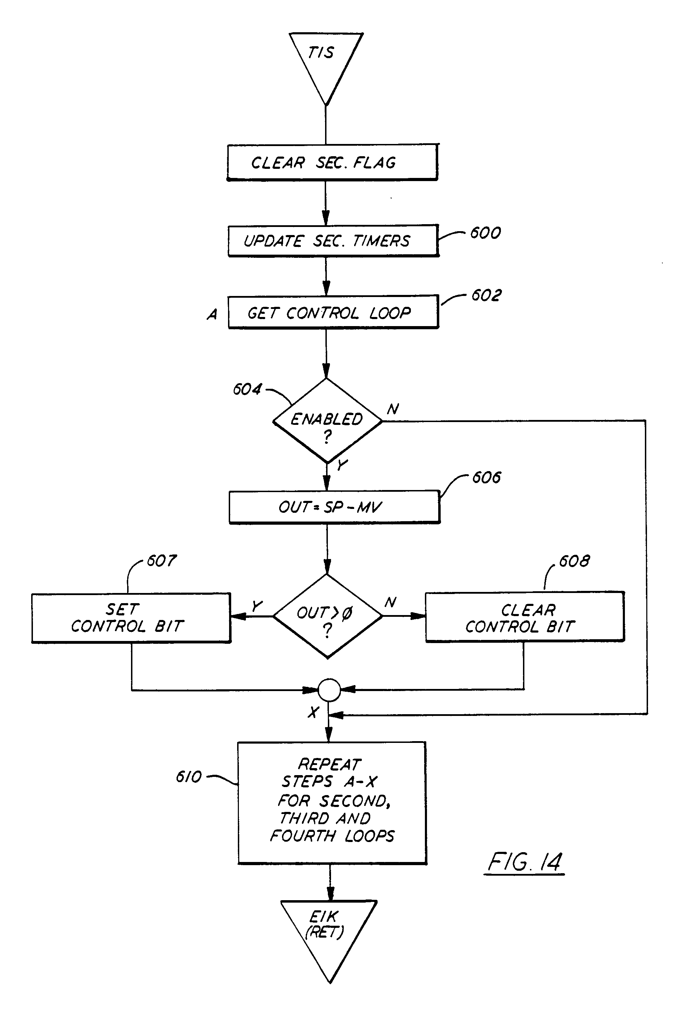

Every second all one-second timers are decremented at step 550, the count stored at 552, and their corresponding status bits set or cleared (555). This includes the setting of flag bits (TCFL) and appropriate Timer Counter Enable bits (TCEN) depending on whether an ABORT is to occur at the occurrence of the timer flag. Finally, the control program 559 (CTR), accepting setpoints (557) from the sequencing program 515, loads the new output status for the controlled devices into the CONTROL register for subsequent loading into the contact output registers of internal RAM. During the next pass through the TMRO program, these outputs are fed to the controlled devices. As shown in FIG. 8, the timed functions occur in the order MIN, SEC and TICK. A flowchart for the one second program, T1S, is shown in FIG. 14.

As shown in FIG. 14, the first function for the one second timer program includes the clearing of the one second flag (SECF) in the STATUS register (see FIG. 12) . All one second timers are then decremented, as shown in FIG. 23 and at 600 in FIG. 14. Program TIS then obtains the loop status from the sequencing program at 602, and determines if the corresponding control bit in the CONTROL register 206 for the particular loop has been enabled at 604. Each loop corresponds to one of the four measured analog process variables, pressure, temperature, humidity and gas concentration. This is also shown in FIG. 24. As indicated, the lower four bits of the CONTROL register 206 correspond to the status of the four loops. If the loop is enabled, a value is determined by subtracting a measured input value, e.g., gas concentration or pressure, from a stored set point value from the sequencing program, as shown at 606. If this value is greater than 0, a corresponding one of the four upper bits in the CTRL register is set at 607. If the CTRL register bit is 0, then the corresponding CONTROL register bit is cleared, as shown at 608.

At 610, the program gets the next loop and repeats steps A-X for that loop. Then the next two loops are obtained and steps A-X repeated sequentially for those two loops. When all four loops have been performed, a return is made to the main dispatching program.

The interrelationship between the analog input data, set points, control register, control program (CTR), output loading program (CTL) and contact outputs CCO are shown in FIG. 24. As shown, program CTR retrieves analog input data ADI, setpoints SP and the control register (CTRL) status from memory. The new status for the control register is then determined in accordance with the flowchart of FIGS. 14 and the new status loaded into the CTRL register. Program CTL then loads the appropriate outputs for controlling the valves and heater into the appropriate contact output register in memory. During the TMRO program these outputs are then coupled to the controlled devices by the program WCO. See FIGS. 10 and 20.

### 4. Minute Timer (T1M)

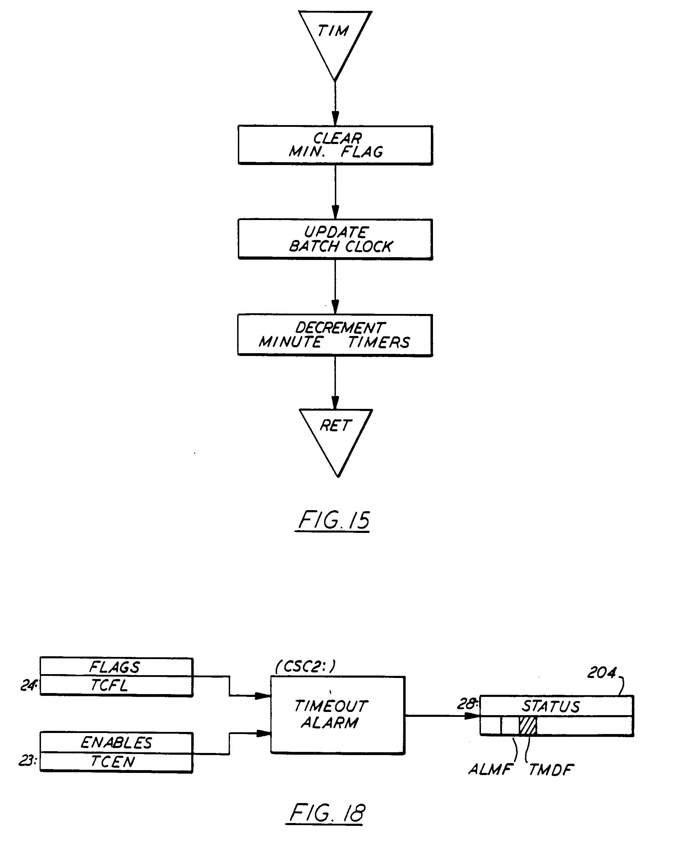

At one-minute intervals, an optional batch time clock 119 may be updated as shown at 610. This clock may be used to initiate the display of process conditions by an appropriate printing or display device. All one-minute timers are decremented at 620, and their corresponding status bits are set or cleared at 630. The T1M program is summarized in the flowchart of FIG. 15.

A sample listing of the software for the gas sterilant system according to the invention is appended below.

```
$TITLE(PROGRAM FOR SC1 STERILIZATION CONTROLLER)
;
;
;***********************************************************************
;*          CONSTANT DEFINITIONS
;*
;***********************************************************************
MCHAN   EQU     07H                     ;MAX A/D CHAN NUMBER
CHMSK   EQU     07H                     ;A/D CHANNEL MASK
BNK0    EQU     00H                     ;RB0
BNK1    EQU     08H                     ;RB1
BNK2    EQU     10H                     ;RB2
BNK3    EQU     18H                     ;RB3
STATE   EQU     R6                      ;CURRENT STATE
ABORT   EQU     R7                      ;ABORT STATE
SSTA    EQU     0                       ;SRAM OFFSET FOR STATE
SABO    EQU     2                       ;SRAM OFFSET FOR ABORT
SCNT    EQU     4                       ;SRAM OFFSET FOR COUNT
SMAX    EQU     38                      ;MAX. VALID STATE
VDLY    EQU     8                       ;VALVE DELAY (400 MSEC.)
HDLY    EQU     2                       ;HEATER DELAY (2 MIN)
TVAC    EQU     30                      ;EVAC TIME (30 MIN)
LKHT    EQU     5                       ;LEAK HOLD TIME (5 MIN)
PVAC    EQU     242                     ;EVAC PRESSURE (95% FS)
PRLK    EQU     223                     ;PRESS. LEAK LIM. (80% FS)
HUMT    EQU     30                      ;HUMIDIF. TIME (30 MIN.)
HNOM    EQU     207                     ;NOM. HUM. LEVEL (81% FS)
HUMH    EQU     90                      ;HUM. HOLD TIME (90 MIN.)
TLOW    EQU     0                       ;MIN. STERIL. TEMP.(0% FS)
TMAX    EQU     255                     ;MAX. STERIL. TEMP.(100%)
CNCT    EQU     15                      ;CONC. TIME (15 MIN.)
CNOM    EQU     64                      ;NOM. STERIL. CONC.
CONH    EQU     100                     ;GAS HOLD TIME (100 MIN)
TSTR    EQU     200                     ;STERIL. TIME (200 MIN)
TEVC    EQU     30                      ;EVAC. TIME (30 MIN.)
PN2T    EQU     15                      ;N2 PRESS. TIME (15 MIN)
DSRB    EQU     30                      ;DESORB. TIME (30 MIN)
TLGH    EQU     15                      ;LOW GAS HOLD TIME (15)
CNTM    EQU     5                       ;MIN. NO. OF PURGE CYCLES
CMIN    EQU     25                      ;MIN. CONCENTRATION (10%)
PATM    EQU     12                      ;ATM PRESS. (5% FS)
PMAX    EQU     28                      ;MAX OPER. PRESS. (11% FS)
TDMP    EQU     15                      ;DUMP HOLD TIME (15 MIN)
PSP1    EQU     60                      ;PRESSURE SETPOINT
TSP1    EQU     60                      ;TEMPERATURE SETPOINT
HSP1    EQU     60                      ;HUMIDITY SETPOINT
CSP1    EQU     60                      ;CONCENTRATION SETPOINT
;
;
```

```
;*************************************************************
;*          EXTERNAL DEVICE ADDRESSES
;*
;*************************************************************
;*          EXTERNAL SHADOW RAM
SRAM      XDATA      2000H                ;SHADOW RAM ADDRESS
;
;*          ANALOG INPUTS
IN0       XDATA      6000H                ;CHAN-0 ADDRESS (PRESS.)
IN1       XDATA      6001H                ;CHAN-1 ADDRESS (TEMP.)
IN2       XDATA      6002H                ;CHAN-2 ADDRESS (HUM.)
IN3       XDATA      6003H                ;CHAN-3 ADDRESS (CONC.)
IN4       XDATA      6004H                ;CHAN-4 ADDRESS
IN5       XDATA      6005H                ;CHAN-5 ADDRESS
IN6       XDATA      6006H                ;CHAN-6 ADDRESS
IN7       XDATA      6007H                ;CHAN-7 ADDRESS
;
;*          CLOCK PORT
CLK       XDATA      4000H                ;CLOCK ADDRESS
;
;*          CONTACT INPUTS
X0        XDATA      0C000H               ;CCI-0  ADDRESS
X1        XDATA      0C001H               ;CCI-1  ADDRESS
X2        XDATA      0C002H               ;CCI-2  ADDRESS
X3        XDATA      0C003H               ;CCI-3  ADDRESS
;
;*          SWITCHES
SW1       XDATA      0C004H               ;SWITCH ADDRESS
;
;*          CONTACT OUTPUTS
Y0        XDATA      0E000H               ;CCO-0  ADDRESS
Y1        XDATA      0E001H               ;CCO-1  ADDRESS
Y2        XDATA      0E002H               ;CCO-2  ADDRESS
Y3        XDATA      0E003H               ;CCO-3  ADDRESS
;
;*          WATCHDOG TIMER
WDT       XDATA      0E004H               ;WATCHDOG RESET ADDRESS
```

18

```
;*******************************************************************
;*        DATA-BASE ALLOCATIONS
;*
;*******************************************************************
        DSEG
        ORG     05H             ;TIME COUNTERS
TICK    DS      1               ;TICK COUNT
TSEC    DS      1               ;SEC. COUNT
TMIN    DS      1               ;MIN. COUNT
        ORG     0CH             ;SIO BUFFER POINTERS
RPUT    DS      1               ;RCV PUT POINTER
RTAK    DS      1               ;RCV TAKE POINTER
TPUT    DS      1               ;XMT PUT POINTER
TTAK    DS      1               ;XMT TAKE POINTER
        ORG     18H             ;TIC TIMERS
TTM0    DS      1               ;TTIMER-0
TTM1    DS      1               ;TTIMER-1
        ORG     1AH             ;SECOND TIMERS
STM0    DS      1               ;STIMER-0
STM1    DS      1               ;STIMER-1
        ORG     1CH             ;MINUTE TIMERS
MTM0    DS      1               ;MTIMER-0
MTM1    DS      1               ;MTIMER-1
        ORG     1EH             ;COUNTERS
CNT0    DS      1               ;COUNTR-0
CNT1    DS      1               ;COUNTR-1
        BSEG
        ORG     20H             ;INTERNAL BIT SPACE
STAT    DATA    20H             ;STATUS BYTE
CTRL    DATA    21H             ;CONTROL BYTE
TCEN    DATA    22H             ;TIMER/COUNTER ENABLES
TCFL    DATA    23H             ;TIMER/COUNTER FLAGS
MSK0    DATA    24H             ;OUTPUT MASK REGISTER
MSK1    DATA    25H             ;OUTPUT MASK REGISTER
MSK2    DATA    26H             ;OUTPUT MASK REGISTER
MSK3    DATA    27H             ;OUTPUT MASK REGISTER
        ORG     28H             ;IMAGED I/O BITS
CCO0    DATA    28H             ;OUTPUT PORT 1
CCO1    DATA    29H             ;OUTPUT PORT 1
CCO2    DATA    2AH             ;OUTPUT PORT 2
CCO3    DATA    2BH             ;OUTPUT PORT 3
CCI0    DATA    2CH             ;INPUT PORT 0
CCI1    DATA    2DH             ;INPUT PORT 1
CCI2    DATA    2EH             ;INPUT PORT 2
CCI3    DATA    2FH             ;INPUT PORT 3
        DSEG
        ORG     30H             ;ANALOG DATA IMAGE
ADI0    DS      1               ;PRESS. INPUT
ADI1    DS      1               ;TEMP. INPUT
ADI2    DS      1               ;HUM. INPUT
ADI3    DS      1               ;CONC. INPUT
ADI4    DS      1               ;CHANNEL 4 INPUT
ADI5    DS      1               ;CHANNEL 5 INPUT
ADI6    DS      1               ;CHANNEL 6 INPUT
ADI7    DS      1               ;CHANNEL 7 INPUT
```

19

```
                ORG         38H         ;INTERNAL DATA AREA
        STP0    DS          1           ;PRESS. SETPOINT
        STP1    DS          1           ;TEMP. SETPOINT
        STP2    DS          1           ;HUM. SETPOINT
        STP3    DS          1           ;CONC. SETPOINT
                ORG         3CH         ;BATCH TIME CLOCK
        TIME    DS          1           ;BATCH TIME
        ;

;*****************************************************************
;*          DATA DEFINITIONS
;*
;*          [STATUS & CONTROL]
;*
;*****************************************************************
;           STATUS
TICF    BIT         STAT.0          ;TICK FLAG
SECF    BIT         STAT.1          ;SECOND FLAG
MINF    BIT         STAT.3          ;MINUTE FLAG
RCVF    BIT         STAT.4          ;RCV FLAG
XMTF    BIT         STAT.5          ;XMT FLAG
TMOF    BIT         STAT.6          ;TIMEOUT FLAG
ALMF    BIT         STAT.7          ;ALARM FLAG
;
;           CTRL
CEN0    BIT         CTRL.0          ;PRESS. LOOP ENABLE
CEN1    BIT         CTRL.1          ;TEMP. LOOP ENABLE
CEN2    BIT         CTRL.2          ;HUM. LOOP ENABLE
CEN3    BIT         CTRL.3          ;CONC. LOOP ENABLE
CTR0    BIT         CTRL.4          ;PRESS. LOOP OUTPUT
CTR1    BIT         CTRL.5          ;TEMP. LOOP OUTPUT
CTR2    BIT         CTRL.6          ;HUM. LOOP OUTPUT
CTR3    BIT         CTRL.7          ;CONC. LOOP OUTPUT
;           TCEN
TEN0    BIT         TCEN.0          ;TT0 ENABLE
TEN1    BIT         TCEN.1          ;TT1 ENABLE
TEN2    BIT         TCEN.2          ;ST0 ENABLE
TEN3    BIT         TCEN.3          ;ST1 ENABLE
TEN4    BIT         TCEN.4          ;MT0 ENABLE
TEN5    BIT         TCEN.5          ;MT1 ENABLE
TEN6    BIT         TCEN.6          ;MT2 ENABLE
TEN7    BIT         TCEN.7          ;MT3 ENABLE
;
;           TCFL
TFL0    BIT         TCFL.0          ;TT0 TIMEOUT
TFL1    BIT         TCFL.1          ;TT1 TIMEOUT
TFL2    BIT         TCFL.2          ;ST0 TIMEOUT
TFL3    BIT         TCFL.3          ;ST1 TIMEOUT
TFL4    BIT         TCFL.4          ;MT0 TIMEOUT
TFL5    BIT         TCFL.5          ;MT1 TIMEOUT
TFL6    BIT         TCFL.6          ;CT0 UNDERFLOW
TFL7    BIT         TCFL.7          ;CT1 UNDERFLOW
;
```

```
;**************************************************************
;*        DATA DEFINITIONS
;*
;*        [OUTPUT PORTS]
;*
;**************************************************************
;            OPORT_0
;                                              ;DOOR-OPEN
LT01     BIT        CC00.0                     ;DOOR-OPEN
LT02     BIT        CC00.1                     ;EVAC-FAIL
LT03     BIT        CC00.2                     ;FILL-FAIL
LT04     BIT        CC00.3                     ;STERIL-FAIL
LT05     BIT        CC00.4                     ;PURGE-FAIL
LT06     BIT        CC00.5                     ;LOAD-UNSTERILE
LT07     BIT        CC00.6                     ;SPARE
LT08     BIT        CC00.7                     ;TEST-FAIL
;
;            OPORT-1
LT11     BIT        CC01.0                     ;READY-FOR-CYCLE
LT12     BIT        CC01.1                     ;CYCLE-IN-PROGRESS
LT13     BIT        CC01.2                     ;EVAC-IN-PROGRESS
LT14     BIT        CC01.3                     ;FILL-IN-PROGRESS
LT15     BIT        CC01.4                     ;STERIL-IN-PROGRESS
LT16     BIT        ·CC01.5                    ;PURGE-IN-PROGRESS
LT17     BIT        CC01.6                     ;REMOVE-LOAD
LT18     BIT        CC01.7                     ;SPARE
;
;            OPORT_2
VV01     BIT        CC02.0                     ;OPEN-MAIN-VAC-VALVE
VV02     BIT        CC02.1                     ;OPEN-MAIN-GAS-VALVE
VV03     BIT        CC02.2                     ;OPEN-MAIN-DUMP-VALVE
VV04     BIT        CC02.3                     ;OPEN-GAS-CTRL-VALVE
VV05     BIT        CC02.4                     ;OPEN-N2-CTRL-VALVE
VV06     BIT        CC02.5                     ;OPEN-STEAM-CTRL-VALVE
VV07     BIT        CC02.6                     ;OPEN-BREAK-VALVE
VV08     BIT        CC02.7                     ;OPEN-DUMP-VAC-VALVE
;
;            OPORT_3
PP01     BIT        CC03.0                     ;TURN-P1-ON
HT01     BIT        CC03.1                     ;TURN-H1-ON
SPR1     BIT        CC03.2                     ;SPARE
SPR2     BIT        CC03.3                     ;SPARE
SPR3     BIT        CC03.4                     ;SPARE
SPR4     BIT        CC03.5                     ;SPARE
ADZC     BIT        CC03.6                     ;A/D ZERO CALIB.
LGG1     BIT        CC03.7                     ;CONC. HIGH GAIN SWITCH
```

```
;**********************************************************************
;*
;*        DATA DEFINITIONS
;*
;*        [INPUT PORTS]
;*
;*
;**********************************************************************
;               IPORT_0
LSC1        BIT        CCI0.0        ;V1-CLOSED
LSC2        BIT        CCI0.1        ;V2-CLOSED
LSC3        BIT        CCI0.2        ;V3-CLOSED
LSC4        BIT        CCI0.3        ;V4-CLOSED
LSC5        BIT        CCI0.4        ;V5-CLOSED
LSC6        BIT        CCI0.5        ;V6-CLOSED
LSC7        BIT        CCI0.6        ;V7-CLOSED
LSC8        BIT        CCI0.7        ;V8-CLOSED
;
;               IPORT_1
LSO1        BIT        CCI1.0        ;V1-OPEN
LSO2        BIT        CCI1.1        ;V2-OPEN
LSO3        BIT        CCI1.2        ;V3-OPEN
LSO4        BIT        CCI1.3        ;V4-OPEN
LSO5        BIT        CCI1.4        ;V5-OPEN
LSO6        BIT        CCI1.5        ;V6-OPEN
LSO7        BIT        CCI1.6        ;V7-OPEN
LSO8        BIT        CCI1.7        ;V8-OPEN
;
;               IPORT_2
DSC1        BIT        CCI2.0        ;DOOR-SU-CLOSED
TSC1        BIT        CCI2.1        ;TEMP-SU-CLOSED
SUC1        BIT        CCI2.2        ;MAN-SU1-CLOSED
SUC2        BIT        CCI2.3        ;MAN-SU2-CLOSED
SIO1        BIT        CCI2.4        ;SPARE
SIO2        BIT        CCI2.5        ;SPARE
SIO3        BIT        CCI2.6        ;SPARE
SIO4        BIT        CCI2.7        ;SPARE
;
;               IPORT_3
SIO5        BIT        CCI3.0        ;SPARE
SIO6        BIT        CCI3.1        ;SPARE
SIO7        BIT        CCI3.2        ;SPARE
SIO8        BIT        CCI3.3        ;SPARE
SIO9        BIT        CCI3.4        ;SPARE
SI10        BIT        CCI3.5        ;SPARE
SI11        BIT        CCI3.6        ;SPARE
SI12        BIT        CCI3.7        ;SPARE
;
```

```
;************************************************************
;*          MASK BIT DEFINITIONS
;*
;************************************************************
;          MASK-REG-0
MVC1      BIT       MSK0.0        ;V1-CLOSED-MASK
MVC2      BIT       MSK0.1        ;V2-CLOSED-MASK
MVC3      BIT       MSK0.2        ;V3-CLOSED-MASK
MVC4      BIT       MSK0.3        ;V4-CLOSED-MASK
MVC5      BIT       MSK0.4        ;V5-CLOSED-MASK
MVC6      BIT       MSK0.5        ;V6-CLOSED-MASK
MVC7      BIT       MSK0.6        ;V7-CLOSED-MASK
MVC8      BIT       MSK0.7        ;V8-CLOSED-MASK
;
;          MASK-REG-1
MVO1      BIT       MSK1.0        ;V1-OPEN-MASK
MVO2      BIT       MSK1.1        ;V2-OPEN-MASK
MVO3      BIT       MSK1.2        ;V3-OPEN-MASK
MVO4      BIT       MSK1.3        ;V4-OPEN-MASK
MVO5      BIT       MSK1.4        ;V5-OPEN-MASK
MVO6      BIT       MSK1.5        ;V6-OPEN-MASK
MVO7      BIT       MSK1.6        ;V7-OPEN-MASK
MVO8      BIT       MSK1.7        ;V8-OPEN-MASK
;
;          MASK-REG-2
MDC1      BIT       MSK2.0        ;DS-CLOSED-MASK
MTC1      BIT       MSK2.1        ;TS-CLOSED-MASK
MSC1      BIT       MSK2.2        ;SW1-CLOSED-MASK
MSC2      BIT       MSK2.3        ;SW2-CLOSED-MASK
;
```

```
;*********************************************************************
;*          INTERRUPT VECTORS
;*
;*********************************************************************
          CSEG
          ORG       0000H
RSTV:     LJMP      INIT                    ;RESET VECTOR
;
          ORG       000BH
TINT:     LJMP      TMR0                    ;TIMER0 VECTOR
;
          ORG       0013H
PINT:     LJMP      PURF                    ;PWR FAIL VECTOR
;
          ORG       001BH
TM1V:     RETI                              ;TIMER1 VECTOR
;
          ORG       0023H
SIOV:     LJMP      SIOHND                  ;SERIAL DATA VECTOR
;
;*********************************************************************
;          POWER FAIL HANDLER
;
;*********************************************************************
PURF:     CLR       P1.6                    ;STORE SRAM DATA
          SETB      P1.6                    ;DISABLE STORE
          RETI                              ;INTERRUPT RETURN
;



;*********************************************************************
;*          TIMER INTERRUPT HANDLER
;*
;*********************************************************************
          ORG       0030H
TMR0:     PUSH      PSW                     ;SAVE PROC. STATUS
          PUSH      ACC                     ;SAVE ACCUMULATOR
          PUSH      DPL                     ;SAVE DP(L)
          PUSH      DPH                     ;SAVE DP(H)
          MOV       PSW,#BNK0               ;USE RB0
          CLR       EA                      ;DISABLE INTERRUPTS
          ACALL     RRT                     ;RESET AND RESTART TIMERS
          ACALL     RCI                     ;READ CONTACT INPUTS
          ACALL     CSC                     ;CONTACT STATUS CHECK
          ACALL     WCO                     ;WRITE CONTACT OUTPUTS
          ACALL     RAI                     ;READ ANALOG INPUTS
          SETB      EA                      ;RESTORE INTERRUPTS
TRTN:     POP       DPH                     ;RESTORE DP(H)
          POP       DPL                     ;RESTORE DP(L)
          POP       ACC                     ;RESTORE ACCUMULATOR
          POP       PSW                     ;RESTORE PROC. STATUS
          RETI                              ;RETURN FROM TIMER0 INT.
;
```

24

```
;**********************************************************************
;*        TMR0 SUBROUTINES
;*
;**********************************************************************
RRT:      CLR     TR0                         ;STOP TIMER0
          MOV     A,#LOW(-3120+7)             ;LOAD COUNT(L)
          ADD     A,TL0                       ;CORRECT FOR OVERRUN
          MOV     TL0,A                       ;RELOAD COUNTER(L)
          MOV     A,#HIGH(-3120+7)            ;REPEAT FOR COUNT(H)
          ADDC    A,TH0                       ;GET CORRECTED HIGH BYTE
          MOV     TH0,A                       ;LOAD COUNTER(H)
          SETB    TR0                         ;RESTART TIMER
CLOCK:    DJNZ    TICK,CLK3                   ;IF 50-MSEC
          MOV     TICK,#8                     ;  RELOAD TICK COUNT
          SETB    TICF                        ;  SET 50-MSEC FLAG
          DJNZ    TSEC,CLK2                   ;  IF 1-SEC
          MOV     TSEC,#20                    ;    RELOAD TSEC COUNT
          SETB    SECF                        ;    SET 1-SEC FLAG
          DJNZ    TMIN,CLK1                   ;    IF 1-MIN
          MOV     TMIN,#60                    ;      RELOAD TMIN COUNT
          SETB    MINF                        ;      SET 1-MIN FLAG
          SJMP    CLK4                        ;      END
CLK1:     CLR     MINF                        ;    ELSE, CLR MIN. FLAG
          SJMP    CLK4                        ;    END
CLK2:     CLR     SECF                        ;  ELSE, CLR SEC. FLAG
          SJMP    CLK4                        ;  END
CLK3:     CLR     TICF                        ;ELSE, CLR TIC. FLAG
CLK4:     NOP                                 ;  END
          RET                                 ;RETURN FROM TIMER PROG.
```

```
RCI:    MOV     DPTR,#X0        ;POINT CONTACT INPUTS
        MOV     R0,#CCI0        ;POINT DATA-BASE IMAGE
        MOV     R4,#4           ;FOR R4:=4 DOWNTO 0 DO
CI1:    CLR     P1.4            ;   ENABLE I/O
        MOVX    A,@DPTR         ;   GET INPUT
        SETB    P1.4            ;   DISABLE I/O
        MOV     @R0,A           ;   STORE IT IN DATA-BASE
        INC     DPTR            ;   POINT NEXT INPUT
        INC     R0              ;   POINT NEXT STORAGE
        DJNZ    R4,CI1          ;   END
        RET                     ;RETURN
;
CSC:    CLR     ALMF            ;CLEAR ALARM FLAG
        MOV     A,CCO2          ;GET VALVE OUTPUTS
        XRL     A,CCI1          ;COMPARE WITH LSO INPUTS
        ANL     A,MSK1          ;MASK OFF VO DON'T CARES
        MOV     R2,A            ;SAVE PARTIAL RESULT
        MOV     A,CCO2          ;GET VALVE OUTPUTS
        CPL     A               ;MAKE CLOSED NORMAL
        XRL     A,CCI0          ;COMPARE WITH LSC INPUTS
        ANL     A,MSK0          ;MASK OFF VC DON'T CARES
        ORL     A,R2            ;ADD PREV. RESULT
        JZ      CSC2            ;   IF MISMATCH
        SETB    ALMF            ;      SET ALARM FLAG
CSC2:   NOP                     ;      END
        MOV     A,TCFL          ;GET TIMEOUTS
        ANL     A,TCEN          ;TEST IF ENABLED
        JZ      CSC4            ;IF (TMO.AND.TEN)
        SETB    TMOF            ;   SET TIMEOUT FLAG
        SJMP    CSC5            ;   END
CSC4:   CLR     TMOF            ;ELSE, CLEAR TIMEOUT FLAG
CSC5:   NOP                     ;   END
        RET                     ;RETURN
;
UCO:    MOV     DPTR,#Y0        ;POINT CONTACT OUTPUTS
        MOV     R0,#CCO0        ;POINT DATA-BASE IMAGE
        MOV     R4,#4           ;FOR R4:=4 DOWNTO 0 DO
CO1:    MOV     A,@R0           ;   GET OUTPUT DATA
        CPL     A               ;   INVERT IT FOR OUTPUT
        CLR     P1.4            ;   ENABLE I/O
        MOVX    @DPTR,A         ;   LOAD OUTPUT LATCH
        SETB    P1.4            ;   DISABLE I/O
        INC     DPTR            ;   POINT NEXT OUTPUT
        INC     R0              ;   POINT NEXT DATA
        DJNZ    R4,CO1          ;   END
        RET                     ;RETURN
```

```
RAI:      MOV      DPTR,#IN0      ;POINT FIRST ANALOG CHAN.
          MOV      R0,#ADI0       ;POINT FIRST ANALOG DATA
          MOV      R4,#8          ;FOR R4:=8 DOWNTO 0 DO
RA1:      CLR      P1.4           ;   ENABLE I/O
          MOVX     A,@DPTR        ;   GET ANALOG DATA
          SETB     P1.4           ;   DISABLE I/O
          ACALL    FILTER         ;   FILTER ANALOG DATA
          MOV      @R0,A          ;   LOAD IT INTO DATA BASE
          INC      DPTR           ;   POINT NEXT CHANNEL
          INC      R0             ;   POINT NEXT DATA
          DJNZ     R4,RA1         ;   END
          RET                     ;RETURN
;
FILTER:   MOV      B,#020H        ;LOAD FILT. CONST. CB
          MUL      AB             ;B,A:=0.125*R(I)
          PUSH     B              ;SAVE PROD(H)
          PUSH     ACC            ;SAVE PROD(L)
          MOV      B,#0E0H         ;LOAD (1-CB) CONST.
          MOV      A,@R0          ;GET X(I-1)
          MUL      AB             ;B,A:=0.875*X(I-1)
          MOV      R2,B           ;SAVE HIGH BYTE
          POP      B              ;LOAD PROD(L)
          ADD      A,B            ;ADD LOW BYTES
          XCH      A,R2           ;GET HIGH BYTE
          POP      B              ;LOAD PROD(H)
          ADDC     A,B            ;A,R2 IS FILTERED DATA
          RET                     ;RETURN
;


;**********************************************************************
;          SCHEDULED TIME FUNCTIONS
;
;**********************************************************************
T50:      CLR      TICF           ;CLEAR TICK FLAG
          MOV      PSW,#BNK2      ;USE RB2
          ACALL    RWT            ;RESET WATCHDOG TIMER
          ACALL    DTT            ;DECREMENT TICK TIMERS
          ACALL    SEQ            ;PERFORM SEQUENCE LOGIC
          ACALL    CTL            ;LOAD CONTROL OUTPUTS
          RET                     ;RETURN TO DISPATCHING
;
T1K:      CLR      SECF           ;CLEAR 1-SEC FLAG
          MOV      PSW,#BNK2       ;USE RB2
          ACALL    DST            ;DECREMENT SECOND TIMERS
          ACALL    CTR            ;PERFORM CONTROL ACTIONS
          RET
;
T1M:      CLR      MINF           ;CLEAR 1-MIN FLAG
          MOV      PSW,#BNK2      ;USE RB2
          ACALL    UBC            ;UPDATE BATCH CLOCK
          ACALL    DMT            ;DECREMENT MINUTE TIMERS
          RET
;
```

27

```
RWT:    MOV     DPTR,#WDT       ;POINT WATCHDOG TIMER
        CLR     A               ;CLEAR ACCUMULATOR
        CLR     P1.4            ;ENABLE I/O
        MOVX    @DPTR,A         ;RESET WATCHDOG TIMER
        SETB    P1.4            ;DISABLE I/O
        RET
;
WBC:    MOV     R0,#TIME        ;POINT TIME(L)
        CLR     C               ;CLEAR CARRY
        XCH     A,@R0           ;GET TIME(L)
        INC     A               ;INCREMENT IT
        XCH     A,@R0           ;UPDATE TIME(L)
        INC     R0              ;POINT TIME(H)
        XCH     A,@R0           ;GET TIME(H)
        ADDC    A,#0            ;PROPAGATE CARRY
        XCH     A,@R0           ;UPDATE TIME(H)
        RET
;
```

28

```
;****************************************************************
;*          CONTROL CALCULATIONS
;*
;*
;****************************************************************
CTR:      MOV       R0,#STP0              ;POINT SETPOINT
          MOV       R1,#ADI0              ;POINT DATA
          CLR       C                     ;CLEAR CARRY
          MOV       A,@R0                 ;GET PRESS. SETPOINT
          SUBB      A,@R1                 ;SUBTRACT MEAS. PRESS.
          JNC       CT2                   ;IF MV>SP
          SETB      CTR0                  ;   INCREASE OUTPUT
          SJMP      CT3                   ;   END
CT2:      CLR       CTR0                  ;ELSE, DECR. OUTPUT
CT3:      NOP                             ;   END
          INC       R0                    ;POINT NEXT SETPOINT
          INC       R1                    ;POINT NEXT MEASUREMENT
          CLR       C                     ;CLEAR CARRY
          MOV       A,@R0                 ;GET TEMP. SETPOINT
          SUBB      A,@R1                 ;SUBTRACT MEAS. TEMP.
          JNC       CT4                   ;IF MV>SP
          CLR       CTR1                  ;   DECREASE OUTPUT
          SJMP      CT5                   ;   END
CT4:      SETB      CTR1                  ;ELSE, INCR. OUTPUT
CT5:      NOP                             ;   END
          INC       R0                    ;POINT NEXT SETPOINT
          INC       R1                    ;POINT NEXT MEASUREMENT
          CLR       C                     ;CLEAR CARRY
          MOV       A,@R0                 ;GET HUM. SETPOINT
          SUBB      A,@R1                 ;SUBTRACT HUM. MEAS.
          JNC       CT6                   ;IF MV>SP
          CLR       CTR2                  ;   DECREASE OUTPUT
          SJMP      CT7                   ;   END
CT6:      SETB      CTR2                  ;ELSE, INCREASE OUTPUT
CT7:      NOP                             ;   END
          INC       R0                    ;POINT NEXT SETPOINT
          INC       R1                    ;POINT NEXT MEASUREMENT
          CLR       C                     ;CLEAR CARRY
          MOV       A,@R0                 ;GET CONC. SETPOINT
          SUBB      A,@R1                 ;SUBTRACT CONC. MEAS.
          JNC       CT8                   ;IF MV>SP
          CLR       CTR3                  ;   DECREASE OUTPUT
          SJMP      CT9                   ;   END
CT8:      SETB      CTR3                  ;ELSE, INCR. OUTPUT
CT9:      NOP                             ;   END
          RET                             ;RETURN
;
```

```
;********************************************************************
;*        SOFTWARE TICK TIMERS (50 MSEC)
;*
;********************************************************************
DTT:    MOV     R0,#TTM0        ;POINT FIRST TICK TIMER
        MOV     A,@R0           ;GET LAST COUNT
        JZ      TT1             ;IF COUNT<>0
        DEC     A               ;  DECREMENT ACC.
        MOV     @R0,A           ;  UPDATE COUNT
        JZ      TT1             ;  IF NOT TIMEOUT
        CLR     TFL0            ;    CLEAR FLAG
        SJMP    TT2             ;    END
TT1:    SETB    TFL0            ;  ELSE, SET FLAG
TT2:    NOP                     ;  END
;
        MOV     R0,#TTM1        ;POINT SECOND TICK TIMER
        MOV     A,@R0           ;GET LAST COUNT
        JZ      TT4             ;IF COUNT<>0
        DEC     A               ;  DECREMENT ACC.
        MOV     @R0,A           ;  UPDATE COUNT
        JZ      TT4             ;  IF NOT TIMEOUT
        CLR     TFL1            ;    CLEAR FLAG
        SJMP    TT5             ;    END
TT4:    SETB    TFL1            ;  ELSE, SET FLAG
TT5:    NOP                     ;  END
        RET                     ;RETURN
;
;********************************************************************
;*        SOFTWARE SECOND TIMERS
;*
;********************************************************************
DST:    MOV     R0,#STM0        ;POINT FIRST SEC. TIMER
        MOV     A,@R0           ;GET LAST COUNT
        JZ      ST1             ;IF COUNT<>0
        DEC     A               ;  DECREMENT ACC.
        MOV     @R0,A           ;  UPDATE COUNT
        JZ      ST1             ;  IF NOT TIMEOUT
        CLR     TFL2            ;    CLEAR FLAG
        SJMP    ST2             ;    END
ST1:    SETB    TFL2            ;  ELSE, SET FLAG
ST2:    NOP                     ;  END
;
        MOV     R0,#STM1        ;POINT NEXT SECOND TIMER
        MOV     A,@R0           ;GET LAST COUNT
        JZ      ST4             ;IF COUNT<>0
        DEC     A               ;  DECREMENT ACC.
        MOV     @R0,A           ;  UPDATE COUNT
        JZ      ST4             ;  IF NOT TIMEOUT
        CLR     TFL3            ;    CLEAR FLAG
        SJMP    ST5             ;    END
ST4:    SETB    TFL3            ;  ELSE, SET FLAG
ST5:    NOP                     ;  END
        RET                     ;RETURN
;
```

```
;*******************************************************************
;*          SOFTWARE MINUTE TIMERS
;*
;*******************************************************************
DMT:    MOV     R0,#MTM0        ;POINT FIRST MIN. TIMER
        MOV     A,@R0           ;GET LAST COUNT
        JZ      MT1             ;IF COUNT<>0
        DEC     A               ;   DECREMENT ACC.
        MOV     @R0,A           ;   UPDATE COUNT
        JZ      MT1             ;   IF NOT TIMEOUT
        CLR     TFL4            ;     CLEAR FLAG
        SJMP    MT2             ;     END
MT1:    SETB    TFL4            ;   ELSE, SET FLAG
MT2:    NOP                     ;   END
;
        MOV     R0,#MTM1        ;POINT SECOND MIN. TIMER
        MOV     A,@R0           ;GET LAST COUNT
        JZ      MT4             ;IF COUNT<>0
        DEC     A               ;   DECREMENT ACC.
        MOV     @R0,A           ;   UPDATE COUNT
        JZ      MT4             ;   IF NOT TIMEOUT
        CLR     TFL5            ;     CLEAR FLAG
        SJMP    MT5             ;     END
MT4:    SETB    TFL5            ;   ELSE, SET FLAG
MT5:    NOP                     ;   END
        RET                     ;RETURN
;*******************************************************************
;*          SOFTWARE COUNTERS
;*
;*******************************************************************
DCT0:   MOV     R0,#CNT0        ;POINT FIRST COUNTER
        MOV     A,@R0           ;GET LAST COUNT
        JZ      DC1             ;IF COUNT<>0
        DEC     A               ;   DECREMENT ACC.
        MOV     @R0,A           ;   UPDATE COUNT
        JZ      DC1             ;   IF NOT ZERO
        CLR     TFL6            ;     CLEAR FLAG
        SJMP    DC2             ;     END
DC1:    SETB    TFL6            ;   ELSE, SET FLAG
DC2:    NOP                     ;   END
        RET                     ;RETURN
;
DCT1:   MOV     R0,#CNT1        ;POINT SECOND COUNTER
        MOV     A,@R0           ;GET LAST COUNT
        JZ      DC3             ;IF COUNT<>0
        DEC     A               ;   DECREMENT ACC.
        MOV     @R0,A           ;   UPDATE COUNT
        JZ      DC3             ;   IF NOT ZERO
        CLR     TFL7            ;     CLEAR FLAG
        SJMP    DC4             ;     END
DC3:    SETB    TFL7            ;   ELSE, SET FLAG
DC4:    NOP                     ;   END
        RET                     ;RETURN
;
```

```
;********************************************************************
;*          CONTROL OUTPUTS
;*
;********************************************************************
CTL:        MOV             C,CTR0                  ;GET OUTPUT-0
            ANL             C,CEN0                  ;ALLOW IF ENABLED
            MOV             VV05,C                  ;OUTPUT TO V5
;
            MOV             C,CTR1                  ;GET OUTPUT-1
            ANL             C,CEN1                  ;ALLOW IF ENABLED
            MOV             HT01,C                  ;OUTPUT TO H1
;
            MOV             C,CTR2                  ;GET OUTPUT-2
            ANL             C,CEN2                  ;ALLOW IF ENABLED
            MOV             VV06,C                  ;OUTPUT TO V6
;
            MOV             C,CTR3                  ;GET OUTPUT-3
            ANL             C,CEN3                  ;ALLOW IF ENABLED
            MOV             VV04,C                  ;OUTPUT TO V4
;
            RET
;
```

```
;************************************************************************
;*        POWER-ON INITIALIZATION
;*
;************************************************************************
INIT:   MOV     SP,#0060H               ;INITIALIZE STACK POINTER
        MOV     PSW,#BNK0               ;USE RB0
        CLR     A                       ;CLEAR ACCUMULATOR
        MOV     R0,#2                   ;POINT LOWEST RAM LOC.
        MOV     R1,#126                 ;FOR R1:=126 DOWNTO 0 DO
ILP:    MOV     @R0,A                   ;   CLEAR MEMORY LOC.
        INC     R0                      ;   POINT NEXT LOCATION
        DJNZ    R1,ILP                  ;   END
        MOV     TICK,#8                 ;INITIALIZE TICK COUNT
        MOV     TSEC,#20                ;INITIALIZE SEC. COUNT
        MOV     TMIN,#60                ;INITIALIZE MIN. COUNT
        MOV     PSW,#BNK1               ;USE RB1
        MOV     RPUT,#40H               ;INITIALIZE RPUT POINTER
        MOV     RTAK,#40H               ;INITIALIZE RTAK POINTER
        MOV     TPUT,#50H               ;INITIALIZE TPUT POINTER
        MOV     TTAK,#50H               ;INITIALIZE TTAK POINTER
        MOV     PSW,#BNK2               ;USE RB2
        MOV     STATE,#0                ;STATE:=0
        MOV     ABORT,#0                ;ABORT:=0
        MOV     SCON,#052H              ;SET SERIAL PORT BITS
        MOV     TMOD,#061H              ;SET TIMER MODES
        MOV     87H,#00H                ;SET SMOD:=0 IN PCON
        MOV     IP,#002H                ;SET INTERRUPT PRIORITIES
        MOV     IE,#096H                ;ENABLE INTERRUPTS
        MOV     TL0,#LOW(-3120)         ;LOAD COUNT(L)
        MOV     TH0,#HIGH(-3120)        ;LOAD COUNT(H)
        MOV     TH1,#-13                ;SET BAUD RATE (1200)
        MOV     A,#0FFH                 ;SET ACCUM. ALL 1'S
        CLR     P1.4                    ;ENABLE I/O
        MOV     DPTR,#Y0                ;POINT Y0 OUTPUTS
        MOVX    @DPTR,A                 ;CLEAR Y0
        MOV     DPTR,#Y1                ;POINT Y1 OUTPUTS
        MOVX    @DPTR,A                 ;CLEAR Y1
        MOV     DPTR,#Y2                ;POINT Y2 OUTPUTS
        MOVX    @DPTR,A                 ;CLEAR Y2
        MOV     DPTR,#Y3                ;POINT Y3 OUTPUTS
        MOVX    @DPTR,A                 ;CLEAR Y3
        SETB    P1.4                    ;DISABLE I/O
        ACALL   RWT                     ;RESET WATCHDOG TIMER
        MOV     TIME,#0                 ;CLEAR TIME(L)
        MOV     TIME+1,#0               ;CLEAR TIME(H)
        SETB    TR1                     ;START BAUD CLOCK
        SETB    TR0                     ;START TIMER
        SJMP    MAIN                    ;START MAIN PROGRAM
;
TEST:   RET                             ;TEST COMPUTER FUNCTIONS
```

```
;***********************************************************************
;*            SEQUENCING PROGRAM
;*
;***********************************************************************
SEQ:     NOP                                    ;REPEAT
         MOV      PSW,#BNK2                      ;   USE RB2
         MOV      A,STATE                        ;   GET CURRENT STATE
         ADD      A,#NOT(SMAX)                   ;   COMPARE MAX. STATE
         JNC      SQ1                            ;   IF INVALID STATE
         MOV      A,#31                          ;     TAKE STATE #31
         MOV      STATE,A                        ;     SET STATE TO #31
         SJMP     SQ2                            ;     END
SQ1:     MOV      A,STATE                        ;   ELSE, USE CURRENT STATE
SQ2:     NOP                                     ;     END
         RL       A                              ;   MAKE IT 4-BYTE-
         RL       A                              ;   ADDRESS OFFSET
         MOV      DPTR,#JMPTBL                   ;   OFFSET IN JUMP TABLE
         JMP      @A+DPTR                        ;   PERFORM STATE
SEQR:    MOV      C,ALMF                         ;   GET ALARM FLAG
         ORL      C,TMOF                         ;   OR WITH TIMEOUT FLAG
         JNC      SQ3                            ;   IF (ALM.OR.TMO)
         MOV      A,ABORT                        ;     GET ABORT STATE
         MOV      STATE,A                        ;     SET STATE TO ABORT
         CLR      F0                             ;     CLEAR HOLD FLAG
SQ3:     NOP                                     ;     END
         JNB      F0,SEQ                         ;UNTIL HOLD
         RET                                     ;RETURN


;***********************************************************************
;*            MAIN DISPATCHING PROGRAM
;*
;***********************************************************************
MAIN:    NOP                                     ;DO FOREVER
         JNB      MINF,MN1                       ;   IF 1-MIN TIME
         LCALL    T1M                            ;     DO 1-MIN FUNCTIONS
MN1:     JNB      SECF,MN2                       ;   IF 1-SEC TIME
         LCALL    T1K                            ;     DO 1-SEC FUNCTIONS
MN2:     JNB      TICF,MN3                       ;   IF TICK TIME
         LCALL    T50                            ;     DO TICK FUNCTIONS
MN3:     JNB      RCVF,MN4                       ;   IF RCV TIME
         LCALL    RCV                            ;     DO RCV FUNCTIONS
MN4:     JNB      XMTF,MN5                       ;   IF XMT TIME
         LCALL    XMT                            ;     DO XMT FUNCTIONS
MN5:     LCALL    TEST                           ;   ELSE, PERFORM TESTS
         SJMP     MAIN                           ;END
;
GTCT:    MOV      A,#1                           ;READ SRAM
         RET
RCV:     CLR      RCVF                           ;RESET RCV FLAG
         RET
XMT:     CLR      XMTF                           ;RESET XMT FLAG
         RET
SIOHND:  RET                                     ;SERIAL I/O HANDLER


$INCLUDE(STATES.SRC)
;
         END
```

EP 0 211 073 B1

```
;*******************************************************************
;*          STATE JUMP TABLE
;*
;*******************************************************************
JMPTBL:   LJMP      STATE0
          DB        0
          LJMP      STATE1
          DB        0
          LJMP      STATE2
          DB        0
          LJMP      STATE3
          DB        0
          LJMP      STATE4
          DB        0
          LJMP      STATE5
          DB        0
          LJMP      STATE6
          DB        0
          LJMP      STATE7
          DB        0
          LJMP      STATE8
          DB        0
          LJMP      STATE9
          DB        0
          LJMP      STATE10
          DB        0
          LJMP      STATE11
          DB        0
          LJMP      STATE12
          DB        0
          LJMP      STATE13
          DB        0
          LJMP      STATE14
          DB        0
          LJMP      STATE15
          DB        0
          LJMP      STATE16
          DB        0
          LJMP      STATE17
          DB        0
          LJMP      STATE18
          DB        0
          LJMP      STATE19
          DB        0
          LJMP      STATE20
          DB        0
          LJMP      STATE21
          DB        0
          LJMP      STATE22
          DB        0
          LJMP      STATE23
          DB        0
          LJMP      STATE24
          DB        0
          LJMP      STATE25
```

35

```
DB        0
LJMP      STATE26
DB        0
LJMP      STATE27
DB        0
LJMP      STATE28
DB        0
LJMP      STATE29
DB        0
LJMP      STATE30
DB        0
LJMP      STATE31
DB        0
LJMP      STATE32
DB        0
LJMP      STATE33
DB        0
LJMP      STATE34
DB        0
LJMP      STATE35
DB        0
LJMP      STATE36
DB        0
LJMP      STATE37
DB        0
LJMP      STATE38
DB        0
```

```
        ;
        STATE0:     MOV         STATE,#1            ;STATE:=1
                    MOV         ABORT,#1            ;ABORT:=1
                    MOV         STAT,#00H           ;RESET STATUS
                    MOV         CTRL,#00H           ;RESET CONTROLS
                    MOV         TCEN,#00H           ;RESET ALARMS
                    MOV         TCFL,#00H           ;RESET TIMEOUT FLAGS
                    MOV         MSK0,#00H           ;RESET CLOSED MASKS
                    MOV         MSK1,#00H           ;RESET OPEN MASKS
                    MOV         MSK2,#00H           ;RESET MISC. MASKS
                    MOV         MSK3,#00H           ;RESET MISC. MASKS
                    MOV         CCO0,#00H           ;RESET ALARM LIGHTS
                    MOV         CCO1,#00H           ;RESET RUN LIGHTS
                    MOV         CCO2,#40H           ;RESET ALL VALVES
                    MOV         CCO3,#00H           ;RESET MISC. OUTPUTS
                    CLR         F0                  ;CLEAR HOLD FLAG
                    LJMP        SEQR                ;RETURN
        ;
        STATE1:     JNB         DSC1,S11            ;IF DOOR CLOSED
                    MOV         STATE,#2            ;   STATE:=2
                    MOV         ABORT,#29           ;   ABORT:=29
                    CLR         LT01                ;   DOOR-OPEN(OFF)
                    SETB        LT11                ;   READY-FOR-CYCLE(ON)
                    CLR         F0                  ;   CLEAR HOLD FLAG
                    SJMP        S12                 ;   END
        S11:        SETB        LT01                ;ELSE, DOOR-OPEN(ON)
                    CLR         LT11                ;   READY-FOR-CYCLE(OFF)
                    SETB        F0                  ;   SET HOLD FLAG
        S12:        NOP                             ;   END
                    LJMP        SEQR                ;RETURN
        ;
        STATE2:     JNB         SUC1,S21            ;IF START-CYCLE(PUSHED)
                    MOV         STATE,#3            ;   STATE:=3
                    MOV         ABORT,#29           ;   ABORT:=29
                    CLR         LT11                ;   READY-FOR-CYCLE(OFF)
                    SETB        LT12                ;   CYCLE-IN-PROGRESS(ON)
                    MOV         CNT0,#CNTM          ;   LOAD MIN. COUNT
                    CLR         TFL6                ;   CLEAR COUNT FLAG
                    CLR         MVC7                ;   CLEAR VC7 MASK
                    CLR         MVO7                ;   CLEAR VO7 MASK
                    CLR         VVO7                ;   CLOSE-BREAK-VALVE
                    MOV         TTM0,#VDLY          ;   LOAD TIMEOUT DELAY
                    CLR         TFL0                ;   RESET TIMEOUT FLAG
                    SETB        TEN0                ;   ENABLE TIMEOUT ALARM
                    CLR         F0                  ;   CLEAR HOLD FLAG
                    SJMP        S23                 ;   END
        S21:        JB          DSC1,S22            ;ELSE, IF DOOR-OPEN
                    MOV         STATE,#1            ;     STATE:=1
                    MOV         ABORT,#29           ;     ABORT:=29
                    CLR         F0                  ;     CLEAR HOLD FLAG
                    SJMP        S23                 ;     END
        S22:        SETB        F0                  ;   ELSE, SET HOLD FLAG
        S23:        NOP                             ;   END
                    LJMP        SEQR                ;RETURN
        ;
```

```
STATE3:   JNB     LSC7,S31        ;IF V7 CLOSED
          MOV     STATE,#4        ;   STATE:=4
          MOV     ABORT,#29       ;   ABORT:=29
          CLR     TEN0            ;   CLEAR TIMEOUT ENABLE
          SETB    MVC7            ;   SET VC7 MASK
          SETB    MVO7            ;   SET VO7 MASK
          SETB    HTO1            ;   TURN HEATER ON
          MOV     MTM0,#HDLY      ;   LOAD HEATER TIMEOUT
          CLR     TFL4            ;   RESET TIMEOUT FLAG
          SETB    TEN4            ;   ENABLE TIMEOUT ALARM
          CLR     F0              ;   CLEAR HOLD FLAG
          SJMP    S33             ;   END
S31:      JB      DSC1,S32        ;ELSE, IF DOOR OPEN
          MOV     A,ABORT         ;     GET ABORT STATE
          MOV     STATE,A         ;     STATE:=ABORT-1
          SETB    LTO1            ;     DOOR-OPEN(ON)
          CLR     F0              ;     CLEAR HOLD FLAG
          SJMP    S33             ;     END
S32:      SETB    F0              ;   ELSE, SET HOLD FLAG
S33:      NOP                     ;     END
          LJMP    SEQR            ;RETURN
;
```

```
STATE4:   JNB     TSC1,S41        ;IF HEATER ON
          MOV     STATE,#5        ;   STATE:=5
          MOV     ABORT,#29       ;   ABORT:=29
          CLR     TEN4            ;   CLEAR TIMEOUT ENABLE
          SETB    MTC1            ;   SET TEMP SW MASK
          MOV     STP1,#TSP1      ;   LOAD TEMP. SETPOINT
          SETB    CEN1            ;   ENABLE TEMP. CONTROL
          CLR     MVC1            ;   CLEAR VC1 MASK
          CLR     MVO1            ;   CLEAR VO1 MASK
          SETB    VVO1            ;   OPEN V1
          MOV     TTMO,#VDLY      ;   LOAD TIMEOUT DELAY
          CLR     TFLO            ;   RESET TIMEOUT FLAG
          SETB    TENO            ;   ENABLE TIMEOUT ALARM
          CLR     FO              ;   CLEAR HOLD FLAG
          SJMP    S43             ;   END
S41:      JB      DSC1,S42        ;ELSE, IF DOOR OPEN
          MOV     A,ABORT         ;     GET ABORT STATE
          MOV     STATE,A         ;     STATE:=ABORT-1
          SETB    LTO1            ;     DOOR-OPEN(ON)
          CLR     FO              ;     CLEAR HOLD FLAG
          SJMP    S43             ;     END
S42:      SETB    FO              ;   ELSE, SET HOLD FLAG
S43:      NOP                     ;     END
          LJMP    SEQR            ;RETURN
;
STATE5:   JNB     LSO1,S51        ;IF VAC VALVE OPEN
          MOV     STATE,#6        ;   STATE:=6
          MOV     ABORT,#29       ;   ABORT:=29
          CLR     TENO            ;   CLEAR TIMEOUT ENABLE
          SETB    MVC1            ;   SET VC1 MASK
          SETB    MVO1            ;   SET VO1 MASK
          SETB    PPO1            ;   TURN P1 ON
          SETB    LT13            ;   EVAC-IN-PROGRESS(ON)
          MOV     MTMO,#TVAC      ;   LOAD EVAC TIME
          CLR     TFL4            ;   RESET TIMEOUT FLAG
          CLR     FO              ;   CLEAR HOLD FLAG
          SJMP    S53             ;   END
S51:      JB      DSC1,S52        ;ELSE, IF DOOR OPEN
          MOV     A,ABORT         ;     GET ABORT STATE
          MOV     STATE,A         ;     STATE:=ABORT-1
          SETB    LTO1            ;     DOOR-OPEN(ON)
          CLR     FO              ;     CLEAR HOLD FLAG
          SJMP    S53             ;     END
S52:      SETB    FO              ;   ELSE, SET HOLD FLAG
          NOP                     ;     END
S53:      LJMP    SEQR            ;RETURN
;
STATE6:   JNB     TFL4,S62        ;IF EVAC TIME
          CLR     C               ;   CLEAR CARRY
          MOV     A,ADIO          ;   GET PRESSURE
          SUBB    A,#PVAC         ;   SUBTRACT PRESS. LIMIT
          JC      S61             ;   IF P.LE.PVAC
          MOV     STATE,#7        ;     STATE:=7
          MOV     ABORT,#29       ;     ABORT:=29
          CLR     MVC1            ;     CLEAR VC1 MASK
```

```
        CLR     MVO1            ;    CLEAR VO1 MASK
        CLR     VVO1            ;    CLOSE V1
        MOV     TTMO,#VDLY      ;    LOAD TIMEOUT
        CLR     TFLO            ;    RESET TIMEOUT FLAG
        SETB    TENO            ;    ENABLE TIMEOUT ALARM
        CLR     FO              ;    CLEAR HOLD FLAG
        SJMP    S63             ;    END
 S61:   SETB    LTO2            ; ELSE, EVAC-FAIL(ON)
        MOV     A,ABORT         ;    GET ABORT STATE
        MOV     STATE,A         ;    STATE:=ABORT-1
        CLR     FO              ;    CLEAR HOLD FLAG
        SJMP    S63             ;    END
 S62:   SETB    FO              ;ELSE, SET HOLD FLAG
 S63:   NOP                     ;  END
        LJMP    SEQR            ;RETURN
;
```

```
STATE7:  JNB   LSC1,S71       ;LF V1 CLOSED
         MOV   STATE,#8       ;  STATE:=8
         MOV   ABORT,#29      ;  ABORT:=29
         CLR   TEN0           ;  DISABLE TIMEOUT
         SETB  MVC1           ;  SET VC1 MASK
         SETB  MVO1           ;  SET VO1 MASK
         MOV   MTM0,#LKHT     ;  LOAD LEAK HOLD TIME
         CLR   TFL4           ;  RESET TIMEOUT FLAG
         CLR   F0             ;  CLEAR HOLD FLAG
         SJMP  S72            ;  END
S71:     SETB  F0             ;ELSE, SET HOLD FLAG
         NOP                  ;  END
S72:     LJMP  SEQR           ;RETURN
;
STATE8:  JNB   TFL4,S82       ;IF LEAK HOLD TIME
         CLR   C              ;  CLEAR CARRY
         MOV   A,ADI0         ;  GET PRESSURE
         SUBB  A,#PRLK        ;  SUBTRACT LEAK LIMIT
         JC    S81            ;  IF P.LE.PRLK
         MOV   STATE,#9       ;    STATE:=9
         MOV   ABORT,#30      ;    ABORT:=30
         MOV   STP2,#HSP1     ;    GET HUM. SETPOINT
         CLR   MVO6           ;    CLEAR VO6 MASK
         CLR   MVC6           ;    CLEAR VC6 MASK
         SETB  CEN2           ;    ENABLE HUM. LOOP (V6)
         MOV   MTM0,#HUMT     ;    LOAD HUM. TIMER
         CLR   TFL4           ;    RESET TIMEOUT FLAG
         CLR   LT13           ;    EVAC-IN-PROGRESS(OFF)
         SETB  LT14           ;    FILL-IN-PROGRESS(ON)
         CLR   F0             ;    CLEAR HOLD FLAG
         SJMP  S83            ;    END
S81:     SETB  LT02           ;  ELSE, EVAC-FAIL(ON)
         MOV   A,ABORT        ;    GET ABORT STATE
         MOV   STATE,A        ;    STATE:=ABORT-1
         CLR   F0             ;    CLEAR HOLD FLAG
         SJMP  S83            ;    END
S82:     SETB  F0             ;ELSE, SET HOLD FLAG
S83:     NOP                  ;  END
         LJMP  SEQR           ;RETURN
;
STATE9:  JNB   TFL4,S92       ;IF HUM. TIME
         CLR   C              ;  CLEAR CARRY
         MOV   A,ADI3         ;  GET HUMIDITY
         SUBB  A,#HNOM        ;  SUBTRACT HUM. LEVEL
         JC    S91            ;  IF HUM.GE.HNOM
         MOV   STATE,#10      ;    STATE:=10
         MOV   ABORT,#30      ;    ABORT:=30
         MOV   MTM0,#HUMH     ;    LOAD HUM. HOLD TIMER
         CLR   TFL4           ;    RESET TIMEOUT FLAG
         CLR   F0             ;    CLEAR HOLD FLAG
         SJMP  S83            ;    END
S91:     SETB  LT03           ;  ELSE, FILL-FAIL(ON)
         MOV   A,ABORT        ;    GET ABORT STATE
         MOV   STATE,A        ;    STATE:=ABORT-2
         SJMP  S93            ;    END
```

41

```
S92:       SETB      FO              ;ELSE, SET HOLD FLAG
S93:       NOP                       ;  END
           LJMP      SEQR            ;RETURN
;
STATE10:   JNB       TFL4,S101       ;IF HUM. HOLD TIME
           MOV       STATE,#11       ;   STATE:=11
           MOV       ABORT,#30       ;   ABORT:=30
           CLR       MVC2            ;   CLEAR VC2 MASK
           CLR       MVO2            ;   CLEAR VO2 MASK
           SETB      VVO2            ;   OPEN V2
           CLR       MVC8            ;   CLEAR VC8 MASK
           CLR       MVO8            ;   CLEAR VO8 MASK
           SETB      VVO8            ;   OPEN V8
           MOV       TTM0,#VDLY      ;   LOAD VALVE TIMEOUT
           CLR       TFL0            ;   RESET TIMEOUT FLAG
           SETB      TEN0            ;   ENABLE TIMEOUT ALARM
           MOV       STP0,#PSP1      ;   GET PRESS. SETPOINT
           CLR       MVO5            ;   CLEAR VO5 MASK
           CLR       MVC5            ;   CLEAR VC5 MASK
           SETB      CEN0            ;   ENABLE PRESS. LOOP (V5)
           CLR       FO              ;   CLEAR HOLD FLAG
           SJMP      S102            ;   END
S101:      SETB      FO              ;ELSE, SET HOLD FLAG
S102:      NOP                       ;    END
           LJMP      SEQR            ;RETURN
;
```

42

```
STATE11:    MOV     C,LSO2          ;TEST V2 OPEN-
            ANL     C,LSO8          ;AND V8 OPEN
            JNC     S111            ;IF (V2.AND.V8) OPEN
            MOV     STATE,#12       ;   STATE:=12
            MOV     ABORT,#31       ;   ABORT:=31
            CLR     TEN0            ;   DISABLE TIMEOUT FLAG
            SETB    MVC2            ;   SET VC2 MASK
            SETB    MVO2            ;   SET VO2 MASK
            SETB    MVC8            ;   SET VC8 MASK
            SETB    MVO8            ;   SET VO8 MASK
            MOV     STP3,#CSP1      ;   GET CONC. SETPOINT
            CLR     MVO4            ;   CLEAR VO4 MASK
            CLR     MVC4            ;   CLEAR VC4 MASK
            SETB    CEN3            ;   ENABLE CONC. LOOP (V4)
            MOV     MTM0,#CNCT      ;   LOAD CONC. TIMER
            CLR     TFL4            ;   RESET TIMEOUT FLAG
            CLR     LT14            ;   FILL-IN-PROGRESS(OFF)
            SETB    LT15            ;   STERIL-IN-PROGRESS(ON)
            CLR     F0              ;   CLEAR HOLD FLAG
            SJMP    S112            ;   END
S111:       SETB    F0              ;   ELSE, SET HOLD FLAG
S112:       NOP                     ;      END
            LJMP    SEQR            ;RETURN
;
STATE12:    JNB     TFL4,S122       ;IF CONC. TIME
            CLR     C               ;   CLEAR CARRY
            MOV     A,ADI3          ;   GET CONC.
            SUBB    A,#CNOM         ;   SUBTRACT CONC. LEVEL
            JC      S121            ;   IF CONC.GE.CNOM
            MOV     STATE,#13       ;      STATE:=13
            MOV     ABORT,#31       ;      ABORT:=31
            MOV     MTM0,#CONH      ;      LOAD CONC. HOLD TIMER
            CLR     TFL4            ;      RESET TIMEOUT FLAG
            CLR     F0              ;      CLEAR HOLD FLAG
            SJMP    S123            ;      END
S121:       SETB    LT04            ;   ELSE, STERIL-FAIL(ON)
            MOV     A,ABORT         ;      GET ABORT STATE
            MOV     STATE,A         ;      STATE:=ABORT-3
            CLR     F0              ;      CLEAR HOLD FLAG
            SJMP    S123            ;      END
S122:       SETB    F0              ;ELSE, SET HOLD FLAG
S123:       NOP                     ;   END
            LJMP    SEQR            ;RETURN
;
STATE13:    JNB     TFL4,S132       ;IF GAS HOLD TIME
            CLR     C               ;   CLEAR CARRY
            MOV     A,ADI3          ;   GET CONC.
            SUBB    A,#CNOM         ;   SUBTRACT CONC. LEVEL
            JC      S131            ;   IF CONC.GE.CNOM
            MOV     STATE,#14       ;      STATE:=14
            MOV     ABORT,#31       ;      ABORT:=31
            CLR     F0              ;      CLEAR HOLD FLAG
            SJMP    S133            ;      END
S131:       SETB    LT04            ;   ELSE, STERIL-FAIL(ON)
            MOV     A,ABORT         ;      GET ABORT STATE

            MOV     STATE,A         ;      STATE:=ABORT-3
            CLR     F0              ;      CLEAR HOLD FLAG
            SJMP    S133            ;      END
S132:       SETB    F0              ;ELSE, SET HOLD FLAG
S133:       NOP                     ;   END
            LJMP    SEQR            ;RETURN
;
```

```
STATE14: CLR       C                ;CLEAR CARRY
         MOV       A,ADI1           ;GET TEMP.
         SUBB      A,#TLOU          ;SUBTRACT MIN. TEMP.
         JC        S141             ;IF TEMP.GE.TMIN
         CLR       C                ;   CLEAR CARRY
         MOV       A,#TMAX          ;   GET MAX. TEMP LEVEL
         SUBB      A,ADI1           ;   SUBTRACT TEMP.
         JC        S141             ;   IF TEMP.LE.TMAX
         MOV       STATE,#15        ;     STATE:=15
         MOV       ABORT,#31        ;     ABORT:=31
         MOV       MTMO,#TSTR       ;     LOAD STERIL. TIMER
         CLR       TFL4             ;     RESET TIMEOUT FLAG
         CLR       FO               ;     CLEAR HOLD FLAG
         SJMP      S142             ;     END
S141:    SETB      LTO4             ;ELSE, STERIL-FAIL(ON)
         MOV       A,ABORT          ;   GET ABORT STATE
         MOV       STATE,A          ;   STATE:=ABORT-3
         CLR       FO               ;   CLEAR HOLD FLAG
S142:    NOP                        ;   END
         LJMP      SEQR             ;RETURN
;
STATE15: JNB       TFL4,S151        ;IF STERIL. TIME
         MOV       STATE,#16        ;   STATE:=16
         MOV       ABORT,#31        ;   ABORT:=31
         CLR       CENO             ;   PRESS. LOOP (OFF)
         CLR       CEN2             ;   HUM. LOOP (OFF)
         CLR       CEN3             ;   GAS LOOP (OFF)
         CLR       CTRO             ;   PRESS. OUTPUT (OFF)
         CLR       CTR2             ;   HUM. OUTPUT (OFF)
         CLR       CTR3             ;   GAS OUTPUT (OFF)
         CLR       VVO6             ;   CLOSE V6
         CLR       VVO5             ;   CLOSE V5
         CLR       VVO4             ;   CLOSE V4
         MOV       TTMO,#VDLY       ;   LOAD TIMEOUT DELAY
         CLR       TFLO             ;   RESET TIMEOUT FLAG
         SETB      TENO             ;   ENABLE TIMEOUT ALARM
         CLR       FO               ;   CLEAR HOLD FLAG
         SJMP      S152             ;   END
S151:    SETB      FO               ;ELSE, SET HOLD FLAG
S152:    NOP                        ;   END
         LJMP      SEQR             ;RETURN
;
STATE16: MOV       C,LSC4           ;TEST V4 CLOSED
         ANL       C,LSC5           ;AND V5 CLOSED
         ANL       C,LSC6           ;AND V6 CLOSED
         JNC       S161             ;IF (V4,V5,& V6) CLOSED
         MOV       STATE,#17        ;   STATE:=17
         MOV       ABORT,#31        ;   ABORT:=31
         CLR       TENO             ;   DISABLE TIMEOUT ALARM
         SETB      MVC4             ;   SET VC4 MASK
         SETB      MVO4             ;   SET VO4 MASK
         SETB      MVC5             ;   SET VC5 MASK
         SETB      MVO5             ;   SET VO5 MASK
         SETB      MVC6             ;   SET VC6 MASK
         SETB      MVO6             ;   SET VO6 MASK
```

44

```
         CLR       MVC3              ;   CLEAR VC3 MASK
         CLR       MVO3              ;   CLEAR VO3 MASK
         SETB      VVO3              ;   OPEN V3
         CLR       LT15              ;   STERIL-IN-PROGRESS(OFF)
         SETB      LT16              ;   PURGE-IN-PROGRESS(ON)
         MOV       TTMO,#VDLY        ;   LOAD TIMEOUT DELAY
         CLR       TFLO              ;   RESET TIMEOUT FLAG
         SETB      TENO              ;   ENABLE TIMEOUT ALARM
         CLR       FO                ;   CLEAR HOLD FLAG
         SJMP      S162              ;   END
S161:    SETB      FO                ;ELSE, SET HOLD FLAG
S162:    NOP                         ;   END
         LJMP      SEQR              ;RETURN
;
STATE17: MOV       C,LSO3            ;TEST V3 OPEN-
         ANL       C,LSO8            ;AND V8 OPEN
         JNC       S171              ;IF (V3.AND.V8) OPEN
         MOV       STATE,#18         ;   STATE:=18
         MOV       ABORT,#31         ;   ABORT:=31
         CLR       TENO              ;   DISABLE TIMEOUT ALARM
         SETB      MVC3              ;   SET VC3 MASK
         SETB      MVO3              ;   SET VO3 MASK
         SETB      MVC8              ;   SET VC8 MASK
         SETB      MVO8              ;   SET VO8 MASK
         MOV       MTMO,#TEVC        ;   LOAD EVAC. TIMER
         CLR       TFL4              ;   RESET TIMEOUT FLAG
         CLR       FO                ;   CLEAR HOLD FLAG
         SJMP      S172              ;   END
S171:    SETB      FO                ;ELSE, SET HOLD FLAG
S172:    NOP                         ;   END
         LJMP      SEQR              ;RETURN
;
```

45

```
STATE18: JNB      TFL4,S181           ;IF EVAC. TIME
         MOV      STATE,#19           ;   STATE:=19
         MOV      ABORT,#31           ;   ABORT:=31
         CLR      MVC3                ;   CLEAR VC3 MASK
         CLR      MVO3                ;   CLEAR VO3 MASK
         CLR      VVO3                ;   CLOSE V3
         CLR      MVC8                ;   CLEAR VC8 MASK
         CLR      MVO8                ;   CLEAR VO8 MASK
         CLR      VVO8                ;   CLOSE V8
         MOV      TTMO,#VDLY          ;   LOAD VALVE TIMER
         CLR      TFLO                ;   RESET TIMEOUT FLAG
         SETB     TENO                ;   ENABLE TIMEOUT ALARM
         CLR      FO                  ;   CLEAR HOLD FLAG
         SJMP     S182                ;   END
S181:    SETB     FO                  ;ELSE, SET HOLD FLAG
S182:    NOP                          ;   END
         LJMP     SEQR                ;RETURN
;
STATE19: MOV      C,LSC3              ;TEST V3 CLOSED-
         ANL      C,LSC8              ;AND V8 CLOSED
         JNC      S191                ;IF (V3.AND.V8) CLOSED
         MOV      STATE,#20           ;   STATE:=20
         MOV      ABORT,#32           ;   ABORT:=32
         CLR      TENO                ;   DISABLE TIMEOUT ALARM
         SETB     MVC8                ;   SET VC8 MASK
         SETB     MVO8                ;   SET VO8 MASK
         MOV      STPO,#PSP1          ;   GET PRESS. SETPOINT
         CLR      MVO5                ;   CLEAR VO5 MASK
         CLR      MVC5                ;   CLEAR VC5 MASK
         SETB     CENO                ;   ENABLE PRESS. CONTROL (V5)
         MOV      MTMO,#PN2T          ;   LOAD N2 PRESS. TIMER
         CLR      TFL4                ;   RESET TIMEOUT FLAG
         CLR      FO                  ;   CLEAR HOLD FLAG
         SJMP     S192                ;   END
S191:    SETB     FO                  ;ELSE, SET HOLD FLAG
S192:    NOP                          ;   END
         LJMP     SEQR                ;RETURN
;
STATE20: JNB      TFL4,S202           ;IF REPRESS. TIME
         CLR      C                   ;   CLEAR CARRY
         MOV      A,#PMAX             ;   GET MIN.PRESS. LEVEL
         SUBB     A,ADIO              ;   SUBTRACT PRESSURE
         JC       S201                ;   IF PRESS.GE.PMAX
         MOV      STATE,#21           ;     STATE:=21
         MOV      ABORT,#32           ;     ABORT:=32
         CLR      CENO                ;     N2 LOOP (OFF)
         CLR      CTRO                ;     N2 OUTPUT (OFF)
         CLR      VVO5                ;     CLOSE N2 VALVE
         MOV      TTMO,#VDLY          ;     LOAD VALVE TIMEOUT
         CLR      TFLO                ;     RESET TIMEOUT FLAG
         SETB     TENO                ;     ENABLE TIMEOUT ALARM
         CLR      FO                  ;     CLEAR HOLD FLAG
         SJMP     S202                ;     END
S201:    SETB     LTO5                ;   ELSE, PURGE-FAIL(ON)
         MOV      A,ABORT             ;     GET ABORT STATE

         MOV      STATE,A             ;     STATE:=ABORT-4
         CLR      FO                  ;     CLEAR HOLD TIMER
         SJMP     S203                ;     END
S202:    SETB     FO                  ;ELSE, SET HOLD TIMER
S203:    NOP                          ;   END
         LJMP     SEQR                ;RETURN
;
```

```
STATE21:  JNB    LSC5,S211      ;IF V5 CLOSED
          MOV    STATE,#22      ;   STATE:=22
          MOV    ABORT,#33      ;   ABORT:=33
          CLR    TEN0           ;   DISABLE TIMEOUT ALARM
          SETB   MVC5           ;   SET VC5 MASK
          SETB   MVO5           ;   SET VO5 MASK
          CLR    MVC3           ;   CLEAR VC3 MASK
          CLR    MVO3           ;   CLEAR VO3 MASK
          SETB   VVO3           ;   OPEN V3
          CLR    MVC8           ;   CLEAR VC8 MASK
          CLR    MVO8           ;   CLEAR VO8 MASK
          SETB   VVO8           ;   OPEN V8
          MOV    TTM0,#VDLY     ;   LOAD TIMEOUT DELAY
          CLR    TFL0           ;   RESET TIMEOUT FLAG
          SETB   TEN0           ;   ENABLE TIMEOUT ALARM
          CLR    F0             ;   CLEAR HOLD FLAG
          SJMP   S212           ;   END
S211:     SETB   F0             ;ELSE, SET HOLD FLAG
S212:     NOP                   ;   END
          LJMP   SEQR           ;RETURN
;
STATE22:  MOV    C,LSO3         ;TEST V3 OPEN-
          ANL    C,LSO8         ;AND V8 OPEN
          JNC    S221           ;IF (V3.AND.V8) OPEN
          MOV    STATE,#23      ;   STATE:=23
          MOV    ABORT,#33      ;   ABORT:=33
          CLR    TEN0           ;   DISABLE TIMEOUT ALARM
          SETB   MVC3           ;   SET VC3 MASK
          SETB   MVO3           ;   SET VO3 MASK
          SETB   MVC8           ;   SET VC8 MASK
          SETB   MVO8           ;   SET VO8 MASK
          MOV    MTM0,#DSRB     ;   LOAD DESORB TIMER
          CLR    TFL4           ;   RESET TIMEOUT FLAG
          CLR    F0             ;   CLEAR HOLD FLAG
          SJMP   S222           ;   END
S221:     SETB   F0             ;   ELSE, SET HOLD FLAG
S222:     NOP                   ;     END
          LJMP   SEQR           ;RETURN
;
STATE23:  JNB    TFL4,S231      ;IF DESORB TIME
          MOV    STATE,#24      ;   STATE:=24
          MOV    ABORT,#34      ;   ABORT:=34
          CLR    MVC3           ;   CLEAR VC3 MASK
          CLR    MVO3           ;   CLEAR VO3 MASK
          CLR    VVO3           ;   CLOSE V3
          CLR    MVC8           ;   CLEAR VC8 MASK
          CLR    MVO8           ;   CLEAR VO8 MASK
          CLR    VVO8           ;   CLOSE V8
          CLR    MVC2           ;   CLEAR VC2 MASK
          CLR    MVO2           ;   CLEAR VO2 MASK
          CLR    VVO2           ;   CLOSE V2
          MOV    TTM0,#VDLY     ;   LOAD TIMEOUT DELAY
          CLR    TFL0           ;   RESET TIMEOUT FLAG
          SETB   TEN0           ;   ENABLE TIMEOUT ALARM
          CLR    F0             ;   CLEAR HOLD FLAG

          SJMP   S232           ;   END
S231:     SETB   F0             ;ELSE, SET HOLD FLAG
S232:     NOP                   ;   END
          LJMP   SEQR           ;RETURN
;
```

```
STATE24: MOV     C,LSC3          ;TEST V3 CLOSED-
         ANL     C,LSC8          ;AND V8 CLOSED-
         ANL     C,LSC2          ;AND V2 CLOSED
         JNC     S241            ;IF (V2,V3,V8 CLOSED)
         MOV     STATE,#25       ;   STATE:=25
         MOV     ABORT,#35       ;   ABORT:=35
         CLR     TENO            ;   DISABLE TIMEOUT MASK
         SETB    MVC3            ;   SET VC3 MASK
         SETB    MVO3            ;   SET VO3 MASK
         SETB    MVC8            ;   SET VC& MASK
         SETB    MVO8            ;   SET VO6 MASK
         SETB    MVC2            ;   SET VC2 MASK
         SETB    MVO2            ;   SET VO2 MASK
         SETB    LGG1            ;   SWITCH TO HIGH GAIN
         MOV     MTMO,#TLGH      ;   START LOW GAS HOLD
         LCALL   DCTO            ;   DECREMENT PURGE COUNT
         CLR     TFL4            ;   RESET TIMEOUT FLAG
         CLR     F0              ;   CLEAR HOLD FLAG
         SJMP    S242            ;   END
S241:    SETB    F0              ;ELSE, SET HOLD FLAG
S242:    NOP                     ;   END
         LJMP    SEQR            ;RETURN
         ;
STATE25: JNB     TFL4,S252       ;IF LOW-HOLD TIME
         CLR     C               ;   CLEAR CARRY
         MOV     A,#CMIN         ;   GET MAX. LEVEL
         SUBB    A,ADI3          ;   SUBTRACT CONC.
         ORL     C,/TFL6         ;   OR CARRY WITH COUNT FLAG
         JC      S251            ;   IF (CONC.LE.CMIN).AND.TFL4=1
         MOV     STATE,#26       ;      STATE:=26
         MOV     ABORT,#36       ;      ABORT:=36
         CLR     CEN1            ;      DISABLE TEMP. CTRL
         CLR     PPO1            ;      TURN PUMP OFF
         CLR     HTO1            ;      TURN HEATER OFF
         CLR     LGG1            ;      SET LOW GAIN
         CLR     F0              ;      CLEAR HOLD FLAG
         SJMP    S253            ;      END
S251:    MOV     A,ABORT         ;   ELSE, GET ABORT STATE
         MOV     STATE,A         ;      STATE:=35
         CLR     F0              ;      CLEAR HOLD FLAG
         SJMP    S253            ;      END
S252:    SETB    F0              ;ELSE, SET HOLD FLAG
S253:    NOP                     ;   END
         LJMP    SEQR            ;RETURN
         ;
STATE26: LCALL   GTCT            ;DECREMENT & GET CYCLE CNT
         JNZ     S261            ;IF LAST RUN
         MOV     STATE,#27       ;   STATE:=27
         MOV     ABORT,#36       ;   ABORT:=36
         CLR     MVC2            ;   CLEAR VC2 MASK
         CLR     MVO2            ;   CLEAR VO2 MASK
         SETB    VVO2            ;   OPEN V2
         CLR     MVC3            ;   CLEAR VC3 MASK
         CLR     MVO3            ;   CLEAR VO3 MASK
         SETB    VVO3            ;   OPEN V3
```

48

```
        CLR     MVC4            ; CLEAR VC4 MASK
        CLR     MVO4            ; CLEAR VO4 MASK
        SETB    VVO4            ; OPEN V4
        CLR     MVC8            ; CLEAR VC8 MASK
        CLR     MVO8            ; CLEAR VO8 MASK
        SETB    VVO8            ; OPEN V8
        MOV     MTMO,#TDMP      ; LOAD DUMP TIMER
        CLR     TFL4            ; RESET TIMEOUT FLAG
        CLR     FO              ; CLEAR HOLD FLAG
        SJMP    S262            ; END
S261:   MOV     STATE,#28       ;ELSE, STATE:=28
        MOV     ABORT,#36       ;   ABORT:=36
        CLR     FO              ;   CLEAR HOLD FLAG
S262:   NOP                     ;   END
        LJMP    SEQR            ;RETURN
```

```
STATE27: JNB      TFL4,S271        ;IF DUMP-TIME
         MOV      STATE,#28        ;   STATE:=28
         MOV      ABORT,#36        ;   ABORT:=36
         CLR      VVO2             ;   CLOSE V2
         CLR      VVO3             ;   CLOSE V3
         CLR      VVO4             ;   CLOSE V4
         CLR      VVO8             ;   CLOSE V8
         CLR      FO               ;   CLEAR HOLD FLAG
         SJMP     S272             ;   END
S271:    SETB     FO               ;ELSE, SET HOLD FLAG
S272:    NOP                       ;   END
         LJMP     SEQR             ;RETURN
;
STATE28: MOV      STATE,#37        ;STATE:=37
         MOV      ABORT,#36        ;ABORT:=36
         CLR      MVO5             ;CLR VO5 MASK
         CLR      MVC5             ;CLR VC5 MASK
         SETB     CENO             ;PRESS. CONTROL(ON)
         MOV      STPO,#PATM       ;SET ATM. SETPOINT
         CLR      FO               ;CLEAR HOLD FLAG
         LJMP     SEQR             ;RETURN
;
STATE29: MOV      C,LSC1           ;TEST V1 CLOSED-
         ANL      C,LSC2           ;AND V2 CLOSED-
         ANL      C,LSC3           ;AND V3 CLOSED-
         ANL      C,LSC4           ;AND V4 CLOSED-
         ANL      C,LSC5           ;AND V5 CLOSED-
         ANL      C,LSC6           ;AND V6 CLOSED-
         ANL      C,LSO7           ;AND V7 OPEN-
         ANL      C,LSC8           ;AND V8 CLOSED-
         ANL      C,SWC2           ;AND SW2 PUSHED
         JNC      S291             ;IF RESET
         MOV      STATE,#2         ;   STATE:=2
         MOV      ABORT,#0         ;   ABORT:=0
         MOV      STAT,#00H        ;   RESET STATUS
         MOV      CCOO,#00H        ;   RESET ALARM LIGHTS
         MOV      CCO1,#01H        ;   RESET RUN LIGHTS
         CLR      FO               ;   CLEAR HOLD FLAG
         SJMP     S292             ;   END
S291:    MOV      CTRL,#00H        ;ELSE, RESET CONTROLS
         MOV      TCEN,#00H        ;   RESET ALARMS
         MOV      MSKO,#00H        ;   RESET CLOSED MASKS
         MOV      MSK1,#00H        ;   RESET OPEN MASKS
         MOV      MSK2,#00H        ;   RESET MISC. MASKS
         MOV      CCO2,#40H        ;   RESET VALVES
         MOV      CCO3,#00H        ;   RESET MISC. OUTPUTS
         MOV      CCO1,#00H        ;   TURN CYCLE LIGHTS OFF
         SETB     LTO2             ;   EVAC-FAIL(ON)
         SETB     FO               ;   SET HOLD FLAG
S292:    NOP                       ;   END
         LJMP     SEQR             ;RETURN
;
```

50

```
STATE30: MOV      C,LSC1           ;TEST V1 CLOSED-
         ANL      C,LSC2           ;AND V2 CLOSED-
         ANL      C,LSC3           ;AND V3 CLOSED-
         ANL      C,LSC4           ;AND V4 CLOSED-
         ANL      C,LSC5           ;AND V5 CLOSED-
         ANL      C,LSC6           ;AND V6 CLOSED-
         ANL      C,LSO7           ;AND V7 OPEN-
         ANL      C,LSC8           ;AND V8 CLOSED-
         ANL      C,SUC2           ;AND SU2 PUSHED
         JNC      S301             ;IF RESET
         MOV      STATE,#2         ;   STATE:=2
         MOV      ABORT,#0         ;   ABORT:=0
         MOV      STAT,#00H        ;   RESET STATUS
         MOV      CCO0,#00H        ;   RESET ALARM LIGHTS
         MOV      CCO1,#01H        ;   RESET RUN LIGHTS
         CLR      FO               ;   CLEAR HOLD FLAG
         SJMP     S302             ;   END
S301:    MOV      CTRL,#00H        ;ELSE, RESET CONTROLS
         MOV      TCEN,#00H        ;   RESET ALARMS
         MOV      MSK0,#00H        ;   RESET CLOSED MASKS
         MOV      MSK1,#00H        ;   RESET OPEN MASKS
         MOV      MSK2,#00H        ;   RESET MISC. MASKS
         MOV      CCO2,#40H        ;   RESET VALVES
         MOV      CCO3,#00H        ;   RESET MISC. OUTPUTS
         MOV      CCO1,#00H        ;   TURN CYCLE LIGHTS OFF
         SETB     LT03             ;   FILL-FAIL(ON)
         SETB     FO               ;   SET HOLD FLAG
S302:    NOP                       ;   END
         LJMP     SEQR             ;RETURN
```

```
STATE31: MOV      C,LSC1           ;TEST V1 CLOSED-
         ANL      C,LSO2           ;AND V2 OPEN-
         ANL      C,LSC3           ;AND V3 CLOSED
         ANL      C,LSC4           ;AND V4 CLOSED-
         ANL      C,LSC6           ;AND V6 CLOSED-
         ANL      C,LSC7           ;AND V7 CLOSED-
         ANL      C,LSC8           ;AND V8 CLOSED-
         ANL      C,SWC2           ;AND SW2 PUSHED
         JNC      S311             ;IF RESET
         MOV      STATE,#20        ;  STATE:=20
         MOV      ABORT,#31        ;  ABORT:=32
         MOV      STAT,#00H        ;  RESET STATUS
         MOV      MSK0,#0EFH       ;  SET ALL CLOSED MASKS
         MOV      MSK1,#0EFH       ;  SET ALL OPEN MASKS
         MOV      MSK2,#001H       ;  SET MISC. MASKS
         MOV      CCO0,#00H        ;  RESET ALARM LIGHTS
         MOV      CCO1,#22H        ;  RESET RUN LIGHTS
         CLR      F0               ;  CLEAR HOLD FLAG
         SJMP     S312             ;  END
S311:    MOV      CTRL,#03H        ;ELSE, RESET CONTROLS
         MOV      TCEN,#00H        ;  RESET ALARMS
         MOV      MSK0,#00H        ;  RESET CLOSED MASKS
         MOV      MSK1,#00H        ;  RESET OPEN MASKS
         MOV      MSK2,#00H        ;  RESET MISC. MASKS
         MOV      CCO2,#02H        ;  RESET ALL VALVES
         MOV      CCO3,#01H        ;  RESET MISC. OUTPUTS
         SETB     LTO4             ;  STERIL-FAIL(ON)
         SETB     F0               ;  SET HOLD FLAG
S312:    NOP                       ;  END
         LJMP     SEQR             ;RETURN
;
STATE32: MOV      C,LSC5           ;TEST V5 CLOSED
         ANL      C,SWC2           ;AND SW2
         JNC      S321             ;IF (V5 CLOSED & SW2 PUSHED)
         MOV      STATE,#19        ;  STATE:=19
         MOV      ABORT,#32        ;  ABORT:=32
         CLR      F0               ;  CLEAR HOLD FLAG
         SJMP     S322             ;  END
S321:    MOV      CCO2,#02H        ;ELSE, RESET ALL VALVES
         SETB     F0               ;  SET HOLD FLAG
S322:    NOP                       ;  END
         LJMP     SEQR             ;RETURN
;
STATE33: MOV      C,SWC2           ;TEST SW2
         JNC      S331             ;IF PUSHED
         MOV      STATE,#23        ;  STATE:=23
         MOV      ABORT,#33        ;  ABORT:=34
         CLR      F0               ;  CLEAR HOLD FLAG
         SJMP     S332             ;  END
S331:    SETB     F0               ;ELSE, SET HOLD FLAG
S332:    NOP                       ;  END
         LJMP     SEQR             ;RETURN
;
STATE34: MOV      C,SWC2           ;TEST SW2
         JNC      S341             ;IF PUSHED

         MOV      STATE,#25        ;  STATE:=25
         MOV      ABORT,#35        ;  ABORT:=35
         LCALL    DCTO             ;  DECREMENT PURGE COUNT
         CLR      F0               ;  CLEAR HOLD FLAG
         SJMP     S342             ;  END
S341:    SETB     F0               ;ELSE, SET HOLD FLAG
S342:    NOP                       ;  END
         LJMP     SEQR             ;RETURN
```

52

```
STATE35: MOV    C,LSC1         ;TEST V1 CLOSED-
         ANL    C,LSO2         ;AND V2 OPEN-
         ANL    C,LSC3         ;AND V3 CLOSED-
         ANL    C,LSC4         ;AND V4 CLOSED-
         ANL    C,LSC5         ;AND V5 CLOSED-
         ANL    C,LSC6         ;AND V6 CLOSED-
         ANL    C,LSC7         ;AND V7 CLOSED-
         ANL    C,LSC8         ;AND V8 CLOSED-
         JNC    S351           ;IF RESET
         MOV    STATE,#20      ;   STATE:=20
         MOV    ABORT,#32      ;   ABORT:=32
         MOV    STAT,#00H      ;   RESET STATUS
         MOV    MSK0,#0EFH     ;   SET ALL CLOSED MASKS
         MOV    MSK1,#0EFH     ;   SET ALL OPEN MASKS
         MOV    MSK2,#001H     ;   SET MISC. MASKS
         MOV    CCO0,#00H      ;   RESET ALARM LIGHTS
         MOV    CCO1,#22H      ;   RESET RUN LIGHTS
         MOV    STP0,#PSP1     ;   LOAD PRESS. SETPOINT
         SETB   CENO           ;   ENABLE PRESSURE CONTROL
         MOV    MTM0,#PN2T     ;   SET PRESSURE TIMER
         CLR    TFL4           ;   CLEAR TIMER FLAG
         CLR    F0             ;   CLEAR HOLD FLAG
         SJMP   S352           ;   END
S351:    MOV    CTRL,#03H      ;ELSE, RESET CONTROLS
         MOV    TCEN,#00H      ;   RESET ALARMS
         MOV    MSK0,#00H      ;   RESET CLOSED MASKS
         MOV    MSK1,#00H      ;   RESET OPEN MASKS
         MOV    MSK2,#00H      ;   RESET MISC. MASKS
         MOV    CCO2,#02H      ;   RESET ALL VALVES
         MOV    CCO3,#01H      ;   RESET MISC. OUTPUTS
         SETB   F0             ;   SET HOLD FLAG
S352:    NOP                   ;   END
         LJMP   SEQR           ;RETURN
         ;


STATE36: MOV    C,SWC2         ;TEST SW2
         JNC    S361           ;IF PUSHED
         MOV    STATE,#26      ;   STATE:=26
         MOV    ABORT,#37      ;   ABORT:=37
         CLR    F0             ;   CLEAR HOLD FLAG
         SJMP   S362           ;   END
S361:    SETB   F0             ;ELSE, SET HOLD FLAG
S362:    NOP                   ;   END
         LJMP   SEQR           ;RETURN
         ;
```

```
STATE37:  CLR      C              ;CLEAR CARRY
          MOV      A,#PATM        ;GET ATM SETPOINT
          SUBB     A,ADIO         ;SUBTRACT PRESSURE
          JC       S371           ;IF PRESS.GT.ATM
          MOV      STATE,#38      ;   STATE:=38
          MOV      ABORT,#0       ;   ABORT:=0
          CLR      MVC7           ;   CLEAR VC7 MASK
          CLR      MVO7           ;   CLEAR VO7 MASK
          CLR      CENO           ;   N2 LOOP(OFF)
          CLR      CTRO           ;   N2 OUTP(OFF)
          CLR      VVO5           ;   CLOSE N2 VALVE
          SETB     VVO7           ;   OPEN V7
          CLR      LT16           ;   PURGE-IN-PROGRESS(OFF)
          SETB     LT17           ;   REMOVE-LOAD(ON)
          CLR      FO             ;   CLEAR HOLD FLAG
          SJMP     S372           ;   END
S371:     SETB     FO             ;ELSE, SET HOLD FLAG
S372:     NOP                     ;   END
          LJMP     SEQR           ;RETURN
;
STATE38:  JNB      SUC2,S381      ;IF SU2 PUSHED
          MOV      STATE,#0       ;   STATE:=0 (RESET)
          MOV      ABORT,#0       ;   ABORT:=0
          CLR      FO             ;   CLEAR HOLD FLAG
          SJMP     S382           ;   END
S381:     SETB     FO             ;ELSE, SET HOLD FLAG
S382:     NOP                     ;   END
          LJMP     SEQR           ;RETURN
;
```

In the foregoing specification, the invention has been described with reference to a specific exemplary embodiment thereof. It will, however, be evident that various modifications and changes may be made thereunto without parting from the broader spirit and scope of the invention as set forth in the appended claims. For example, as will be appreciated by those of ordinary skill in the art familiar with this specification, the apparatus disclosed herein may be suitable for use in connection with various types of gaseous treatment systems, such as those which employ toxic gases, e.g., without limitation, bleaching gases, fumigants, sterilants, etc. The specification and drawings are, accordingly, to be regarded in an illustrative rather than a restrictive sense.

### Claims

1. Apparatus for treating articles with a gas comprising:
   chamber means (10) for receiving an article to be treated;
   means (20, 22) for supplying the gas to the chamber means (10) comprising valve means (20) coupled to the chamber means (10) for supplying the gas to the chamber means, means (20) for removing the gas from the chamber means (10) after a predetermined time interval, means (5) for measuring a plurality of parameters in said chamber means and for generating a plurality of electrical signals associated with ones of the measured parameters, electronic control means (100) receiving said plurality of electrical signals associated with ones of measured parameters from said chamber means (10) for controlling said valve means (20) and said means (20) for removing, said electronic control means (100) comprise computer means for cycling said apparatus through a plurality of states in accordance with a predetermined sequence of instructions, said computer means including means for aborting the operation of said apparatus characterized in that said aborting means abort the operation to one of a plurality of defined failure states having predefined conditions in response to a failure of said apparatus, said selected failure state being dependent on the state in said cycle in which the failure occurred and further comprising means (S2) for cycling said apparatus in accordance with said predetermined sequence to a further defined state once one of said defined failure states is reached, said further defined state comprising one of the states in accordance with said predetermined sequence of instructions, said further defined state being dependent on the defined failure state reached and being a state which maintains said apparatus within acceptable standards of safety.

2. The apparatus recited in claim 1 wherein said gas is a sterilizing gas, whereby said article is sterilized by said gas.

3. The apparatus recited in claim 2, wherein said sterilizing gas is generated from at least two component parts, and further including first means for receiving a first component part of the gas, second means for receiving a second component part of the gas, means for allowing said first and second component parts to react with each other to generate said sterilizing gas, said means for allowing reaction being controlled by said computer means in response to the measurement of selected ones of said plurality of measured parameters.

4. The apparatus recited in claim 3, further comprising valve means (V5) for supplying a stable gas to said chamber means (10).

5. The apparatus recited in claim 3 or 4, further comprising valve means (V7) for supplying filtered air to said chamber means (10).

6. The apparatus recited in any of claims 3 to 5, further comprising valve means (V6) for supplying water vapor to said chamber means (10) to affect the humidity level in said chamber.

7. The apparatus recited in any of claims 3 to 6 wherein said plurality of measured parameters include the temperature, pressure and humidity in said chamber means (10) and the concentration of said sterilizing gas in said chamber means.

8. The apparatus recited in any of claims 2 to 7 wherein said sterilizing gas comprises chlorine dioxide.

9. The apparatus recited in any of claims 3 to 7 wherein said sterilizing gas comprises chlorine dioxide and said first component comprises chlorine gas and said second component comprises sodium chlorite.

10. The apparatus recited in any of claims 1 to 9 wherein said means for removing comprises vacuum pump means and additional valve means.

11. The apparatus recited in any of claims 1 to 10, wherein said valve means (Vi) comprises first and second switch means (LSio, LSic), said first switch means (LSio) indicating when said valve means (Vi) is open and said second switch means (LSic) indicating when said valve means (Vi) is closed, said first and second switch means being in opposite states such that when said first switch means is closed, said second switch means is open.

12. The apparatus recited in any of claims 1 to 11, wherein said electronic control means (100) comprises memory means, and further comprising means for receiving input signals from said valve means (Vi) indicative of the closed or open condition of said valve means and means for transmitting output signals to said valve means to selectively open or close said valve means, record of image signals of said input and output signals being stored in said memory means.

13. The apparatus recited in claim 12, further comprising means for disabling said output signals from being transmitted to said valve means (Vi) except when an enabling signal is issued by said computer means.

14. The apparatus recited in any of claims 1 to 13, further comprising means for monitoring for proper operation of said computer means, said monitoring mean issuing a disabling signal to prevent actuation of said valve means in the event of a failure of said computer means.

15. The apparatus recited in any of claims 12 to 14, further comprising mask means stored in said memory means, said computer means comparing said image signals of said input and output signals and generating an alarm signal if said input and output image signals do not agree in response to the setting of a bit in said mask means.

55

**EP 0 211 073 B1**

**16.** The apparatus recited in any of claims 11 to 15, further comprising means for monitoring the state of said first and second switch means, and further comprising means for generating an alarm signal if said first and second switch means are not in the proper states.

**17.** The apparatus recited in any of claims 1 to 16, wherein said valve means (Vi) moves between a first state and a second state in response to instructions from said computer means, and further comprising timer means (112) for generating an alarm signal if said valve means (Vi) does not move from said first to second state in a predetermined time interval.

**18.** The apparatus recited in any of claims 3 to 17, wherein said means for reacting comprises second valve means (V4) for allowing said first and second components to react with each other to generate said sterilizing gas, said second valve means (V4) being controlled by said computer means in response to the measurement of selected ones of said plurality of measured parameters.

**Patentansprüche**

**1.** Vorrichtung zum Behandeln von Gegenständen mit einem Gas, die aufweist:
eine Kammereinrichtung (10) zum Aufnehmen eines zu behandelnden Gegenstands;
eine Einrichtung (20, 22) zum Zuführen des Gases zu der Kammereinrichtung (10) mit einer an der Kammereinrichtung (10) angeschlossenen Ventileinrichtung (20) zum Zuführen des Gases zu der Kammereinrichtung (10), einer Einrichtung (20) zum Entfernen des Gases aus der Kammereinrichtung (10) nach einem vorbestimmten Zeitintervall, einer Einrichtung (5) zum Messen mehrerer Parameter in der Kammereinrichtung und zum Erzeugen mehrerer elektrischer Signale, die den jeweils gemessen Parametern entsprechen, einer elektronischen Steuereinrichtung (100), die die mehreren elektrischen Signale empfängt, die den jeweils gemessenen Parametern von der Kammereinrichtung (10) entsprechen, zum Steuern der Ventileinrichtung (20) und der Einrichtung (20) zum Entfernen, wobei die elektronische Steuereinrichtung (100) eine Computereinrichtung aufweist, um die Vorrichtung zyklisch mehrere Zustände in Übereinstimmung mit einer vorbestimmten Befehlsfolge durchlaufen zu lassen und die Computereinrichtung eine Einrichtung zum Abbruch des Betriebs der Vorrichtung aufweist,
dadurch gekennzeichnet, daß die Abbrucheinrichtung den Betrieb zu einem von mehreren definierten Fehlerzuständen mit vordefinierten Bedingungen als Reaktion auf einen Fehler der Vorrichtung abbricht, wobei der ausgewählte Fehlerzustand vom Zustand in dem Zyklus abhängig ist, in dem der Fehler auftrat, und ferner eine Einrichtung (S2) aufweist, um die Vorrichtung zyklisch in Übereinstimmung mit der vorbestimmten Folge zu einem weiteren definierten Zustand zu überführen, sobald einer der definierten Fehlerzustände erreicht ist, wobei der weitere definierte Zustand einer der Zustände in Übereinstimmung mit der vorbestimmten Befehlsfolge ist, der weitere definierte Zustand von dem erreichten definierten Fehlerzustand abhängt und ein Zustand ist, der zulässige Sicherheitsnormen für die Vorrichtung aufrechterhält.

**2.** Vorrichtung nach Anspruch 1, wobei das Gas ein Sterilisiergas ist und der Gegenstand durch das Gas sterilisiert wird.

**3.** Vorrichtung nach Anspruch 2, wobei das Sterilisiergas aus mindestens zwei Bestandteilen erzeugt wird, und die ferner aufweist: eine erste Einrichtung zum Aufnehmen eines ersten Bestandteils des Gases, eine zweite Einrichtung zum Aufnehmen eines zweiten Bestandteils des Gases, eine Einrichtung, um den ersten und zweiten Bestandteil des Gases miteinander zur Bildung des Sterilisiergases reagieren zu lassen, wobei die Einrichtung zum Reagieren durch die Computereinrichtung als Reaktion auf die Messung ausgewählter Parameter der mehreren gemessenen Parameter gesteuert wird.

**4.** Vorrichtung nach Anspruch 3, ferner mit einer Ventileinrichtung (V5) zum Zuführen eines stabilen Gases zu der Kammereinrichtung (10).

**5.** Vorrichtung nach Anspruch 3 oder 4, ferner mit einer Ventileinrichtung (V7) zum Zuführen von gefilterter Luft zu der Kammereinrichtung (10).

**6.** Vorrichtung nach einem der Ansprüche 3 bis 5, ferner mit einer Ventileinrichtung (V6) zum Zuführen von Wasserdampf zu der Kammereinrichtung (10), um den Feuchtigkeitsgehalt in der Kammer zu beeinflussen.

56

7. Vorrichtung nach einem der Ansprüche 3 bis 6, wobei zu den mehreren gemessenen Parameter die Temperatur, der Druck und die Feuchtigkeit in der Kammereinrichtung (10) sowie die Konzentration des Sterilisiergases in der Kammereinrichtung gehören.

8. Vorrichtung nach einem der Ansprüche 2 bis 7, wobei das Sterilisiergas Chlordioxid aufweist.

9. Vorrichtung nach einem der Ansprüche 3 bis 7, wobei das Sterilisiergas Chlordioxid aufweist, der erste Bestandteil Chlorgas aufweist und der zweite Bestandteil Natriumchlorit aufweist.

10. Vorrichtung nach einem der Ansprüche 1 bis 9, wobei die Einrichtung zum Entfernen eine Vakuumpumpeneinrichtung und eine zusätzliche Ventileinrichtung aufweist.

11. Vorrichtung nach einem der Ansprüche 1 bis 10, wobei die Ventileinrichtung (Vi) eine erste und zweite Schaltereinrichtung (LSio, LSic) aufweist, die erste Schaltereinrichtung (LSio) anzeigt, wann die Ventileinrichtung (Vi) offen ist, und die zweite Schaltereinrichtung (LSic) anzeigt, wann die Ventileinrichtung (Vi) geschlossen ist, und sich die erste und zweite Schaltereinrichtung in entgegengesetzten Zuständen befinden, so daß bei geschlossener erster Schaltereinrichtung die zweite Schaltereinrichtung offen ist.

12. Vorrichtung nach einem der Ansprüche 1 bis 11, wobei die elektronische Steuereinrichtung (100) eine Speichereinrichtung aufweist und ferner aufweist: eine Einrichtung zum Empfangen von Eingabesignalen von der Ventileinrichtung (Vi), die den geschlossenen oder offenen Zustand der Ventileinrichtung anzeigen, und eine Einrichtung zum Übertragen von Ausgabesignalen zu der Ventileinrichtung, um die Ventileinrichtung selektiv zu öffnen oder zu schließen, wobei ein Satz von Abbildungssignalen der Eingabe- und Ausgabesignale in der Speichereinrichtung gespeichert wird.

13. Vorrichtung nach Anspruch 12, ferner mit einer Einrichtung zum Sperren der Übertragung der Ausgabesignale zu der Ventileinrichtung (Vi), außer dann, wenn ein Freigabesignal durch die Computereinrichtung ausgegeben wird.

14. Vorrichtung nach einem der Ansprüche 1 bis 13, ferner mit einer Einrichtung zum Überwachen des ordnungsgemäßen Betriebs der Computereinrichtung, wobei die Überwachungseinrichtung ein Sperrsignal ausgibt, um bei einem Fehler der Computereinrichtung eine Betätigung der Ventileinrichtung zu verhindern.

15. Vorrichtung nach einem der Ansprüche 12 bis 14, ferner mit einer in der Speichereinrichtung gespeicherten Maskeneinrichtung, wobei die Computereinrichtung die Abbildungssignale der Eingabe- und Ausgabesignale vergleicht und ein Alarmsignal erzeugt, wenn die Abbildungssignale der Eingaben und Ausgaben nicht als Reaktion auf das Setzen eines Bits in der Maskeneinrichtung übereinstimmen.

16. Vorrichtung nach einem der Ansprüche 11 bis 15, ferner mit einer Einrichtung zum Überwachen des Zustands der ersten und zweiten Schaltereinrichtung und ferner mit einer Einrichtung zum Erzeugen eines Alarmsignals, wenn sich die erste und zweite Schaltereinrichtung nicht in den ordnungsgemäßen Zuständen befinden.

17. Vorrichtung nach einem der Ansprüche 1 bis 16, wobei sich die Ventileinrichtung (Vi) zwischen einem ersten Zustand und einem zweiten Zustand als Reaktion auf Befehle von der Computereinrichtung bewegt, und ferner mit einer Zeitgebereinrichtung (112) zum Erzeugen eines Alarmsignals, wenn sich die Ventileinrichtung (Vi) nicht in einem vorbestimmten Zeitintervall von dem ersten in den zweiten Zustand bewegt.

18. Vorrichtung nach einem der Ansprüche 3 bis 17, wobei die Einrichtung zum Reagieren eine zweite Ventileinrichtung (V4) aufweist, um den ersten und zweiten Bestandteil zur Bildung des Sterilisiergases miteinander reagieren zu lassen, und die zweite Ventileinrichtung (V4) durch die Computereinrichtung als Reaktion auf die Messung ausgewählter Parameter der mehreren gemessenen Parameter gesteuert wird.

57

**Revendications**

1. Appareil pour le traitement d'objets avec un gaz, comprenant : une chambre (10) pour la réception d'un objet à traiter; des dispositifs (20, 22) pour le débit du gaz dans la chambre (10) comprenant un dispositif à vannes (20) couplés à la chambre (10) pour le débit du gaz dans la chambre, le dispositif (20) pour éliminer les gaz de la chambre (10) après un intervalle de temps prédéterminé, un dispositif (5) pour mesurer une pluralité de paramètres dans la chambre et pour générer une pluralité de signaux électriques associés à l'un des paramètres mesurés, un dispositif de contrôle électronique (100) recevant la pluralité de signaux électriques associés à l'un des paramètres mesurés de la chambre (10) pour le contrôle du dispositif à vannes (20) et du dispositif (20) d'évacuation, le dispositif de contrôle électronique (100) comprend un ordinateur pour la mise en cycle de l'appareil par une pluralité de stades suivant une séquence prédéterminée d'instructions, l'ordinateur comprenant un dispositif d'avortement de l'opération de l'appareil, caractérisé en ce que l'état d'avortement interrompt l'opération à une ou une pluralité d'états de défaillance définis ayant des conditions prédéterminées en réponse à une défaillance de l'appareil, l'état de défaillance sélectionné étant dépendant de l'état du cycle au cours duquel la défaillance se produit et comprenant de plus un dispositif (S2) pour la mise en cycle de l'appareil suivant la séquence prédéterminée jusqu'à un autre état défini une fois que l'état de défaillance défini est atteint, l'état défini comprenant un des états suivant la séquence prédéterminée d'instructions, l'état défini supplémentaire étant dépendant de l'état de défaillance défini atteint et étant un état qui maintient l'appareil dans des standards acceptables de sécurité.

2. Appareil suivant la revendication 1, où le gaz est un gaz stérilisant, par lequel les objets sont stérilisés par le gaz.

3. Appareil suivant la revendication 2, où le gaz stérilisant est généré à partir d'au moins deux composants, et comprenant, de plus, un premier dispositif pour recevoir un premier composant du gaz, un second dispositif pour recevoir un deuxième composant du gaz, un dispositif pour permettre aux premier et deuxième composants du gaz de réagir l'un avec l'autre pour générer le gaz stérilisant, ce dispositif de réaction étant contrôlé par l'ordinateur en réponse aux mesures qui ont été sélectionnées à partir d'une pluralité de paramètres mesurés.

4. Appareil suivant la revendication 3, comprenant de plus une vanne (V5) pour le débit d'un gaz stable dans la chambre (10).

5. Appareil suivant la revendication 3 ou 4, comprenant de plus une vanne (V7) pour le débit d'air filtré dans la chambre (10).

6. Appareil suivant l'une quelconque des revendications 3 à 5, comprenant de plus une vanne (V6) pour l'apport de vapeur d'eau dans la chambre (10) pour affecter le niveau d'humidité de la chambre.

7. Appareil suivant l'une quelconque des revendications 3 à 6, où la pluralité de paramètres mesurés comprend la température, la pression et l'humidité dans la chambre (10) et la concentration du gaz stérilisant dans la chambre.

8. Appareil suivant l'une quelconque des revendications 2 à 7, où le gaz stérilisant comprend le dioxyde de chlore.

9. Appareil suivant l'une quelconque des revendications 3 à 7, où le gaz stérilisant comprend le dioxyde de chlore et le premier composant comprend le gaz chlore et le deuxième composant comprend le chlorite de sodium.

10. Appareil suivant l'une quelconque des revendications 1 à 9, où le dispositif pour l'élimination comprend une pompe à vide et une vanne supplémentaire.

11. Appareil suivant l'une quelconque des revendications 1 à 10, où la vanne (Vi) comprend un premier et un deuxième commutateur (LSio, LSic) le premier commutateur (LSio) indiquant quand la vanne (Vi) est ouverte et le deuxième commutateur (LSic) indiquant quand la vanne (Vi) est fermée, le premier et le deuxième commutateur étant en état opposé, de sorte que quand le premier commutateur est fermé, le second commutateur est ouvert.

12. Appareil suivant l'une quelconque des revendications 1 à 11, où le dispositif de contrôle électronique (100) comprend un dispositif de mémoire et comprend, de plus, un dispositif pour recevoir les signaux d'entrée de la vanne (Vi) indiquant l'état fermé ou ouvert de la vanne et un dispositif de signaux de sortie de transmission de la vanne pour ouvrir ou fermer de manière sélective la vanne, l'enregistrement des signaux images, des signaux d'entrée et de sortie étant placé dans la mémoire.

13. Appareil suivant la revendication 12, comprenant, de plus, un dispositif pour mettre hors service les signaux de sortie, une fois qu'ils sont transmis à la vanne (Vi), sauf quand un signal de mise en oeuvre provient de l'ordinateur.

14. Appareil suivant l'une quelconque des revendications 1 à 13, comprenant, de plus, un dispositif pour contrôler l'opération propre de l'ordinateur, ce dispositif de contrôle assurant un signal de mise hors service pour prévenir la mise en oeuvre des vannes dans le cas d'une défaillance de l'ordinateur.

15. Appareil suivant l'une quelconque des revendications 12 à 14, comprenant, de plus, un dispositif à masques placé dans la mémoire, l'ordinateur comparant les signaux images aux signaux d'entrée et de sortie et générant un signal d'alarme si les signaux images d'entrée et de sortie ne correspondent pas à la mise en place d'un bit dans le masque.

16. Appareil suivant l'une quelconque des revendications 11 à 15, comprenant, de plus, un dispositif pour contrôler l'état des premier et deuxième commutateurs, et comprenant aussi un dispositif de génération d'un signal d'alarme si le premier et le second commutateur ne sont pas dans leur état approprié.

17. Appareil suivant l'une quelconque des revendications 1 à 16, où la vanne (Vi) se déplace entre un premier état et un deuxième état en réponse à l'instruction de l'ordinateur, et comprenant, de plus, une minuterie (112) pour générer un signal d'alarme si la vanne (Vi) ne se déplace pas du premier état au second état dans un intervalle de temps prédéterminé.

18. Appareil suivant l'une quelconque des revendications 3 à 17, où le dispositif de réaction comprend une deuxième vanne (V4) pour permettre au premier et deuxième composant de réagir l'un avec l'autre pour générer le gaz stérilisant, la deuxième vanne (V4) étant contrôlée par l'ordinateur en réponse aux mesures de paramètres sélectionnés parmi une pluralité de paramètres mesurés.

FIG. I

EP 0 211 073 B1

FIG.2

EP 0 211 073 B1

FIG. 3

| ADDRESS | DESCRIPTION | $A_{15}$ | $A_{14}$ | $A_{13}$ | $A_{12}$ |
|---|---|---|---|---|---|
| ∅∅ - FF | INTERNAL RAM | | | | |
| ∅∅∅∅ - ∅FFF | INTERNAL ROM | ∅ | ∅ | ∅ | ∅ |
| 1∅∅∅ - 1FFF | EXTERNAL ROM | ∅ | ∅ | ∅ | 1 |
| 2∅∅∅ - 2∅3F | EXTERNAL SRAM | ∅ | ∅ | 1 | ∅ |
| 4∅∅∅ - 4∅∅F | CLOCK | ∅ | 1 | ∅ | ∅ |
| 6∅∅∅ - 6∅∅7 | A/D READ | ∅ | 1 | 1 | ∅ |
| C∅∅∅ | X∅∅ - X∅7 ⎫ | 1 | 1 | ∅ | ∅ |
| C∅∅1 | X1∅ - X17 ⎬ DIN | 1 | 1 | ∅ | ∅ |
| C∅∅2 | X2∅ - X27 ⎪ | 1 | 1 | ∅ | ∅ |
| C∅∅3 | X3∅ - X37 ⎭ | 1 | 1 | ∅ | ∅ |
| E∅∅∅ | Y∅∅ - Y∅7 ⎫ | 1 | 1 | 1 | ∅ |
| E∅∅1 | Y1∅ - Y17 ⎬ DOU | 1 | 1 | 1 | ∅ |
| E∅∅2 | Y2∅ Y27 ⎪ | 1 | 1 | 1 | ∅ |
| E∅∅3 | Y3∅ Y37 ⎭ | 1 | 1 | 1 | ∅ |
| E∅∅4 | WATCHDOG - RESET | 1 | 1 | 1 | ∅ |

_FIG. 3A (a)_

| FIG. 3A(a) | FIG. 3A(b) |
|---|---|

_FIG. 3A_

ADDRESS BUS BITS

| $A_{11}$ $A_{10}$ $A_9$ $A_8$ | $A_7$ $A_6$ $A_5$ $A_4$ | $A_3$ $A_2$ $A_1$ $A_\phi$ |
|---|---|---|
| ———————— | $A_7$ $A_6$ $A_5$ $A_4$ | $A_3$ $A_2$ $A_1$ $A_\phi$ |
| $A_{11}$ $A_{10}$ $A_9$ $A_8$ | $A_7$ $A_6$ $A_5$ $A_4$ | $A_3$ $A_2$ $A_1$ $A_\phi$ |
| $A_{11}$ $A_{10}$ $A_9$ $A_8$ | $A_7$ $A_6$ $A_5$ $A_4$ | $A_3$ $A_2$ $A_1$ $A_\phi$ |
| $\phi$ $\phi$ $\phi$ $\phi$ | $\phi$ $\phi$ $A_5$ $A_4$ | $A_3$ $A_2$ $A_1$ $A_\phi$ |
| $\phi$ $\phi$ $\phi$ $\phi$ | $\phi$ $\phi$ $\phi$ $\phi$ | $A_3$ $A_2$ $A_1$ $A_\phi$ |
| $\phi$ $\phi$ $\phi$ $\phi$ | $\phi$ $\phi$ $\phi$ $\phi$ | $\phi$ $A_2$ $A_1$ $A_\phi$ |
| $\phi$<br>$\phi$<br>$\phi$<br>$\phi$ | $\phi$<br>$\phi$<br>$\phi$<br>$\phi$ | $\phi$ $\phi$ $\phi$ $\phi$<br>$\phi$ $\phi$ $\phi$ $1$<br>$\phi$ $\phi$ $1$ $\phi$<br>$\phi$ $\phi$ $1$ $1$ |
| $\phi$<br>$\phi$<br>$\phi$<br>$\phi$ | $\phi$<br>$\phi$<br>$\phi$<br>$\phi$ | $\phi$ $\phi$ $\phi$ $\phi$<br>$\phi$ $\phi$ $\phi$ $1$<br>$\phi$ $\phi$ $1$ $\phi$<br>$\phi$ $\phi$ $1$ $1$ |
| $\phi$ | $\phi$ | $\phi$ $1$ $\phi$ $\phi$ |

$$\underline{FIG.\ 3A\,(b)}$$

FIG. 11

FIG. 4

EP 0 211 073 B1

○ LT1 – DOOR OPEN
○ LT2 – EVAC FAIL
○ LT3 – FILL FAIL
○ LT4 – STERIL FAIL
○ LT5 – PURGE FAIL
○ LT6 – LOAD UNSTERILE
○ LT7
○ LT8

○ LT11 – READY FOR CYCLE
○ LT12 – CYCLE IN PROGRESS
○ LT13 – EVAC IN PROGRESS
○ LT14 – FILL IN PROGRESS
○ LT15 – STERIL IN PROGRESS
○ LT16 – PURGE IN PROGRESS
○ LT17 – REMOVE LOAD
○ LT18 –

○

START CYCLE
S1

○

ABORT – RESET
S2

## FIG. 5

FIG. 6A

FIG. 6

FIG. 6B

EP 0 211 073 B1

FIG.7a | FIG.7b

## FIG. 7

PROCESS

| | | | 29 | | | | | | | | | 30 | | | 31 | | | | | | | |
|---|---|---|---|---|---|---|---|---|---|---|---|---|---|---|---|---|---|---|---|---|---|---|---|
| | | | 0 | 1 | 2 | 3 | 4 | 5 | 6 | 7 | 8 | 9 | 10 | 11 | 12 | 13 | 14 | 15 | 16 | 17 | 18 | 19 |
| DOOR OPEN | LT01 | | 1 | 1 | 0 | 0 | 0 | 0 | 0 | 0 | 0 | 0 | 0 | 0 | 0 | 0 | 0 | 0 | 0 | 0 | 0 | 0 |
| EVAC FAIL | LT02 | | 0 | 0 | 0 | 0 | 0 | 0 | 0 | 0 | 0 | 0 | 0 | 0 | 0 | 0 | 0 | 0 | 0 | 0 | 0 | 0 |
| FILL FAIL | LT03 | C | 0 | 0 | 0 | 0 | 0 | 0 | 0 | 0 | 0 | 0 | 0 | 0 | 0 | 0 | 0 | 0 | 0 | 0 | 0 | 0 |
| STERIL FAIL | LT04 | C | 0 | 0 | 0 | 0 | 0 | 0 | 0 | 0 | 0 | 0 | 0 | 0 | 0 | 0 | 0 | 0 | 0 | 0 | 0 | 0 |
| PURGE FAIL | LT05 | 0 | 0 | 0 | 0 | 0 | 0 | 0 | 0 | 0 | 0 | 0 | 0 | 0 | 0 | 0 | 0 | 0 | 0 | 0 | 0 | 0 |
| LOAD MASTER | LT06 | 0 | 0 | 0 | 0 | 0 | 0 | 0 | 0 | 0 | 0 | 0 | 0 | 0 | 0 | 0 | 0 | 0 | 0 | 0 | 0 | 0 |
| | LT07 | | 0 | 0 | 0 | 0 | 0 | 0 | 0 | 0 | 0 | 0 | 0 | 0 | 0 | 0 | 0 | 0 | 0 | 0 | 0 | 0 |
| | LT08 | | 0 | 0 | 0 | 0 | 0 | 0 | 0 | 0 | 0 | 0 | 0 | 0 | 0 | 0 | 0 | 0 | 0 | 0 | 0 | 0 |
| READY | LT11 | | 0 | 0 | 1 | 0 | 0 | 0 | 0 | 0 | 0 | 0 | 0 | 0 | 0 | 0 | 0 | 0 | 0 | 0 | 0 | 0 |
| CYCLE | LT12 | | 0 | 0 | 0 | 1 | 1 | 1 | 1 | 1 | 1 | 1 | 1 | 1 | 1 | 1 | 1 | 1 | 1 | 1 | 1 | 1 |
| EVAC | LT13 | C | 0 | 0 | 0 | 0 | 0 | 0 | 1 | 1 | 1 | 0 | 0 | 0 | 0 | 0 | 0 | 0 | 0 | 0 | 0 | 0 |
| FILL | LT14 | C | 0 | 0 | 0 | 0 | 0 | 0 | 0 | 0 | 0 | 1 | 1 | 1 | 0 | 0 | 0 | 0 | 0 | 0 | 0 | 0 |
| STERIL | LT15 | 0 | 0 | 0 | 0 | 0 | 0 | 0 | 0 | 0 | 0 | 0 | 0 | 0 | 1 | 1 | 1 | 1 | 1 | 0 | 0 | 0 |
| PURGE | LT16 | 1 | 0 | 0 | 0 | 0 | 0 | 0 | 0 | 0 | 0 | 0 | 0 | 0 | 0 | 0 | 0 | 0 | 0 | 1 | 1 | 1 |
| ROM LOAD | LT17 | | 0 | 0 | 0 | 0 | 0 | 0 | 0 | 0 | 0 | 0 | 0 | 0 | 0 | 0 | 0 | 0 | 0 | 0 | 0 | 0 |
| | LT18 | | 0 | 0 | 0 | 0 | 0 | 0 | 0 | 0 | 0 | 0 | 0 | 0 | 0 | 0 | 0 | 0 | 0 | 0 | 0 | 0 |
| MAIN VAC | VV01 | | 0 | 0 | 0 | 0 | 0 | 1 | 1 | 0 | 0 | 0 | 0 | 0 | 0 | 0 | 0 | 0 | 0 | 0 | 0 | 0 |
| GAS ENABLE | VV02 | | 0 | 0 | 0 | 0 | 0 | 0 | 0 | 0 | 0 | 0 | 0 | 1 | 1 | 1 | 1 | 1 | 1 | 1 | 1 | 1 |
| VAC CTRL | VV03 | C | 0 | 0 | 0 | 0 | 0 | 0 | 0 | 0 | 0 | 0 | 0 | 0 | 0 | 0 | 0 | 0 | 0 | 1 | 1 | 0 |
| GAS CTRL | VV04 | C | 0 | 0 | 0 | 0 | 0 | 0 | 0 | 0 | 0 | 0 | 0 | 0 | C | C | C | C | 0 | 0 | 0 | 0 |
| N2 CTRL | VV05 | 0 | 0 | 0 | 0 | 0 | 0 | 0 | 0 | 0 | 0 | 0 | 0 | C | C | C | C | C | 0 | 0 | 0 | 0 |
| H2O CTRL | VV06 | 2 | 0 | 0 | 0 | 0 | 0 | 0 | 0 | 0 | 0 | C | C | C | C | C | C | C | 0 | 0 | 0 | 0 |
| ATM VENT | VV07 | | 1 | 1 | 1 | 0 | 0 | 0 | 0 | 0 | 0 | 0 | 0 | 0 | 0 | 0 | 0 | 0 | 0 | 0 | 0 | 0 |
| VAC VLV. | VV08 | | 0 | 0 | 0 | 0 | 0 | 0 | 0 | 0 | 0 | 0 | 0 | 1 | 1 | 1 | 1 | 1 | 1 | 1 | 1 | 0 |
| VAC PUMP | PP01 | C | 0 | 0 | 0 | 0 | 0 | 0 | 1 | 1 | 1 | 1 | 1 | 1 | 1 | 1 | 1 | 1 | 1 | 1 | 1 | 1 |
| HEATER | HT01 | C/0 | 0 | 0 | 0 | 0 | 1 | C | C | C | C | C | C | C | C | C | C | C | C | C | C | C |
| GAIN CHG. | GC1 | 3 | 0 | 0 | 0 | 0 | 0 | 0 | 0 | 0 | 0 | 0 | 0 | 0 | 0 | 0 | 0 | 0 | 0 | 0 | 0 | 0 |

DISPLAYS / VALVES

## FIG. 7a

69

STATES     ABORT

32 | 33 | 34 35 | 36

| 20 | 21 | 22 | 23 | 24 | 25 | 26 | 27 | 28 | 29 | 30 | 31 | 32 | 33 | 34 | 35 | 36 | 37 | 38 |
|---|---|---|---|---|---|---|---|---|---|---|---|---|---|---|---|---|---|---|
| 0 | 0 | 0 | 0 | 0 | 0 | 0 | 0 | 0 | 0 | 0 | 0 | 0 | 0 | 0 | 0 | 0 | 0 | 0 |
| 0 | 0 | 0 | 0 | 0 | 0 | 0 | 0 | 0 | 1 | 0 | 0 | 0 | 0 | 0 | 0 | 0 | 0 | 0 |
| 0 | 0 | 0 | 0 | 0 | 0 | 0 | 0 | 0 | 0 | 1 | 0 | 0 | 0 | 0 | 0 | 0 | 0 | 0 |
| 0 | 0 | 0 | 0 | 0 | 0 | 0 | 0 | 0 | 0 | 0 | 1 | 0 | 0 | 0 | 0 | 0 | 0 | 0 |
| 0 | 0 | 0 | 0 | 0 | 0 | 0 | 0 | 0 | 0 | 0 | 0 | 1 | 0 | 0 | 0 | 0 | 0 | 0 |
| 0 | 0 | 0 | 0 | 0 | 0 | 0 | 0 | 0 | 0 | 0 | 0 | 0 | 0 | 0 | 0 | 0 | 0 | 0 |
| 0 | 0 | 0 | 0 | 0 | 0 | 0 | 0 | 0 | 0 | 0 | 0 | 0 | 0 | 0 | 0 | 0 | 0 | 0 |
| 0 | 0 | 0 | 0 | 0 | 0 | 0 | 0 | 0 | 0 | 0 | 0 | 0 | 0 | 0 | 0 | 0 | 0 | 0 |
| 0 | 0 | 0 | 0 | 0 | 0 | 0 | 0 | 0 | 0 | 0 | 0 | 0 | 0 | 0 | 0 | 0 | 0 | 1 |
| 1 | 1 | 1 | 1 | 1 | 1 | 1 | 1 | 1 | 0 | 0 | 1 | 1 | 1 | 1 | 1 | 1 | 0 | 0 |
| 0 | 0 | 0 | 0 | 0 | 0 | 0 | 0 | 0 | 0 | 0 | 0 | 0 | 0 | 0 | 0 | 0 | 0 | 0 |
| 0 | 0 | 0 | 0 | 0 | 0 | 0 | 0 | 0 | 0 | 0 | 0 | 0 | 0 | 0 | 0 | 0 | 0 | 0 |
| 0 | 0 | 0 | 0 | 0 | 0 | 0 | 0 | 0 | 0 | 0 | 0 | 0 | 0 | 0 | 0 | 0 | 0 | 0 |
| 1 | 1 | 1 | 1 | 1 | 1 | 1 | 1 | 1 | 0 | 0 | 1 | 1 | 1 | 1 | 1 | 1 | 0 | 0 |
| 0 | 0 | 0 | 0 | 0 | 0 | 0 | 0 | 0 | 0 | 0 | 0 | 0 | 0 | 0 | 0 | 0 | 1 | 0 |
| 0 | 0 | 0 | 0 | 0 | 0 | 0 | 0 | 0 | 0 | 0 | 0 | 0 | 0 | 0 | 0 | 0 | 0 | 0 |
| 0 | 0 | 0 | 0 | 0 | 0 | 0 | 0 | 0 | 0 | 0 | 0 | 0 | 0 | 0 | 0 | 0 | 0 | 0 |
| 1 | 1 | 1 | 1 | 0 | 0 | 0 | 1 | 0 | 0 | 0 | 1 | 1 | 1 | 0 | 1 | 0 | 0 | 0 |
| 0 | 0 | 1 | 1 | 0 | 0 | 0 | 1 | 0 | 0 | 0 | 0 | 0 | 1 | 0 | 0 | 0 | 0 | 0 |
| 0 | 0 | 0 | 0 | 0 | 0 | 0 | 1 | 0 | 0 | 0 | 0 | 0 | 0 | 0 | 0 | 0 | 0 | 0 |
| 0 | 0 | 0 | 0 | 0 | 0 | 0 | 0 | C | 0 | 0 |  | 0 | 0 | 0 | C | C | C | 0 |
| 0 | 0 | 0 | 0 | 0 | 0 | 0 | 0 | 0 | 0 | 0 | 0 | 0 | 0 | 0 | 0 | 0 | 0 | 0 |
| 0 | 0 | 0 | 0 | 0 | 0 | 0 | 0 | 0 | 1 | 1 | 0 | 0 | 0 | 0 | 0 | 0 | 1 | 1 |
| 0 | 0 | 1 | 1 | 0 | 0 | 0 | 1 | 0 | 0 | 0 | 0 | 0 | 1 | 0 | 0 | 0 | 0 | 0 |
| 1 | 1 | 1 | 1 | 1 | 1 | 0 | 0 | 0 | 0 | 0 | 1 | 1 | 1 | 1 | 1 | 0 | 0 | 0 |
| C | C | C | C | C | C | 0 | 0 | 0 | 0 | 0 | C | C | C | C | C | 0 | 0 | 0 |
| 0 | 0 | 0 | 0 | 0 | 1 | 0 | 0 | 0 | 0 | 0 | 0 | 0 | 0 | 1 | 0 | 0 | 0 | 0 |

FIG. 7b

70

SEQ

SEQ: ○

GET
CURRENT STATE    *180*

STATE
>
MAX
? *182*    —Y→    SET
STATE = 31 *184*

N

PERFORM
STATE (ST)    *186*

SEQR: ○

ALARM
OR
TIMEOUT
? *200a*    —Y→    SET
STATE = ABORT *201*

N

N←    FØ
SET
? *202*

Y

RET

*FIG. 7A*

71

ST

188

COMPUTE
DEPENDENT EVENT

RESULT
TRUE
?

189

Y

N

192

SET
NEXT STATE

193

SET
ABORT STATE

194

DISABLE
EVENT TIMEOUT

195

CLEAR
ACTION MASKS

196

PERFORM
ACTIONS

197

ENABLE
ACTION TIMEOUTS

198

CLEAR
HOLD FLAG    (FØ)

190

SET
HOLD FLAG    (FØ)

SEQR

FIG. 7B

72

FIG.8

FIG.13

73

FIG.9

300 (MAIN DISPATCH PROGRAM)

FIG. 10A

FIG. 10B

FIG. 10

FIG. 12

FIG. 12A

DATA MEMORY MAP

DATA MEMORY MAP

FIG. 12B

FIG. 14

FIG. 15

FIG. 18

EP 0 211 073 B1

| TIMERS |
|--------|
| TICK |
| TSEC |
| TMIN |

(RRT)

| RESET & RESTART TIMERS |
|---|

| TIMER∅ | |
|--------|--------|
| TH∅ | TL∅ |

| TIMERS |
|--------|
| TICK |
| TSEC |
| TMIN |

| STAT |
|------|

MINF  SECF  TICF

FLAGS

FIG. 16

| | CONTACT INPUTS |
|------|------|
| C∅∅∅ : | X∅ |
| C∅∅1 : | X1 |
| C∅∅2 : | X2 |
| C∅∅3 : | X3 |

(RCI)

| READ CONTACT INPUTS |
|---|

| | CONTACT INPUT STATUS |
|------|------|
| 2C : | CCI∅ |
| 2D : | CCI1 |
| 2E : | CCI2 |
| 2F : | CCI3 |

FIG. 17

| CONTACT INPUT STATUS | |
|---|---|
| 2C: | CCIØ |
| 2D: | CCI 1 |
| 2E: | CCI2 |
| 2F: | CCI3 |

| MASKS | |
|---|---|
| 24: | MSKØ |
| 25: | MSKI |
| 26: | MSK2 |
| 27: | MSK3 |

(CSC)

CONTACT STATUS CHECK

ALARM

| STATUS | |
|---|---|
| | |

ALMF

| CONTACT OUTPUT STATUS | |
|---|---|
| 28: | CCOØ |
| 29: | CCO1 |
| 2A: | CCO2 |
| 2B: | CCO3 |

_FIG. 19_

| CONTACT OUTPUT STATUS | |
|---|---|
| 28: | CCOØ |
| 29: | CCO1 |
| 2A: | CCO2 |
| 2B: | CCO3 |

(WCO)

WRITE CONTACT OUTPUTS

| CONTACT OUTPUTS | |
|---|---|
| EØØØ: | YØ |
| EØØI: | YI |
| EØØ2: | Y2 |
| EØØ3: | Y3 |

_FIG. 20_

| | ANALOG INPUTS |
|---|---|
| 6000: | IN0 |
| 6001: | IN1 |
| 6002: | IN2 |
| 6003: | IN3 |
| 6004: | IN4 |
| 6005: | IN5 |
| 6006: | IN6 |
| 6007: | IN7 |

(RAI)

READ ANALOG INPUTS

| | ANALOG INPUT DATA |
|---|---|
| 30: | ADI0 |
| 31: | ADI1 |
| 32: | ADI2 |
| 33: | ADI3 |
| 34: | ADI4 |
| 35: | ADI5 |
| 36: | ADI6 |
| 37: | ADI7 |

FIG. 21

TUP

DECREMENT TIMER

TIME = 0 ?

Y → SET FLAG

N → CLEAR FLAG

RET

FIG. 22

83

FIG. 23

FIG. 24